# EUROPEAN PATENT APPLICATION

(11) **EP 3 903 884 A1**
(43) Date of publication of application: **03.11.2021**
(21) Application number: 19905990.8
(22) Date of filing: 26.12.2019
(51) Int. Cl.: A61P 37/04, C07K 14/725, C12N 5/074, C12N 5/10, C12N 15/12, C12N 15/63, A61K 38/16

(54) **MODIFIED TCR AND PRODUCTION METHOD THEREFOR**

(30) Priority: 26.12.2018 JP 2018242733
(71) Applicant: KIRIN HOLDINGS KABUSHIKI KAISHA, Nakano-ku, Tokyo 164-0001 (JP); Kyoto University, Kyoto-shi, Kyoto 606-8501 (JP)
(72) Inventor: TAKAYANAGI Shinichiro, Tokyo 164-0001 (JP); HASEGAWA Saki, Tokyo 164-0001 (JP); FUKUMOTO Ken, Tokyo 164-0001 (JP); KUNISATO Atsushi, Tokyo 164-0001 (JP); NISHIKAWA Satoshi, Tokyo 100-0004 (JP); KANEKO Shin, Kyoto-shi, Kyoto 606-8501 (JP)
(74) Representative: Hoffmann Eitle
(86) International application number: PCT/JP2019/051311
(87) International publication number: WO 2020/138371

(57) **Abstract**

An object of the invention is to provide a modified T-cell receptor (TCR) which does not cause the antigen responsiveness and which is capable of holding CD3 subunits on a cell membrane and causing the CD3 subunits to function. The invention is an invention related to a modified TCR comprising a first polypeptide and a second polypeptide, wherein the first polypeptide is a polypeptide which comprises a constant region of human TCRα or a fragment of the constant region and which does not comprise a variable region of human TCRα, and the second polypeptide is a polypeptide which comprises a constant region of human TCRβ or a fragment of the constant region and which does not comprise a variable region of human TCRβ.

## Description

### Technical Field

The invention is an invention related to a modified TCR and a production method thereof.

### Background Art

T cells are immune cells derived from hematopoietic stem cells. A T cell expresses an α chain (TCRα) and a β chain (TCRβ) of a T-cell receptor (TCR) on the cell surface, and TCRα and TCRβ form a heterodimer. TCRα and TCRβ are each composed of a variable region and a constant region each with sequence variety, and the antigen recognition site formed with the variable regions of the TCRα/TCRβ heterodimer specifically recognizes the major histocompatibility complex (MHC)-peptide complex on the target cell. The antigen recognition site of the TCRα/TCRβ heterodimer is considered to recognize also whether the target cell is an autologous cell or a non-autologous cell and to be involved in the allorecognition response (alloreaction) in the body.

The TCRα/TCRβ heterodimer forms a complex with CD3 subunits (CD3ε, CD3δ, CD3y and CD3ζ). The TCRα/TCRβ heterodimer itself is specialized in recognizing the antigen and binding to the antigen and is not capable of transmitting signals. Instead, various adopter molecules, coreceptors or enzyme molecules assembled by the antigen recognition through the TCR phosphorylate and activate the intracellular regions of the CD3 subunits, and the signal of antigen stimulation is further transmitted to the downstream. The TCR/CD3 complex signal functions not only as a survival signal or an exclusion signal in the positive selection or the negative selection of immature T cells in the T-cell differentiation process in the thymus but also as an activation signal or a proliferative signal in T cells which have moved to the periphery after the maturation in the thymus.

T cells are roughly classified into helper T cells and cytotoxic T cells, and a T-cell therapy method using the antitumor effect which cytotoxic T cells have has been developed. As a T cell therapy method using autologous T cells, CAR-T therapy for treating a tumor, an infection or the like by expressing a chimeric antigen receptor (CAR) on T cells has been put to practical use and has already been approved in Europe and America as a cellular medicine. In the future, a therapeutic method using non-autologous T cells has been desired for reducing the burden of the patients associated with the collection of T cells as well as for improving the quality of the T cells and reducing the production costs.

In the case of a non-autologous T cell therapy method, however, the onset of tissue injury caused by potent alloreaction caused by various TCRα/TCRβ heterodimers which non-autologous T cells have, namely graft-versus-host disease (GVHD), is a problem. Attempts to reduce or eliminate the alloreaction through treatment such as knockdown or knockout of the TCR gene have been currently made to solve the problem (NPLs 1 and 2).

Recently, a method for inducing differentiation of induced pluripotent stem (iPS) cells established from non-autologous T cells into T cells has been proposed as means for producing a homogeneous highly functional T cell preparation without depending on donors (NPLs 3, 4 and 5).

Moreover, to provide a more versatile non-autologous iPS cell-derived T cell preparation, a method using the iPS cell bank established from the blood cells (non-T cells) of donors with homozygous human leukocyte antigen (HLA) haplotype, which is human MHC, has also been proposed (NPL 6).

In order to efficiently induce differentiation of non-T cell-derived iPS cells into T cells, it is considered to be important to rearrange the functional TCR gene loci during the differentiation and promote proliferation and survival by stimulating thus formed TCR/CD3 complexes *in vitro* (NPL 7).

### Citation List

### Non-Patent Literature (NPL)

NPL 1: Okamoto S. et al, Cancer Res. 69: 9003-9011, 2009
NPL 2: Qasim W. et al, Sci Transl Med. 9, 2017
NPL 3: Nishimura T. et al, Cell Stem Cell. 12: 114-226, 2013
NPL 4: Vizcardo R. et al, Cell Stem Cell. 12: 31-36, 2013
NPL 5: Themeli M. et al, Nat. Biotechnol. 31: 928-33, 2013
NPL 6: Shin Kaneko, The Medical Frontline 69: 724-733, 2014
NPL 7: Minagawa et al., Cell Stem Cell. 23: 1-9, 2018

### Summary of Invention

### Technical Problem

When the TCR gene is knocked down or knocked out, however, the CD3 subunits are no longer held on the surface of cell membrane, and thus the proliferative signal and the survival signal through the TCR/CD3 complex is not input. This means, for example, that a CD3 agonist antibody or the like used for amplifying a donor-derived T cell preparation *ex vivo* does not function anymore, which is a problem in efficient production and formulation of a cellular medicine comprising non-autologous T cells.

In the problem, for proliferation and survival of desired T cells through the CD3 signals at appropriate time, the TCRα/TCRβ complex is desirably expressed on the cell surface. In addition, in order that the allorecognition response by non-autologous T cells is not caused, it is desirable that the TCRα/TCRβ complex does not recognize alloantigens such as an alloHLA-peptide complex.

Accordingly, an object of the invention is to provide a modified TCR which does not cause the antigen responsiveness and which is capable of holding CD3 subunits on the cell membrane and intermediating CD3 signal transduction.

### Solution to Problem

To solve the problems, the present inventors have designed TCRα-deleted variants and TCRβ-deleted variants in which the variable region and the constant region were deleted in a step-wise manner with respect to both TCRα and TCRβ and have examined whether the expression of CD3 subunits on the surface of cell membrane would be observed by introducing combinations of the deleted variants into 293T cells which constitutively expressed CD3 subunits and endogenous TCR-knockout Jurkat cells.

As a result, it was found that a modified TCR that contains a polypeptide which does not contain the variable region of TCRα and which contains the constant region of TCRα or a fragment of the constant region and a polypeptide which does not contain the variable region of TCRβ and which contains the constant region of TCRβ or a fragment of the constant region is capable of holding CD3 subunits on the cell membrane, and it was also found that stimulating the CD3 molecules presented on the cell surface with the modified TCR induces the activation of T cells. That is, it was found that, by expressing the modified TCR in T cells in which the endogenous TCR expression has been inhibited or eliminated, response to stimulation through the TCR/CD3 complex is possible without causing the antigen responsiveness. The invention has been thus completed.

That is, the invention provides the following invention.
1. A modified T-cell receptor (TCR) comprising a first polypeptide and a second polypeptide,
   wherein the first polypeptide is a polypeptide which comprises a constant region of human T-cell receptor α (TCRα) or a fragment of the constant region and which does not comprise a variable region of human TCRα, and
   the second polypeptide is a polypeptide which comprises a constant region of human T-cell receptor β (TCRβ) or a fragment of the constant region and which does not comprise a variable region of human TCRβ.
2. The modified TCR according to 1 above, wherein the constant region of human TCRα or the fragment of the constant region is the polypeptide described in any one of (A1) to (A3) below,
   (A1) a polypeptide consisting of the amino acid sequence of any one of SEQ ID NOs: 1 to 11,
   (A2) a polypeptide consisting of an amino acid sequence having sequence identity of 90% or more with the amino acid sequence of any one of SEQ ID NOs: 1 to 11, and
   (A3) a polypeptide consisting of an amino acid sequence in which one or some amino acids have been substituted, inserted, deleted and/or added in the amino acid sequence of any one of SEQ ID NOs: 1 to 11.
3. The modified TCR according to 1 or 2 above, wherein the constant region of human TCRβ or the fragment of the constant region is the polypeptide described in any one of (B1) to (B3) below,
   (B1) a polypeptide consisting of the amino acid sequence of any one of SEQ ID NOs: 12 to 24,
   (B2) a polypeptide consisting of an amino acid sequence having sequence identity of 90% or more with the amino acid sequence of any one of SEQ ID NOs: 12 to 24, and
   (B3) a polypeptide consisting of an amino acid sequence in which one or some amino acids have been substituted, inserted, deleted and/or added in the amino acid sequence of any one of SEQ ID NOs: 12 to 24.
4. The modified TCR according to any one of 1 to 3 above, wherein the constant region of human TCRα or the fragment of the constant region and the constant region of human TCRβ or the fragment of the constant region are the polypeptides described in any one of (a1) to (a8) below:
   (a1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
   (a2) the constant region of human TCRβ or the fragment of the constant region is a polypeptide consisting of the amino acid sequence of SEQ ID NO: 13, a polypeptide consisting of an amino acid sequence having sequence identity of 90% or more with the amino acid sequence of SEQ ID NO: 13 or a polypeptide consisting of an amino acid sequence in which one or some amino acids have been substituted, inserted, deleted and/or added in the amino acid sequence of SEQ ID NO: 13, and the constant region of human TCRα or the fragment of the constant region is a polypeptide consisting of the amino acid sequence of SEQ ID NO: 1 or 2, a polypeptide consisting of an amino acid sequence having sequence identity of 90% or more with the amino acid sequence of SEQ ID NO: 1 or 2 or a polypeptide consisting of an amino acid sequence in which one or some amino acids have been substituted, inserted, deleted and/or added in the amino acid sequence of SEQ ID NO: 1 or 2;
   (a3) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of any one of SEQ ID NOs: 1 to 3 and 5 to 11, a polypeptide consisting of an amino acid sequence having sequence identity of 90% or more with the amino acid sequence of any one of SEQ ID NOs: 1 to 3 and 5 to 11 or a polypeptide consisting of an amino acid sequence in which one or some amino acids have been substituted, inserted, deleted and/or added in the amino acid sequence of any one of SEQ ID NOs: 1 to 3 and 5 to 11;
   (a4) the constant region of human TCRβ or the fragment of the constant region is a polypeptide consisting of the amino acid sequence of SEQ ID NO: 19, a polypeptide consisting of an amino acid sequence having sequence identity of 90% or more with the amino acid sequence of SEQ ID NO: 19 or a polypeptide consisting of an amino acid sequence in which one or some amino acids have been substituted, inserted, deleted and/or added in the amino acid sequence of SEQ ID NO: 19, and the constant region of human TCRα or the fragment of the constant region is a polypeptide consisting of the amino acid sequence of SEQ ID NO: 10 or 11, a polypeptide consisting of an amino acid sequence having sequence identity of 90% or more with the amino acid sequence of SEQ ID NO: 10 or 11 or a polypeptide consisting of an amino acid sequence in which one or some amino acids have been substituted, inserted, deleted and/or added in the amino acid sequence of SEQ ID NO: 10 or 11;
   (a5) the constant region of human TCRβ or the fragment of the constant region is a polypeptide consisting of the amino acid sequence of SEQ ID NO: 21, a polypeptide consisting of an amino acid sequence having sequence identity of 90% or more with the amino acid sequence of SEQ ID NO: 21 or a polypeptide consisting of an amino acid sequence in which one or some amino acids have been substituted, inserted, deleted and/or added in the amino acid sequence of SEQ ID NO: 21, and the constant region of human TCRα or the fragment of the constant region is a polypeptide consisting of the amino acid sequence of any one of SEQ ID NOs: 7 to 11, a polypeptide consisting of an amino acid sequence having sequence identity of 90% or more with the amino acid sequence of any one of SEQ ID NOs: 7 to 11 or a polypeptide consisting of an amino acid sequence in which one or some amino acids have been substituted, inserted, deleted and/or added in the amino acid sequence of any one of SEQ ID NOs: 7 to 11;
   (a6) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11;
   (a7) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11; and
   (a8) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11.
5. The modified TCR according to any one of 1 to 4 above, wherein the constant region of human TCRα or the fragment of the constant region and the constant region of human TCRβ or the fragment of the constant region are the polypeptides described in any one of (b1) to (b7) below:
   (b1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
   (b2) the constant region of human TCRβ or the fragment of the constant region is a polypeptide consisting of the amino acid sequence of SEQ ID NO: 13, a polypeptide consisting of an amino acid sequence having sequence identity of 90% or more with the amino acid sequence of SEQ ID NO: 13 or a polypeptide consisting of an amino acid sequence in which one or some amino acids have been substituted, inserted, deleted and/or added in the amino acid sequence of SEQ ID NO: 13, and the constant region of human TCRα or the fragment of the constant region is a polypeptide consisting of the amino acid sequence of SEQ ID NO: 2, a polypeptide consisting of an amino acid sequence having sequence identity of 90% or more with the amino acid sequence of SEQ ID NO: 2 or a polypeptide consisting of an amino acid sequence in which one or some amino acids have been substituted, inserted, deleted and/or added in the amino acid sequence of SEQ ID NO: 2;
   (b3) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of any one of SEQ ID NOs: 1 to 3 and 10, a polypeptide consisting of an amino acid sequence having sequence identity of 90% or more with the amino acid sequence of any one of SEQ ID NOs: 1 to 3 and 10 or a polypeptide consisting of an amino acid sequence in which one or some amino acids have been substituted, inserted, deleted and/or added in the amino acid sequence of any one of SEQ ID NOs: 1 to 3 and 10;
   (b4) the constant region of human TCRβ or the fragment of the constant region is a polypeptide consisting of the amino acid sequence of SEQ ID NO: 21, a polypeptide consisting of an amino acid sequence having sequence identity of 90% or more with the amino acid sequence of SEQ ID NO: 21 or a polypeptide consisting of an amino acid sequence in which one or some amino acids have been substituted, inserted, deleted and/or added in the amino acid sequence of SEQ ID NO: 21, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11;
   (b5) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 4 and 6 to 11, a polypeptide consisting of an amino acid sequence having sequence identity of 90% or more with the amino acid sequence of any one of SEQ ID NOs: 4 and 6 to 11 or a polypeptide consisting of an amino acid sequence in which one or some amino acids have been substituted, inserted, deleted and/or added in the amino acid sequence of any one of SEQ ID NOs: 4 and 6 to 11;
   (b6) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 4 and 7 to 11, a polypeptide consisting of an amino acid sequence having sequence identity of 90% or more with the amino acid sequence of any one of SEQ ID NOs: 4 and 7 to 11 or a polypeptide consisting of an amino acid sequence in which one or some amino acids have been substituted, inserted, deleted and/or added in the amino acid sequence of any one of SEQ ID NOs: 4 and 7 to 11; and
   (b7) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 4 and 6 to 11, a polypeptide consisting of an amino acid sequence having sequence identity of 90% or more with the amino acid sequence of any one of SEQ ID NOs: 4 and 6 to 11 or a polypeptide consisting of an amino acid sequence in which one or some amino acids have been substituted, inserted, deleted and/or added in the amino acid sequence of any one of SEQ ID NOs: 4 and 6 to 11.
6. The modified TCR according to any one of 1 to 5 above, wherein the constant region of human TCRα or the fragment of the constant region and the constant region of human TCRβ or the fragment of the constant region are the polypeptides described in any one of (c1) to (c5) below:
   (c1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
   (c2) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of SEQ ID NO: 1, a polypeptide consisting of an amino acid sequence having sequence identity of 90% or more with the amino acid sequence of SEQ ID NO: 1 or a polypeptide consisting of an amino acid sequence in which one or some amino acids have been substituted, inserted, deleted and/or added in the amino acid sequence of SEQ ID NO: 1;
   (c3) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11;
   (c4) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11; and
   (c5) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11.
7. The modified TCR according to any one of 1 to 6 above, wherein the first polypeptide comprises at least one selected from AB loop, C strand, DE loop and F strand of the constant region of human TCRα.
8. The modified TCR according to any one of 1 to 7 above, wherein the second polypeptide comprises at least one selected from Helix3, CC loop, Helix4 F strand and FG loop of the constant region of human TCRβ.
9. The modified TCR according to any one of 1 to 8 above, wherein the first polypeptide comprises at least a part of the extracellular domain, the transmembrane domain and the intracellular domain of the constant region of human TCRα, and the second polypeptide comprises at least a part of the extracellular domain, the transmembrane domain and the intracellular domain of the constant region of human TCRβ.
10. A modified TCR comprising a first polypeptide and a second polypeptide,
   wherein the first polypeptide is a polypeptide which comprises a constant region of human TCRα or a fragment of the constant region and which does not comprise a variable region of human TCRα, and
   the second polypeptide is a polypeptide which comprises a constant region of human TCRβ or a fragment of the constant region and which does not comprise a variable region of human TCRβ, and
   wherein the proportion of CD3-positive cells in pluripotent stem cells, hematopoietic stem and progenitor cells or endogenous TCRα gene- and endogenous TCRβ gene-knockdown or knockout T cells into which the modified TCR has been introduced is 1% or more.
11. The modified TCR according to any one of 1 to 10 above, wherein the proportion of CD3-positive cells in pluripotent stem cells, hematopoietic stem and progenitor cells or endogenous TCRα gene- and endogenous TCRβ gene-knockdown or knockout T cells into which the modified TCR has been introduced is two times or more higher than the proportion of CD3-positive cells in corresponding cells into which the modified TCR has not been introduced.
12. The modified TCR according to any one of 1 to 11 above, wherein a pluripotent stem cell or a hematopoietic stem and progenitor cell into which the modified TCR has been introduced exhibits an equivalent or higher T-cell differentiation capacity as compared to that of a corresponding cell into which the full-length TCR has been introduced.
13. The modified TCR according to any one of 1 to 12 above, wherein the alloreactivity of a non-T cell-derived pluripotent stem cell, a hematopoietic stem and progenitor cell or an endogenous TCRα gene- and endogenous TCRβ gene-knockdown or knockout T cell into which the modified TCR has been introduced is lower than that of a corresponding cell into which the full-length TCR has been introduced.
14. The modified TCR according to any one of 1 to 13 above which is capable of holding a CD3 subunit on cell membrane.
15. The modified TCR according to 14 above which is capable of transmitting a TCR/CD3 complex-related signal into cytoplasm.
16. The modified TCR according to 14 or 15 above which is capable of transmitting a TCR/CD3 complex-related signal into cytoplasm through the CD3 subunit held on the cell membrane.
17. The modified TCR according to 16 above which transmits a TCR/CD3 complex-related signal into cytoplasm through the CD3 subunit held on the cell membrane and thus activates a T cell.
18. The modified TCR according to any one of 1 to 17 above for the expression in a pluripotent stem cell, a hematopoietic stem and progenitor cell or an endogenous TCRα gene- and endogenous TCRβ gene-knockdown or knockout T cell.
19. The modified TCR according to 18 above for the expression in a pluripotent stem cell or a hematopoietic stem and progenitor cell to differentiate the cell into a T cell.
20. The modified TCR according to 18 above for the expression in a pluripotent stem cell, a hematopoietic stem and progenitor cell or an endogenous TCRα gene- and endogenous TCRβ gene-knockdown or knockout T cell to produce a T cell which is capable of responding to stimulation through a complex of the modified TCR and CD3.
21. The modified TCR according to any one of 1 to 20 above, wherein the pluripotent stem cell is a non-T cell-derived pluripotent stem cell or an endogenous TCRα gene- and endogenous TCRβ gene-knockdown or knockout T cell-derived pluripotent stem cell.
22. A cell which expresses the modified TCR according to any one of 1 to 21 above.
23. The cell according to 22 above, wherein the cell which expresses the modified TCR is a T cell.
24. A pharmaceutical composition comprising a cell which expresses the modified TCR according to any one of 1 to 21 above.
25. A production method of a cell which expresses a modified TCR, wherein the modified TCR comprises a first polypeptide and a second polypeptide, and the production method comprises the following steps (a) to (c):
   (a) a step of preparing a cell;
   (b) a step of preparing an expression vector comprising a polynucleotide encoding the first polypeptide which comprises a constant region of human TCRα or a fragment of the constant region and which does not comprise a variable region of human TCRα and an expression vector comprising a polynucleotide encoding the second polypeptide which comprises a constant region of human TCRβ or a fragment of the constant region and which does not comprise a variable region of human TCRβ; and
   (c) a step of transforming the cell prepared in the step (a) using the expression vectors prepared in the step (b).
26. A production method of a cell which expresses a modified TCR, wherein the modified TCR comprises a first polypeptide and a second polypeptide, and the production method comprises the following steps (a') to (c'):
   (a') a step of preparing a cell;
   (b') a step of preparing an expression vector comprising a polynucleotide encoding the first polypeptide which comprises a constant region of human TCRα or a fragment of the constant region and which does not comprise a variable region of human TCRα and a polynucleotide encoding the second polypeptide which comprises a constant region of human TCRβ or a fragment of the constant region and which does not comprise a variable region of human TCRβ; and
   (c') a step of transforming the cell prepared in the step (a') using the expression vector prepared in the step (b').
27. The production method according to 25 or 26 above, wherein the cell in the step (a) or (a') is a pluripotent stem cell, a hematopoietic stem and progenitor cell or a TCRα and TCRβ genes-knockdown or knockout T cell.
28. The production method according to any one of 25 to 27 above, wherein the cell which expresses the modified TCR is a T cell for an immune cell therapy.
29. A method for producing a T cell comprising a step of expressing the modified TCR according to any one of 1 to 21 above in a pluripotent stem cell or a hematopoietic stem and progenitor cell and differentiating the cell into a T cell.
30. A method for producing a T cell comprising a step of expressing the modified TCR according to any one of 1 to 21 above in a pluripotent stem cell, a hematopoietic stem and progenitor cell or an endogenous TCRα gene- and endogenous TCRβ gene-knockdown or knockout T cell, wherein the obtained T cell is a T cell which is capable of responding to stimulation through a complex of the modified TCR and CD3.
31. A method of immune cell therapy comprising a step of expressing the modified TCR according to any one of 1 to 21 above in a pluripotent stem cell and differentiating the cell into a T cell and a step of administering the obtained T cell to a subject.
32. A method of immune cell therapy comprising a step of expressing the modified TCR according to any one of 1 to 21 above in an endogenous TCRα gene- and endogenous TCRβ gene-knockdown or knockout T cell and administering the obtained T cell to a subject.
33. A method of immune cell therapy comprising a step of expressing the modified TCR according to any one of 1 to 21 above in a hematopoietic stem and progenitor cell and differentiating the cell into a T cell and a step of administering the obtained T cell to a subj ect.
34. The modified TCR according to any one of 1 to 21 above for use in an immune cell therapy.
35. A pluripotent stem cell T cell for use in an immune cell therapy which is obtained by expressing the modified TCR according to any one of 1 to 21 above in a pluripotent stem cell and differentiating the cell into a T cell.
36. A T cell for use in an immune cell therapy which is obtained by expressing the modified TCR according to any one of 1 to 21 above in an endogenous TCRα gene- and endogenous TCRβ gene-knockdown or knockout T cell.
37. A hematopoietic stem and progenitor cell-derived T cell for use in an immune cell therapy which is obtained by expressing the modified TCR according to any one of 1 to 21 above in a hematopoietic stem and progenitor cell and differentiating the cell into a T cell.
38. Use of the modified TCR according to any one of 1 to 21 above for the manufacture of a composition for an immune cell therapy.
39. Use of a pluripotent stem cell-derived T cell for the manufacture of a composition for an immune cell therapy, wherein the pluripotent stem cell-derived T cell is obtained by expressing the modified TCR according to any one of 1 to 21 above in a pluripotent stem cell and differentiating the cell into a T cell.
40. Use of a T cell for the manufacture of a composition for an immune cell therapy, wherein the T cell is obtained by expressing the modified TCR according to any one of 1 to 21 above in an endogenous TCRα gene- and endogenous TCRβ gene-knockdown or knockout T cell.
41. Use of a T cell for the manufacture of a composition for an immune cell therapy, wherein the T cell is obtained by expressing the modified TCR according to any one of 1 to 21 above in a hematopoietic stem and progenitor cell and differentiating the cell into a T cell.
42. A nucleic acid encoding the modified TCR according to any one of 1 to 21 above.
43. A vector comprising the nucleic acid according to 42 above.
44. A production method of a pharmaceutical composition using the nucleic acid according to 42 above or the vector according to 43 above.
45. A pharmaceutical composition comprising the nucleic acid according to 42 above or the vector according to 43 above.

### Advantageous Effects of the Invention

The modified TCR of the invention comprises a first polypeptide and a second polypeptide. Because the first polypeptide comprises the constant region of human TCRα or a fragment of the constant region and because the second polypeptide comprises the constant region of human TCRβ or a fragment of the constant region, CD3 subunits can be held on the cell membrane. In the modified TCR of the invention, because the first polypeptide and the second polypeptide do not comprise the variable region of TCRα and that of TCRβ, respectively, the antigen recognition ability is lost. When the modified TCR of the invention is expressed in a T cell in which the endogenous TCR expression has been inhibited or eliminated, a T cell which is capable of responding to stimulation through a modified TCR/CD3 complex without causing the antigen responsiveness can be produced.

### Brief Description of Drawings

[Fig. 1] Fig. 1 is a schematic figure of full-length and modified TCR sequences.
[Fig. 2] Fig. 2 is a figure showing the amino acid sequence information of ba1.
[Fig. 3] Fig. 3 is a figure showing the amino acid sequence information of ba2 to ba9.
[Fig. 4] Fig. 4 is a figure showing the amino acid sequence information of ba10 to ba18.
[Fig. 5] Fig. 5 is a figure showing a flow cytometry analysis of the expression of CD3E in CD3-stably expressing 293T cells into which ba vectors and mock vector were introduced.
[Fig. 6] Fig. 6 is a figure showing the expression levels of CD3E in CD3-stably expressing 293T cells into which ba vectors and mock vector were introduced, where the expression levels were quantified by MFI.
[Fig. 7] Fig. 7 is a figure showing a flow cytometry analysis of the expression of CD3D in CD3-stably expressing 293T cells into which ba vectors and mock vector were introduced.
[Fig. 8] Fig. 8 is a figure showing the expression levels of CD3D in CD3-stably expressing 293T cells into which ba vectors and mock vector were introduced, where the expression levels were quantified by MFI.
[Fig. 9] Fig. 9 is a figure showing a flow cytometry analysis of the expression of CD3E in endogenous TCR-knockout Jurkat cells into which ba vectors and mock vector were introduced.
[Fig. 10] Fig. 10 is a figure showing the expression levels of CD3E in endogenous TCR-knockout Jurkat cells into which ba vectors and mock vector were introduced, where the expression levels were quantified by MFI.
[Fig. 11] Fig. 11 is a figure showing a flow cytometry analysis of the expression of CD3D in endogenous TCR-knockout Jurkat cells into which ba vectors and mock vector were introduced.
[Fig. 12] Fig. 12 is a figure showing the expression levels of CD3D in endogenous TCR-knockout Jurkat cells into which ba vectors and mock vector were introduced, where the expression levels were quantified by MFI.
[Fig. 13] Fig. 13 is a figure showing the change in responsiveness to OKT3 antibody stimulation by knockout of endogenous TCR which was analyzed by flow cytometry using the increase in the expression of CD69, which is an activated T cell marker as an indicator.
[Fig. 14] Fig. 14 is a figure showing a flow cytometry analysis of the expression of CD69 in endogenous TCR-knockout Jurkat cells into which ba vectors and mock vector were introduced and which were subjected to OKT3 antibody stimulation.
[Fig. 15] Fig. 15 is a figure showing the expression levels of CD69 in endogenous TCR-knockout Jurkat cells into which ba vectors and mock vector were introduced and which were subjected to OKT3 antibody stimulation, where the expression levels were quantified by MFI.
[Fig. 16] Fig. 16 shows partial sequences at the N-terminus of amino acid sequences of the extracellular domains of modified TCRαs and a schematic figure of the structure of the amino acid sequences. The amino acid sequence to which * is added at the N-terminus is a part of a known human TCRα amino acid sequence (shown in Protein Data Bank ID: 3QJF; https://www.rcsb.org/pdb/explore/remediatedSequence.do?structureId=3QJF).
[Fig. 17] Fig. 17 shows partial sequences at the N-terminus of amino acid sequences of the extracellular domains of modified TCRβs and a schematic figure of the structure of the amino acid sequences. The amino acid sequence to which * is added at the N-terminus is a part of a known human TCRβ amino acid sequence (shown in Protein Data Bank ID: 3QJF; https://www.rcsb.org/pdb/explore/remediatedSequence.do?structureId=3QJF).
[Fig. 18] Fig. 18 shows the average values (n=3) of CD3-positive proportions in cells into which modified TCR expression vectors were introduced.
[Fig. 19] Fig. 19(A) is a figure showing the analysis results of the expression of CD3 and CD45 in FF-WJs524. Fig. 19(B) is a figure showing the analysis results of the expression of CD8β and CD8α in FF-WJs524.
[Fig. 20] Fig. 20(A) is a figure showing the results of culture on DLL4 and Retronectin for 21 days. Fig. 20(B) is a figure showing the analysis results of the expression of CD8β and CD8α.

### Description of Embodiments

### [Modified TCR]

The modified TCR of the invention is a modified TCR comprising a first polypeptide and a second polypeptide and is characterized in that the first polypeptide is a polypeptide which comprises the constant region of human TCRα or a fragment of the constant region and which does not comprise the variable region of human TCRα and that the second polypeptide is a polypeptide which comprises the constant region of human TCRβ or a fragment of the constant region and which does not comprise the variable region of human TCRβ.

The TCRα gene locus is composed of a variable region (V region), a joining region (J region) and a constant region (C region). The TCRβ gene locus is composed of a V region, a diversity region (D region), a J region and a C region. The gene loci of TCR are rearranged on the genome during the differentiation process of a T cell.

The V region and the J region in TCRα and the V region, the D region and the J region in TCRβ are each connected with various combinations, and random nucleotides are inserted or deleted at the connection parts. As a result, functional and highly diverse variable part exons are created.

Moreover, during the process of gene transcription, the variable part exon is connected to the C region exon by RNA splicing, and thus mRNA of a full-length TCR gene is formed. Then, a TCRα chain polypeptide and a TCRβ chain polypeptide translated as proteins form a heterodimer, and TCR is presented on the surface of cell membrane.

The TCRα chain polypeptide and the TCRβ chain polypeptide each have a variable region and a constant region, which are extracellular domains, and a transmembrane domain and an intracellular domain. A constant region in the invention below indicates a C region sequence in a gene and a sequence excluding the variable region domain (a non-variable region) in a polypeptide.

It is known that while there is one kind of constant region gene for TCRα (TRAC gene), there are two kinds of constant region gene for TCRβ (TRBC1 gene and TRBC2 gene). During the rearrangement of the TCRβ gene locus, one gene of TRBC1 and TRBC2 is selected. Based on the sequence information extracted from IMGT (the international ImMunoGeneTics information system), the amino acid sequences of TRBC1 gene and TRBC2 gene are different only by five amino acids, and the C-terminus of TRBC2 has only two more amino acids as compared to TRBC1. The sequence homology of the two is high, and no functional difference has been known.

Examples of the amino acid sequence of the constant region of human TCRα include the amino acid sequence of SEQ ID NO: 1 and the like.

The amino acid sequence of the fragment of the constant region of human TCRα may be any amino acid sequence as long as the amino acid sequence is a partial sequence of the constant region of human TCRα, but examples include the amino acid sequence of any one of SEQ ID NOs: 2 to 11 and the like.

An embodiment of the constant region of human TCRα or the fragment of the constant region in the invention is the polypeptide described in any one of (A1) to (A3) below:
(A1) a polypeptide consisting of the amino acid sequence of any one of SEQ ID NOs: 1 to 11;
(A2) a polypeptide consisting of an amino acid sequence having sequence identity of 90% or more with the amino acid sequence of any one of SEQ ID NOs: 1 to 11; and
(A3) a polypeptide consisting of an amino acid sequence in which one or some amino acids have been substituted, inserted, deleted and/or added in the amino acid sequence of any one of SEQ ID NOs: 1 to 11.

Examples of the amino acid sequence of the constant region of human TCRβ include the amino acid sequence of SEQ ID NO: 12 and the like.

The amino acid sequence of the fragment of the constant region of human TCRβ in the invention may be any amino acid sequence as long as the amino acid sequence is a partial sequence of the constant region of human TCRβ, but examples include the amino acid sequence of any one of SEQ ID NOs: 13 to 24 and the like.

An embodiment of the constant region of human TCRβ or the fragment of the constant region is the polypeptide described in any one of (B1) to (B3) below:
(B1) a polypeptide consisting of the amino acid sequence of any one of SEQ ID NOs: 12 to 24;
(B2) a polypeptide consisting of an amino acid sequence having sequence identity of 90% or more with the amino acid sequence of any one of SEQ ID NOs: 12 to 24; and
(B3) a polypeptide consisting of an amino acid sequence in which one or some amino acids have been substituted, inserted, deleted and/or added in the amino acid sequence of any one of SEQ ID NOs: 12 to 24.

As long as the effects of the invention are exhibited, in the modified TCR of the invention, the length of the amino acid sequence of the constant region of human TCRα or the fragment of the constant region or that of the constant region of human TCRβ or the fragment of the constant region in the above embodiments may be changed, and those in which mutations by Single Nucleotide Polymorphisms (SNPs) derived from human TCR have been introduced are also included. As long as the effects of the invention are exhibited, the modified TCR of the invention also includes those further having known modification (for example, modification described in Michael S. Kuhns et al., Immunity. 26: 357-369, 2007, Aswin Natarajan et al., Cell Reports. 14: 2833-2845, 2016 or Schamel W. Wolfgang et al. Immunological Reviews. 291: 8-25, 2019) for the purpose of functional improvement of TCR.

A polypeptide having an amino acid sequence in which one to some amino acids have been substituted, inserted, deleted and/or added in a target amino acid sequence can be obtained by introducing a site-specific mutation for example to DNA that encodes a polypeptide comprising an amino acid sequence of SEQ ID NOs: 1 to 24 using the site-directed mutagenesis (described in Molecular Cloning, A Laboratory Manual, Second Edition, Cold Spring Harbor Laboratory Press, 1989, Current Protocols in Molecular Biology, John Wiley & Sons. 1987-1997, Nucleic Acids Research. 10, 6487, 1982, Proc. Natl. Acad. Sci. USA. 79, 6409, 1982, Gene, 34: 315, 1985, Nucleic Acids Research. 13: 4431 ,1985 or Proc. Natl. Acad. Sci. USA. 82: 488 ,1985) or the like.

The range of one to some in the substitution, insertion, deletion and/or addition of one to some amino acids in an amino acid sequence is not particularly limited, but, for example, when 100 amino acids in the amino acid sequence are counted as one unit, the range means 1, 2, 3, 4, 5, 6, 7, 8, 9, 10, 11, 12, 13, 14, 15, 16, 17, 18, 19 or 20 residues per unit, preferably around 1, 2, 3, 4, 5, 6, 7, 8, 9 or 10 residues, more preferably around 1, 2, 3, 4 or 5 residues.

The deletion of an amino acid means deletion or disappearance of the amino acid residue in the sequence, and the substitution of an amino acid means that the amino acid residue in the sequence is substituted with another amino acid residue. The insertion or the addition of an amino acid means that a new amino acid residue is additionally inserted or added before or after the sequence or in the sequence.

An example of a specific embodiment of substitution of one to some amino acids is an embodiment in which one to some amino acids are replaced with other chemically similar amino acids. Examples include a case in which a hydrophobic amino acid is substituted with another hydrophobic amino acid, a case in which a polar amino acid is substituted with another polar amino acid having the same charge and the like. Such chemically similar amino acids are known for each amino acid in this technical field.

Specific examples of nonpolar (hydrophobic) amino acids include alanine, valine, glycine, isoleucine, leucine, proline, tryptophan, phenylalanine, methionine and the like. Specific examples of polar (neutral) amino acids include serine, threonine, tyrosine, glutamine, asparagine, cysteine and the like. Specific examples of basic amino acids with a positive charge include arginine, histidine, lysine and the like. Specific examples of acidic amino acids with a negative charge include aspartic acid, glutamic acid and the like.

An amino acid sequence in which one to some amino acids have been substituted, inserted, deleted and/or added in the amino acid sequence which a subject protein has is an amino acid sequence having certain or higher sequence identity with the amino acid sequence that the subject protein has, and an example includes an amino acid sequence having sequence identity of 60% or higher, preferably 65% or higher, preferably 70% or higher, preferably 75% or higher, preferably 80% or higher, preferably 85% or higher, more preferably 90% or higher, further preferably 95% or higher with the amino acid sequence which the subject protein has.

A preferable embodiment of the modified TCR of the invention is a modified TCR in which the constant region of human TCRα or the fragment of the constant region and the constant region of human TCRβ or the fragment of the constant region are the polypeptides described in any one of (a1) to (a8) below:
(a1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
(a2) the constant region of human TCRβ or the fragment of the constant region is a polypeptide consisting of the amino acid sequence of SEQ ID NO: 13, a polypeptide consisting of an amino acid sequence having sequence identity of 90% or more with the amino acid sequence of SEQ ID NO: 13 or a polypeptide consisting of an amino acid sequence in which one or some amino acids have been substituted, inserted, deleted and/or added in the amino acid sequence of SEQ ID NO: 13, and the constant region of human TCRα or the fragment of the constant region is a polypeptide consisting of the amino acid sequence of SEQ ID NO: 1 or 2, a polypeptide consisting of an amino acid sequence having sequence identity of 90% or more with the amino acid sequence of SEQ ID NO: 1 or 2 or a polypeptide consisting of an amino acid sequence in which one or some amino acids have been substituted, inserted, deleted and/or added in the amino acid sequence of SEQ ID NO: 1 or 2;
(a3) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of any one of SEQ ID NOs: 1 to 3 and 5 to 11, a polypeptide consisting of an amino acid sequence having sequence identity of 90% or more with the amino acid sequence of any one of SEQ ID NOs: 1 to 3 and 5 to 11 or a polypeptide consisting of an amino acid sequence in which one or some amino acids have been substituted, inserted, deleted and/or added in the amino acid sequence of any one of SEQ ID NOs: 1 to 3 and 5 to 11;
(a4) the constant region of human TCRβ or the fragment of the constant region is a polypeptide consisting of the amino acid sequence of SEQ ID NO: 19, a polypeptide consisting of an amino acid sequence having sequence identity of 90% or more with the amino acid sequence of SEQ ID NO: 19 or a polypeptide consisting of an amino acid sequence in which one or some amino acids have been substituted, inserted, deleted and/or added in the amino acid sequence of SEQ ID NO: 19, and the constant region of human TCRα or the fragment of the constant region is a polypeptide consisting of the amino acid sequence of SEQ ID NO: 10 or 11, a polypeptide consisting of an amino acid sequence having sequence identity of 90% or more with the amino acid sequence of SEQ ID NO: 10 or 11 or a polypeptide consisting of an amino acid sequence in which one or some amino acids have been substituted, inserted, deleted and/or added in the amino acid sequence of SEQ ID NO: 10 or 11;
(a5) the constant region of human TCRβ or the fragment of the constant region is a polypeptide consisting of the amino acid sequence of SEQ ID NO: 21, a polypeptide consisting of an amino acid sequence having sequence identity of 90% or more with the amino acid sequence of SEQ ID NO: 21 or a polypeptide consisting of an amino acid sequence in which one or some amino acids have been substituted, inserted, deleted and/or added in the amino acid sequence of SEQ ID NO: 21, and the constant region of human TCRα or the fragment of the constant region is a polypeptide consisting of the amino acid sequence of any one of SEQ ID NOs: 7 to 11, a polypeptide consisting of an amino acid sequence having sequence identity of 90% or more with the amino acid sequence of any one of SEQ ID NOs: 7 to 11 or a polypeptide consisting of an amino acid sequence in which one or some amino acids have been substituted, inserted, deleted and/or added in the amino acid sequence of any one of SEQ ID NOs: 7 to 11;
(a6) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11;
(a7) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11; and
(a8) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11.

The modified TCR with the polypeptides of (a1) to (a8) above is further preferably a modified TCR with the polypeptides described in any one of (b1) to (b7) below:
(b1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
(b2) the constant region of human TCRβ or the fragment of the constant region is a polypeptide consisting of the amino acid sequence of SEQ ID NO: 13, a polypeptide consisting of an amino acid sequence having sequence identity of 90% or more with the amino acid sequence of SEQ ID NO: 13 or a polypeptide consisting of an amino acid sequence in which one or some amino acids have been substituted, inserted, deleted and/or added in the amino acid sequence of SEQ ID NO: 13, and the constant region of human TCRα or the fragment of the constant region is a polypeptide consisting of the amino acid sequence of SEQ ID NO: 2, a polypeptide consisting of an amino acid sequence having sequence identity of 90% or more with the amino acid sequence of SEQ ID NO: 2 or a polypeptide consisting of an amino acid sequence in which one or some amino acids have been substituted, inserted, deleted and/or added in the amino acid sequence of SEQ ID NO: 2;
(b3) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of any one of SEQ ID NOs: 1 to 3 and 10, a polypeptide consisting of an amino acid sequence having sequence identity of 90% or more with the amino acid sequence of any one of SEQ ID NOs: 1 to 3 and 10 or a polypeptide consisting of an amino acid sequence in which one or some amino acids have been substituted, inserted, deleted and/or added in the amino acid sequence of any one of SEQ ID NOs: 1 to 3 and 10;
(b4) the constant region of human TCRβ or the fragment of the constant region is a polypeptide consisting of the amino acid sequence of SEQ ID NO: 21, a polypeptide consisting of an amino acid sequence having sequence identity of 90% or more with the amino acid sequence of SEQ ID NO: 21 or a polypeptide consisting of an amino acid sequence in which one or some amino acids have been substituted, inserted, deleted and/or added in the amino acid sequence of SEQ ID NO: 21, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11;
(b5) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 4 and 6 to 11, a polypeptide consisting of an amino acid sequence having sequence identity of 90% or more with the amino acid sequence of any one of SEQ ID NOs: 4 and 6 to 11 or a polypeptide consisting of an amino acid sequence in which one or some amino acids have been substituted, inserted, deleted and/or added in the amino acid sequence of any one of SEQ ID NOs: 4 and 6 to 11;
(b6) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 4 and 7 to 11, a polypeptide consisting of an amino acid sequence having sequence identity of 90% or more with the amino acid sequence of any one of SEQ ID NOs: 4 and 7 to 11 or a polypeptide consisting of an amino acid sequence in which one or some amino acids have been substituted, inserted, deleted and/or added in the amino acid sequence of any one of SEQ ID NOs: 4 and 7 to 11; and
(b7) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 4 and 6 to 11, a polypeptide consisting of an amino acid sequence having sequence identity of 90% or more with the amino acid sequence of any one of SEQ ID NOs: 4 and 6 to 11 or a polypeptide consisting of an amino acid sequence in which one or some amino acids have been substituted, inserted, deleted and/or added in the amino acid sequence of any one of SEQ ID NOs: 4 and 6 to 11.

The modified TCR with the polypeptides of (b1) to (b7) above is further preferably a modified TCR with the polypeptides described in any one of (c1) to (c5) below:
(c1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
(c2) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of SEQ ID NO: 1, a polypeptide consisting of an amino acid sequence having sequence identity of 90% or more with the amino acid sequence of SEQ ID NO: 1 or a polypeptide consisting of an amino acid sequence in which one or some amino acids have been substituted, inserted, deleted and/or added in the amino acid sequence of SEQ ID NO: 1;
(c3) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11;
(c4) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11; and
(c5) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11.

In the modified TCR of the invention, the first polypeptide preferably comprises at least one selected from AB loop, C strand, DE loop and F strand of the constant region of human TCRα.

In the constant region of human TCRα, AB loop, C strand, DE loop and F strand are preferably comprised in this order but may be comprised in a different order.

AB loop is, for example, a structure consisting of the amino acid sequence of the 14th to 20th residues of the amino acid sequence of SEQ ID NO: 1. C strand is, for example, a structure consisting of the amino acid sequence of the 29th to 40th residues of the amino acid sequence of SEQ ID NO: 1. DE loop is, for example, a structure consisting of the amino acid sequence of the 52nd to 57th residues of the amino acid sequence of SEQ ID NO: 1. F strand is, for example, a structure consisting of the amino acid sequence of 67th to 81st residues of the amino acid sequence of SEQ ID NO: 1.

In the modified TCR of the invention, the second polypeptide preferably comprises at least one selected from Helix3, CC loop, Helix4 F strand and FG loop of the constant region of human TCRβ.

In the constant region of human TCRβ, Helix3, CC loop, Helix4 F strand and FG loop are preferably comprised in this order but may be comprised in a different order.

Helix3 is, for example, a structure consisting of the amino acid sequence of the 20th to 26th residues of the amino acid sequence of SEQ ID NO: 12. CC loop is, for example, a structure consisting of the amino acid sequence of the 49th to 54th residues of the amino acid sequence of SEQ ID NO: 12. Helix4 F Strand is, for example, a structure consisting of the amino acid sequence of the 88th to 93th residues of the amino acid sequence of SEQ ID NO: 12. FG loop is, for example, a structure consisting of the amino acid sequence of the 105th to 113th residues of the amino acid sequence of SEQ ID NO: 12.

In the modified TCR of the invention, the first polypeptide preferably comprises at least a part of the extracellular domain, the transmembrane domain and the intracellular domain of the constant region of human TCRα, and the second polypeptide preferably comprises at least a part of the extracellular domain, the transmembrane domain and the intracellular domain of the constant region of human TCRβ.

The extracellular domain of the constant region of human TCRα is, for example, a region consisting of the amino acid sequence of the 1st to 117th residues of the amino acid sequence of SEQ ID NO: 1. The transmembrane domain of the constant region of human TCRα is, for example, a domain consisting of the amino acid sequence of the 118th to 139th residues of the amino acid sequence of SEQ ID NO: 1. A preferable embodiment where at least a part of the extracellular domain of the constant region of human TCRα is comprised is, for example, an embodiment where at least the 82th to 117th amino acids in the region consisting of the amino acid sequence of the 1st to 117th residues of the amino acid sequence of SEQ ID NO: 1 is comprised. The intracellular domain of the constant region of human TCRα is, for example, a domain consisting of the amino acid sequence of the 140th and 141th residues of the amino acid sequence of SEQ ID NO: 1.

The extracellular domain of the constant region of human TCRβ is, for example, a region consisting of the amino acid sequence of the 1st to 146th residues of the amino acid sequence of SEQ ID NO: 12. The transmembrane domain of the constant region of human TCRβ is, for example, a domain consisting of the amino acid sequence of the 147th to 172nd residues of the amino acid sequence of SEQ ID NO: 12. A preferable embodiment comprising at least a part of the extracellular domain of the constant region of human TCRβ is, for example, an embodiment comprising at least the 114th to 146th amino acids in the domain consisting of the amino acid sequence of the 1st to 146th residues of the amino acid sequence of SEQ ID NO: 12. The intracellular domain of the constant region of human TCRβ is, for example, a domain consisting of the amino acid sequence of the 173rd to 179th residues of the amino acid sequence of SEQ ID NO: 12.

Moreover, a polypeptide consisting of an amino acid sequence of a constant region of human TCRα or a fragment of the constant region or an amino acid sequence of a constant region of human TCRβ or a fragment of the constant region which is disclosed in known database such as Protein Data Bank (for example, shown in Protein Data Bank ID: 3QJF; https://www.rcsb.org/pdb/explore/remediatedSequence.do?structureId=3QJF) is also included in the polypeptides of the invention.

The cysteine residues comprised in the amino acid sequences of the constant regions of wild-type TCRα and wild-type TCRβ form a disulfide bond and bind the two chains. In the modified TCR of the invention, the first polypeptide and the second polypeptide may each similarly have an additional cysteine residue to form a disulfide bond in the amino acid sequences of the constant regions or the fragments of the constant regions. The first polypeptide and the second polypeptide may be further modified in a manner that an additional disulfide bond is formed in a chain or/and between the chains, and the stability of the modified TCR can be thus increased.

When the modified TCR of the invention has the structure described above, the modified TCR can associate with CD3 of a mammal having three different chains (γ, δ and ε) and CD3ζ chain and hold the CD3 subunits on the cell membrane. The CD3 subunits play an important role in the signal transduction from TCR to the cytoplasm, and the modified TCR of the invention transmits a TCR/CD3 complex-related signal into the cytoplasm through the CD3 subunits held on the cell membrane and activates the T cell.

The proportion of CD3-positive cells in pluripotent stem cells, hematopoietic stem and progenitor cells or endogenous TCRα gene- and endogenous TCRβ gene-knockdown or knockout T cells into which the modified TCR of the invention has been introduced is preferably 1% or more, 3% or more, 5% or more, 10% or more or 15% or more, more preferably 20% or more, further preferably 40% or more, particularly preferably 60% or more, especially preferably 75% or more, most preferably 80% or more. When the proportion of the CD3-positive cells is at least 1% or more, sufficient response to stimulation can be obtained through the modified TCR/CD3 complex. The proportion of the CD3-positive cells can be assessed by examining the proportion of CD3D-positive cells or CD3E-positive cells using flow cytometry as described below in the Examples.

To obtain sufficient response to stimulation through the modified TCR/CD3 complex, the proportion of CD3-positive cells in pluripotent stem cells, hematopoietic stem and progenitor cells or endogenous TCRα gene- and endogenous TCRβ gene-knockdown or knockout T cells into which the modified TCR of the invention has been introduced is preferably two times or more higher, more preferably five times or more higher, further preferably 10 times or more higher, particularly preferably 20 times or more higher than the proportion of CD3-positive cells in corresponding cells.

A pluripotent stem cell or a hematopoietic stem and progenitor cell into which the modified TCR of the invention has been introduced preferably exhibits an equivalent or higher T-cell differentiation capacity as compared to that of a corresponding cell into which the full-length TCR (wild-type TCR) has been introduced. The T-cell differentiation capacity can be assessed by the method of determining whether a T cell maintains the undifferentiated state described below.

The antigen recognition site formed with the variable regions of the TCRα/TCRβ heterodimer is thought to recognize whether a target cell is an autologous cell or a non-autologous cell. The modified TCR of the invention does not comprise the variable regions of TCRα and TCRβ in the first polypeptide and the second polypeptide. The antigen recognition ability is lost, and the alloreactivity is reduced or eliminated/lost.

The modified TCR of the invention may comprise a signal peptide at the N-terminus.

The modified TCR of the invention can be preferably used for applications: for expressing in a pluripotent stem cell, a hematopoietic stem and progenitor cell or an endogenous TCRα gene- and endogenous TCRβ gene-knockdown or knockout T cell; for expressing in a pluripotent stem cell or a hematopoietic stem and progenitor cell and differentiating the cell into a T cell; for expressing in a pluripotent stem cell, a hematopoietic stem and progenitor cell or an endogenous TCRα gene- and endogenous TCRβ gene-knockdown or knockout T cell and producing a T cell which is capable of responding to stimulation through the modified TCR/CD3 complex; and the like.

### [Cells Expressing Modified TCR and Production Method Thereof]

In the invention, the method for introducing the modified TCR into a cell for producing a cell which expresses the modified TCR is not particularly limited, and a known method can be used appropriately. Examples thereof include a method for introducing the modified TCR into a cell in the form of a polynucleotide encoding the modified TCR, a method for introducing the modified TCR into a cell in the form of a protein and the like.

An embodiment of the production method for introducing the modified TCR into a cell in the form of a polynucleotide encoding the modified TCR is a method comprising the steps (a) to (c) or the steps (a') to (c') below.

(a) A step of preparing a cell;
(b) a step of preparing an expression vector comprising a polynucleotide encoding a first polypeptide which comprises a constant region of human TCRα or a fragment of the constant region and which does not comprise a variable region of human TCRα and an expression vector comprising a polynucleotide encoding a second polypeptide which comprises a constant region of human TCRβ or a fragment of the constant region and which does not comprise a variable region of human TCRβ; and
(c) a step of transforming the cell prepared in the step (a) using the expression vectors prepared in the step (b).

(a') A step of preparing a cell;
(b') a step of preparing an expression vector comprising a polynucleotide encoding a first polypeptide which comprises a constant region of human TCRα or a fragment of the constant region and which does not comprise a variable region of human TCRα and a polynucleotide encoding a second polypeptide which comprises a constant region of human TCRβ or a fragment of the constant region and which does not comprise a variable region of human TCRβ; and
(c') a step of transforming the cell prepared in the step (a') using the expression vector prepared in the step (b').

The kinds of the polynucleotides are not particularly limited and can be selected depending on the gene introduction method, and the polynucleotides may be DNA or RNA.

Examples of the polynucleotide encoding the constant region of human TCRα or the fragment of the constant region include the polynucleotides described in (i) to (v) below and the like:
(i) a polynucleotide consisting of the nucleotide sequence of any one of SEQ ID NOs: 25 to 35;
(ii) a polynucleotide having sequence identity of 90% or more with the nucleotide sequence of any one of SEQ ID NOs: 25 to 35;
(iii) a polynucleotide consisting of a nucleotide sequence which hybridizes with the nucleotide sequence of any one of SEQ ID NOs: 25 to 35 or the complementary nucleotide sequence of the nucleotide sequence under a stringent condition;
(iv) a polynucleotide encoding a polypeptide consisting of the amino acid sequence of any one of SEQ ID NOs: 1 to 11; and
(v) a polynucleotide encoding a polypeptide consisting of an amino acid sequence having sequence identity of 90% or more with the amino acid sequence of any one of SEQ ID NOs: 1 to 11.

Examples of the polynucleotide encoding the constant region of human TCRβ or the fragment of the constant region include the polynucleotides described in (vi) to (x) below and the like:
(vi) a polynucleotide consisting of the nucleotide sequence of any one of SEQ ID NOs: 36 to 48;
(vii) a polynucleotide having sequence identity of 90% or more with the nucleotide sequence of any one of SEQ ID NOs: 36 to 48;
(viii) a polynucleotide consisting of a nucleotide sequence which hybridizes with the nucleotide sequence of any one of SEQ ID NOs: 36 to 48 or the complementary nucleotide sequence of the sequence under a stringent condition;
(ix) a polynucleotide encoding a polypeptide consisting of the amino acid sequence of any one of SEQ ID NOs: 12 to 24; and
(x) a polynucleotide encoding a polypeptide consisting of an amino acid sequence having sequence identity of 90% or more with the amino acid sequence of any one of SEQ ID NOs: 12 to 24.

Examples of a nucleotide sequence that hybridizes under a stringent condition include a nucleotide sequence of a hybridizable polynucleotide that is obtained by a colony hybridization method, a plaque hybridization method, a southern blot hybridization method, a DNA microarray method or the like using a polynucleotide comprising a subject nucleotide sequence as a probe and the like.

Specifically, it is possible to exemplify a nucleotide sequence of DNA or the like that can be identified by washing a filter or a glass slide under the condition of 65°C using a SSC solution with the concentration of 0.1 to 2 times (the composition of the SSC solution with the concentration of 1 time is 150 mmol/L sodium chloride and 15 mmol/L sodium citrate), after performing hybridization (for example, described in Molecular Cloning, A Laboratory Manual, Second Edition, Cold Spring Harbor Laboratory Press, 1989, Current Protocols in Molecular Biology, John Wiley & Sons, 1987-1997 or DNA Cloning 1: Core Techniques, A Practical Approach, Second Edition, Oxford University, 1995) at 65°C in the presence of 0.7 to 1.0 mol/L sodium chloride using a filter or a glass slide on which a DNA sequence derived from a hybridized colony or plaque or a PCR product or DNA oligo having the DNA sequence is fixed.

A polynucleotide comprising a nucleotide sequence which hybridizes under a stringent condition is DNA having certain or higher sequence identity with a nucleotide sequence of a polynucleotide having the nucleotide sequence of a subject gene used as a probe. Examples thereof include DNA having a homology of at least 60% or more to the subject nucleotide sequence, preferably DNA having a homology of 80% or more and further preferably DNA having a homology of 95% or more. When 100 bases in a nucleotide sequence are counted as one unit, an example thereof includes DNA comprising a nucleotide sequence having replacement, insertion, deletion, addition and/or the like of one to some bases, preferably 1 to 40 bases, preferably 1 to 35 bases, preferably 1 to 30 bases, preferably 1 to 25 bases, preferably 1 to 20 bases, more preferably 1 to 15 bases, further preferably 1, 2, 3, 4, 5, 6, 7, 8, 9 or 10 bases, still further preferably 1, 2, 3, 4 or 5 bases per unit in the nucleotide sequence of the subject gene.

The deletion of a base means deletion or disappearance of the base in the sequence, and the replacement of a base means that the base in the sequence is replaced with another base. The insertion or the addition of a base means that a new base is additionally inserted.

Genetic polymorphism is often recognized in a nucleotide sequence of a gene that encodes a protein of a eukaryote. A nucleotide sequence of a gene in which small scale mutations arise in the nucleotide sequence by such polymorphism in the nucleotide sequence of a gene used in the invention is also included in the nucleotide sequence.

The sequence identity in the invention means the identity of two sequences and means that exactly same nucleotides or amino acids are present at same positions in two compared nucleotide or protein sequences which are aligned. Accordingly, when two proteins show identity of 90%, this means that 90% of all the amino acid residues contained in the proteins at the corresponding positions are exactly the same.

A value of sequence identity in the invention may be a value calculated using a sequence identity detection program which is known to one skilled in the art unless otherwise clearly stated, but examples thereof include a value calculated with BLAST (for example, described in J. Mol. Biol., 215, 403, 1990) using the default parameters and the like with respect to nucleotide sequences and a value calculated with BLAST2 (for example, described in Nucleic Acids Res.,25, 3389, 1997, Genome Res., 7, 649, 1997 or http://www.ncbi.nlm.nih.gov/Education/BLASTinfo/information3.html), Pairwise Sequence Alignment based on Needleman-Wunsch algorithm or the like using the default parameters and the like with respect to amino acid sequences.

Regarding the default parameters, for example, G (Cost to open gap) is 5 for a nucleotide sequence and 11 for an amino acid sequence; -E (Cost to extend gap) is 2 for a nucleotide sequence and 1 for an amino acid sequence; -q (Penalty for nucleotide mismatch) is -3; -r (reward for nucleotide match) is 1; -e (expect value) is 10; -W (wordsize) is 11 residues for a nucleotide sequence and 3 residues for an amino acid sequence; -y [Dropoff (X) for blast extensions in bits] is 20 for the blastn and 7 for programs other than the blastn; -X (X dropoff value for gapped alignment in bits) is 15; and -Z (final X dropoff value for gapped alignment in bits) is 50 for the blastn and 25 for programs other than the blastn (for example, described in http://www.ncbi.nlm.nih.gov/blast/htmL/blastcgihelp.htmL).

A gene can be introduced appropriately using a method which is generally used in this field, and the method is not particularly limited. For example, a gene of the invention can be introduced by incorporating the gene in a viral vector such as retrovirus, lentivirus and adenovirus and infecting a cell into which the gene is introduced. Moreover, a gene can be introduced by incorporating the gene into a vector such as nonviral vectors including a plasmid, a bacterial vector, an episomal vector and the like and incorporating the vector into a cell into which the gene is introduced by transfection, electroporation, liposome method, calcium phosphate co-precipitation, DEAE dextran method, microinjection or the like.

A gene of the invention can be inserted to any genome DNA site of a cell, and for example, a gene can be introduced freely using genome editing technology such as transcription activator-like effector nucleases (TALEN) and clustered regulatory interspaced short palindromic repeat (CRISPR)/Cas9, transposon method or the like. Moreover, a gene of the invention can be expressed by introducing into a cell with an artificial chromosome vector or the like.

When more than one gene, such as a gene encoding the constant region of human TCRα or a fragment of the constant region and a gene encoding the constant region of human TCRβ or a fragment of the constant region, are incorporated, the genes can be arranged under the regulation of independent promotors and inserted into a same vector or separate vectors. Moreover, the genes can be linked through an intervening sequence to form an expression cassette using a single promoter.

The intervening sequence is not limited, but examples include an internal ribosome entry site (IRES) sequence, a 2A peptide sequence and the like (described in Szymczak et al., Expert Opin. Biol. Ther., 2005, 5: 627-638). A 2A peptide is a virus-derived peptide sequence of about 20 amino acid residues. When genes are linked with a 2A peptide, the polypeptides are separately expressed because the glycine and the proline at the C-terminus of the 2A peptide are not linked by ribosomal skipping during translation.

An embodiment of the production method in which the modified TCR is introduced into a cell in the form of a protein is a method for expressing the modified TCR on the cell surface also by synthesizing the modified TCR by a protein synthesis system from the polynucleotide and incorporating the modified TCR into a cell. Examples of the method for introducing a protein into a target cell include a method using a protein transduction reagent, a method using a protein transduction domain (PTD)-fused protein, electroporation, microinjection, the method described in Shimono K, et al., Protein Sci. 18(10): 2160-71, 2009 and the like.

### [Cells for Introducing Modified TCR]

Examples of the cell into which the modified TCR is introduced include a pluripotent stem cell, a hematopoietic stem and progenitor cell, an endogenous TCRα gene- and endogenous TCRβ gene-knockdown or knockout T cell and the like.

Pluripotent stem cells in the invention are stem cells having pluripotency, the capacity to differentiate into many cells in the body, and also having growth capacity and include any cells which are at least differentiated/induced into T cells used in the invention. The pluripotent stem cells are preferably derived from a mammal and more preferably derived from a human.

In the invention, the origin means the source of acquisition, and for example, a pluripotent stem cell derived from a mammal means a pluripotent stem cell acquired from a mammal.

The pluripotent stem cells are not particularly limited, but examples include embryonic stem (ES) cells, nuclear transfer embryonic stem (ntES) cells derived from a cloned embryo, germline stem cells (GS cells), embryonic germ cells (EG cells), induced pluripotent stem (iPS) cells, cultured fibroblast cell- or cord blood-derived pluripotent stem cells, bone marrow stem cell-derived pluripotent stem cells (Muse cells) and the like. In the invention, preferable pluripotent stem cells are iPS cells, more preferably human iPS cells because the cells can be obtained without breaking an embryo, an egg cell or the like during the production step.

The pluripotent stem cells of the invention are preferably pluripotent stem cells which do not have endogenous TCRα gene and endogenous TCRβ gene, more preferably non-T cell-derived pluripotent stem cells or endogenous TCRα gene- and endogenous TCRβ gene-knockdown or knockout T cell-derived pluripotent stem cells. Examples thereof include endogenous TCRα gene- and endogenous TCRβ gene-knockdown or knockout T cell-derived induced pluripotent stem cells (T-iPS cells) and the like.

The production method of iPS cells is known in this field, and iPS cells can be produced by introducing reprogramming factors into any somatic cells. Examples of the reprogramming factors include genes such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3 and Glis1 and genetic products. One of the reprogramming factors may be used alone, or a combination thereof may be used.

Examples of the combination of reprogramming factors include the combinations described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, WO2010/147612, Huangfu D, et al., Nat. Biotechnol., 26: 795-797, 2008, Shi Y, et al., Cell Stem Cell, 2: 525-528, 2008, Eminli S, et al., Stem Cells. 26: 2467-2474, 2008, Huangfu D, et al., Nat. Biotechnol. 26: 1269-1275, 2008, Shi Y, et al., Cell Stem Cell, 3, 568-574, 2008, Zhao Y, et al., Cell Stem Cell, 3: 475-479, 2008, Marson A, Cell Stem Cell, 3, 132-135, 2008, Feng B, et al., Nat. Cell Biol. 11:197-203, 2009, R.L. Judson et al., Nat. Biotechnol., 27: 459-461, 2009, Lyssiotis CA, et al., Proc Natl Acad Sci U S A. 106: 8912-8917, 2009, Kim JB, et al., Nature. 461:649-643, 2009, Ichida JK, et al., Cell Stem Cell. 5: 491-503, 2009, Heng JC, et al., Cell Stem Cell. 6:167-74, 2010, Han J, et al., Nature. 463: 1096-100, 2010, Mali P, et al., Stem Cells. 28: 713-720, 2010 or Maekawa M, et al., Nature. 474:225-9, 2011 and the like.

The somatic cells used for producing iPS cells are not particularly limited, but examples thereof include fetal somatic cells, neonatal somatic cells, matured healthy or diseased somatic cells, primary cultured cells, subcultured cells, cells of an established line and the like.

Examples of the somatic cells include (1) tissue stem cells (for example, somatic stem cells) such as neural stem cells, hematopoietic stem cells, mesenchymal stem cells and pulp stem cells, (2) tissue progenitor cells such as hematopoietic progenitor cells or (3) differentiated cells such as blood cells (for example, peripheral blood cells, cord blood cells and the like), myelocytes, lymphocytes, epithelial cells, endothelial cells, muscle cells, fibroblasts (for example, skin cells and the like), bristle cells, hepatocytes, gastric mucosal cells, enterocytes, splenocytes, pancreatic cells (for example, exocrine pancreatic cells and the like), brain cells, pneumocytes, nephrocytes and fat cells and the like. The somatic cells are preferably somatic cells other than T cells (non-T cells), but T cells may also be used.

The T cells as the somatic cells are not particularly limited, but T cells which express CD3 and which express at least one of CD4 and CD8 molecules are preferable.

Examples of the T cells include helper T cells, cytotoxic T cells, regulatory T cells, naive T cells, stem cell memory T cells (TSCMs), central memory T cells (TCMs), effector memory T cells, terminal effector T cells and the like.

Helper T cells are CD4-positive cells and are further classified into Th1 cells, Th2 cells, Th17 cells and the like depending on the expressed cytokines (for example, described in J Allergy Clin. Immunol, 135(3): 626-635, 2012).

Examples of Th1 cells include cells which express IFN-γ, IL-2, TNF-α or the like and the like. Examples of Th2 cells include cells which express IL-4, IL-5, IL-6, IL-10, IL-13 or the like and the like. Examples of Th17 cells include cells which express IL-17, IL-6 or the like and the like.

Cytotoxic T cells are CD8-positive cells and are further classified into Tc1 cells, Tc2 cells and the like depending on the expressed cytokines like helper T cells.

Examples of Tc1 cells include cells which express IFN-γ, IL-2, TNF-α or the like and the like. Examples of Tc2 cells include cells which express IL-4, IL-5, IL-6, IL-10, IL-13 or the like and the like.

Preferable examples of regulatory T cells include CD4(+)CD25(+)FoxP3(+) cells.

Preferable examples of naive T cells include CD4(+)CD45RA(+)CD62L(+)CCR7(+) cells, CD8(+)CD45RA(+)CD62L(+)CCR7(+) cells, CD4(+)CCR7(+)CD45RA(+)CD95(-)CD45RO(-) cells, CD8(+)CCR7(+)CD45RA(+)CD95(-)CD45RO(-) cells and the like.

Preferable examples of stem cell memory T cells include CD4(+)CD45RA(+)CD62L(+)CCR7(+)CD95(+) cells, CD8(+)CD45RA(+)CD62L(+)CCR7(+)CD95(+) cells, CD4(+)CCR7(+)CD45RA(+)CD95(+)CD45RO(+) cells, CD8(+)CCR7(+)CD45RA(+)CD95(+)CD45RO(+) cells and the like.

Preferable examples of central memory T cells include CD4(+)CD45RA(-)CD62L(+)CCR7(+)CD95(+) cells, CD8(+)CD45RA(-)CD62L(+)CCR7(+)CD95(+) cells, CD4(+)CCR7(+)CD45RA(-)CD45RO(+) cells, CD8(+)CCR7(+)CD45RA(-)CD45RO(+) cells and the like.

Preferable examples of effector memory T cells include CD4(+)CCR7(-)CD45RA(-)CD45RO(+) cells, CD8(+)CCR7(-)CD45RA(-)CD45RO(+) cells and the like.

Preferable examples of terminal effector T cells include CD4(+)CD45RA(+)CD62L(-) cells, CD8(+)CD45RA(+)CD62L(-) cells and the like.

The T cells as the somatic cells are preferably undifferentiated T cells. Examples of undifferentiated T cells include naive T cells, stem cell memory T cells and central memory T cells in the order of undifferentiation degree. Of these cells, naive T cells or stem cell memory T cells are particularly preferable to maintain the undifferentiated state of the cells and improve the proliferative ability or the in vivo persistence.

Highly undifferentiated T cells exhibit a high immune cell therapeutic effect. The T cells as the somatic cells are preferably T cells for an immune cell therapy.

Methods for determining whether a T cell maintains the undifferentiated state are a method for determining by detecting the expression of an undifferentiation marker and/or by not detecting the expression of a differentiation marker, a method for determining by detecting the expression of another marker (a gene or a protein), a method for observing the morphological features of the cell and the like. For example, CCR7 is used as an undifferentiation marker of peripheral blood (for example, described in Nature Reviews Immunology, 18: 363-373, 2018).

When T cells are used as the somatic cells, endogenous TCRα gene- and endogenous TCRβ gene-knockdown or knockout T cells are preferable.

The endogenous TCRα gene and the endogenous TCRβ gene mean the TCRα gene and the TCRβ gene that the T cell which is subjected to knockdown or knockout has, the TCRα gene and the TCRβ gene derived from the T cell which is subjected to knockdown or knockout or the TCRα gene and the TCRβ gene derived from a T cell of the same species as that of the T cell which is subjected to knockdown or knockout. That a gene is knocked down means that the transcription level of a specific gene is reduced or the translation is inhibited. That a gene is knocked out means that the nucleotide sequence of a specific gene is broken to inhibit the expression of the gene.

As the production method of the endogenous TCRα gene- and endogenous TCRβ gene-knockdown or knockdown T cell of the invention, a known method such as a method in which small interfering RNAs (siRNAs) to the genes are introduced into the cell and in which the expression of the genes is thus inhibited (described in NPL 1) can be used. As the production method of the endogenous TCRα gene- and endogenous TCRβ gene-knockdown or knockout T cell, a known method such as a method in which a frameshift mutation is caused in the gene regions using genome editing technology such as TALEN and CRISPR/Cas9 and in which the gene coding sequences are thus broken (NPL 2) can be used.

In the invention, the origin from which the somatic cells are taken is not particularly limited but is preferably a mammal, more preferably a human. When the human-derived T cells of the invention are used for blood transfusion, the somatic cells as the origin of the iPS cells are preferably isolated from the subject of the blood transfusion because the type of the human leukocyte antigen (HLA) can be easily matched to the type of the patient who receives the blood transfusion.

An existing cell line may be used as the iPS cells. Examples of human iPS cell lines include line 253G1 (RIKEN Cell Bank No. HPS0002), line 201B7 (RIKEN Cell Bank No. HPS0063), line 409B2 (RIKEN Cell Bank No. HPS0076), line 454E2 (RIKEN Cell Bank No. HPS0077), line HiPS-RIKEN-1A (RIKEN Cell Bank No. HPS0003), line HiPS-RIKEN-2A (RIKEN Cell Bank No. HPS0009), line HiPS-RIKEN-12A (RIKEN Cell Bank No. HPS0029), line Nips-B2 (RIKEN Cell Bank No. HPS0223) and the like.

The cell into which the modified TCR of the invention is introduced may be a hematopoietic stem and progenitor cell. The hematopoietic stem and progenitor cell can be taken from bone marrow, cord blood, mobilized peripheral blood or the like. Such an origin is of the patient himself or a healthy individual such as a related donor and an unrelated donor.

The cell into which the modified TCR of the invention is introduced may also be an endogenous TCRα gene- and endogenous TCRβ gene-knockdown or knockout T cell or the like.

The T cell, the endogenous TCRα gene and the endogenous TCRβ gene, knocking down of a gene and knocking out of a gene and the production method of an endogenous TCRα gene- and endogenous TCRβ gene-knockdown or knockdown T cell are as described above.

The cell into which the modified TCR of the invention is introduced may be a cell into which a chimeric antigen receptor (CAR) has been introduced in advance. Here, a CAR means a fusion protein comprising an extracellular domain binding to an antigen and an intracellular domain derived from a polypeptide which is different from the extracellular domain. Examples of CAR include a fusion protein obtained by binding an antigen recognition site of an antibody to a specific antigen [for example, a light chain (L chain) of a variable region and a heavy chain (H chain) of a variable region or the like] to an intracellular domain of a T-cell receptor such as CD3 and an intracellular domain of a co-stimulatory molecule such as CD28 and 4-1BB and the like (for example, described in JP-T-2015-509716, the term "JP-T" as used herein means a published Japanese translation of a PCT patent application).

The antigen recognition site of the CAR can be selected depending on the target antigen, and a T cell which is specific to the target antigen can be thus produced. For example, when CD19 is the antigen, by cloning the antigen recognition site of an anti-CD 19 antibody and binding to the intracellular domain of CD3 molecule, a CAR can be obtained (for example, described in Cancer Res., 66: 10995-11004, 2006). Moreover, by selecting the kinds, the number or the like of the co-stimulatory molecules to be bound, the strength, the duration or the like of the activation can be controlled (for example, described in Mol Ther., 17: 1453-1464, 2009).

By introducing a CAR, the specificity to the target antigen can be given to the cell into which the modified TCR of the invention is introduced. Moreover, by introducing a CAR, the antigen molecules can be directly recognized, and high immunoreaction can be caused also to a tumor in which the expression level of HLA class I genes is decreased.

### [Differentiation of Modified TCR-Introduced Pluripotent Stem Cell or Primary Hematopoietic Stem and Progenitor Cell into T Cell]

The method for differentiating a modified TCR-introduced pluripotent stem cell into a T cell in the invention can be conducted using a known method. Specific examples include the method described in WO2016/076415 as the production method of CD8-positive cells and the method described in WO2017/221975 as the production method of CD4- and CD8-positive T cells.

The method for differentiating a primary hematopoietic stem and progenitor cell into a T cell can be conducted by the same or similar method as the method for differentiating a pluripotent stem cell into a T cell described above (for example, described in Induction of T-cell development from human cord blood hematopoietic stem cells by Delta-like 1 in vitro; Blood, 105(4): 1431-1439, 2005).

### [Therapeutic Agent, Pharmaceutical Composition and Method of immune cell therapy]

The modified TCR according to the invention, the cell which expresses the modified TCR, the nucleic acid encoding the first polypeptide and the second polypeptide constituting the modified TCR or the expression vector comprising the nucleic acid can be each used as a therapeutic agent. The therapeutic agent may be used alone or can be used in combination with another drug and a therapeutic method which can be used for treating a cancer, an autoimmune disease, heart disease or the like.

The other drug and the other therapeutic method which can be used are not particularly limited, but examples thereof include a molecularly targeted drug, chemotherapy, radiofrequency ablation, surgical treatment, transarterial (chemo) embolization, radiotherapy, heavy ion radiotherapy, radioisotope therapy, transarterial infusion, peptide vaccine therapy, another immunocytotherapy and the like. The therapeutic agent of the invention and the other drug may be administered simultaneously or administered separately and may be administered by a same administration route or different administration routes.

The therapeutic agent can also be provided in the form of a pharmaceutical composition or a composition for an immune cell therapy, alone or in combination with another active ingredient. The components which can be contained in the pharmaceutical composition include, in addition to the therapeutic agent of the invention and another active ingredient, a carrier, a buffer, a stabilizer or the like which is generally blended in this field and which is pharmaceutically acceptable depending on the dosage form. The carrier is physiological saline, phosphate-buffered saline, glucose solution or buffered saline but is not limited to the carriers. A salt, a saccharide, a sugar alcohol or the like can also be used as an additive. Here, the therapeutic agent and the pharmaceutical composition of the invention are intended for administration in the liquid form due to the characteristics of the invention and thus are required to be in the form that can maintain the stability of the active ingredients.

The therapeutic agent and the pharmaceutical composition of the invention can be locally administered or systemically administered and can also be, for example, intravenously administered although the dosage form is not particularly limited. Moreover, the therapeutic agent and the pharmaceutical composition may be administered by injection or infusion into the affected part or near the affected part. The dosage and the administration frequency of the therapeutic agent or the pharmaceutical composition of the invention vary with the body weight of the patient, the gender, the age, the seriousness of the disease or the like and are not particularly limited.

Examples of the method of immune cell therapy of the invention include the following methods: a method comprising a step of expressing the modified TCR of the invention in a non-T cell-derived pluripotent stem cell and differentiating the cell into a T cell and a step of administering the obtained cell to a subject; a method comprising a step of expressing the modified TCR of the invention in an endogenous TCRα gene- and endogenous TCRβ gene-knockdown or knockout T cell-derived pluripotent stem cell and differentiating the cell into a T cell and a step of administering the obtained cell to a subject; a method comprising a step of expressing the modified TCR of the invention in a hematopoietic stem and progenitor cell and differentiating the cell into a T cell and a step of administering the obtained T cell to a subject; a method comprising a step of expressing the modified TCR of the invention in an endogenous TCRα gene- and endogenous TCRβ gene-knockdown or knockout T cell and a step of administering the obtained cell to a subject; and other methods.

As the T cell to be administered, the obtained T cell may be administered directly or administered in the form of a pharmaceutical composition formulated as described above.

The invention is explained in detail below based on Examples, but the embodiments of the invention are not limited to the Examples.

### Examples

### [Example 1]

The capabilities of holding CD3 subunit molecules of modified TCRs were analyzed in 293T cells.

First, a full-length TCR expression vector was constructed. As the full-length TCRα chain and full-length TCRβ chain genes, the TCR gene sequences derived from T cell-derived iPS cell line TKT3v1-7 established by introducing Yamanaka factors into T cell (provided by The University of Tokyo, described in Nishimura et al., Cell Stem Cell.12: 774-786, 2013) were used. The full-length nucleotide sequence of the TCRα used in the Examples is SEQ ID NO: 25, and the full-length nucleotide sequence of the TCRβ is SEQ ID NO: 36. RNA was extracted from T cells obtained by differentiating from line TkT3v1-7, and the 5' RACE cDNAs and the 3' RACE cDNAs of the TCRα and TCRβ genes were obtained using SMARTer RACE 5'/3' Kit (Takara Bio Inc.). The information on the full-length cDNA sequences were obtained by Sanger sequencing of the cDNA products.

The TCR expression vector was constructed by cloning a gene obtained by linking the TCRβ chain gene and the TCRα chain gene in this order with an SGSG linker-T2A peptide sequence in the upstream of IRES of pEF1α-IRES-hKO1 (humanized-codon Kusabira-Orange) vector (referred to as mock vector below) using In-Fusion HD Cloning Kit (Clontech Laboratories, Inc.). This full-length TCR expression vector was named ba1 vector.

Next, a modified TCR expression vector in which the variable region deletion was deleted was constructed. A variable region-deleted TCR gene was obtained by connecting, to just after the sequences encoding the signal peptides of the TCRα and TCRβ sequences, the constant region sequences of the respective TCR sequences by in-frame. The gene was cloned in mock vector like ba1 vector, and the expression vector was constructed (ba2 vector).

Modified TCR expression vectors in which sequences of the constant regions of the TCRα and TCRβ sequences were deleted in a step-wise manner in addition to the variable regions were also constructed. Based on known documents (described in Kuhns et al., Immunity. 26: 357-369, 2007; Natarajan et al., Cell Rep. 14: 2833-2845, 2016), four regions, namely AB loop, C strand, DE loop and F strand, were extracted from the TCRα constant region, and four regions, namely Helix3, CC loop, Helix4 F strand and FG loop, were extracted from the TCRβ constant region. Modified TCR sequences with different patterns were designed by connecting, to just after the sequences encoding the signal peptides of the TCRα and TCRβ sequences, constant region sequences with deletions of nucleotides from the 5'-terminus which were in a step-wise manner to eliminate the respective amino acid regions by in-frame, and the modified TCR sequences were cloned in mock vector. The expression vectors were thus constructed (ba3 to ba18 vectors).

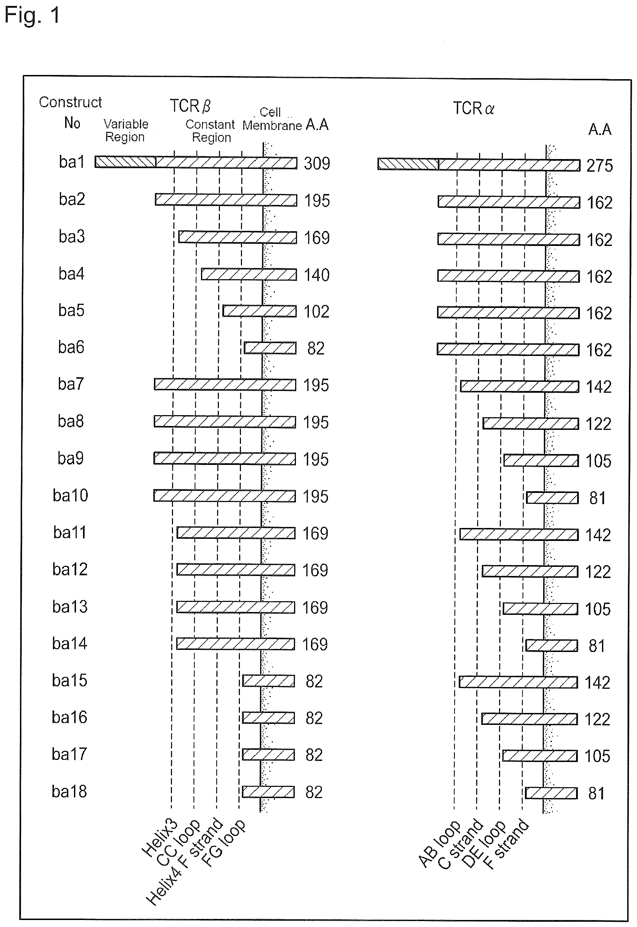

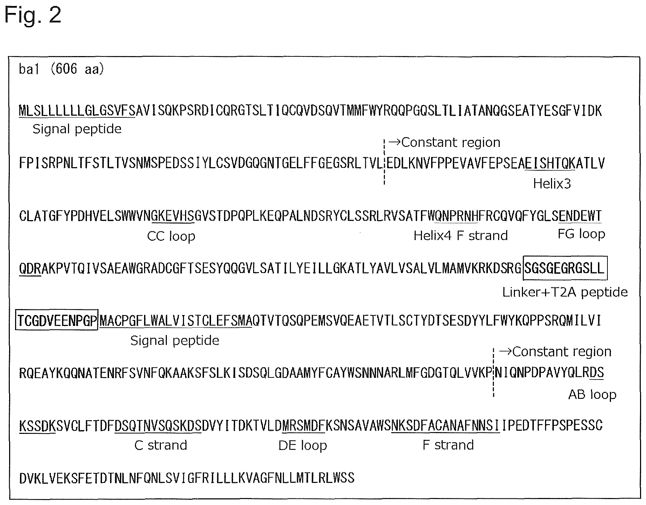

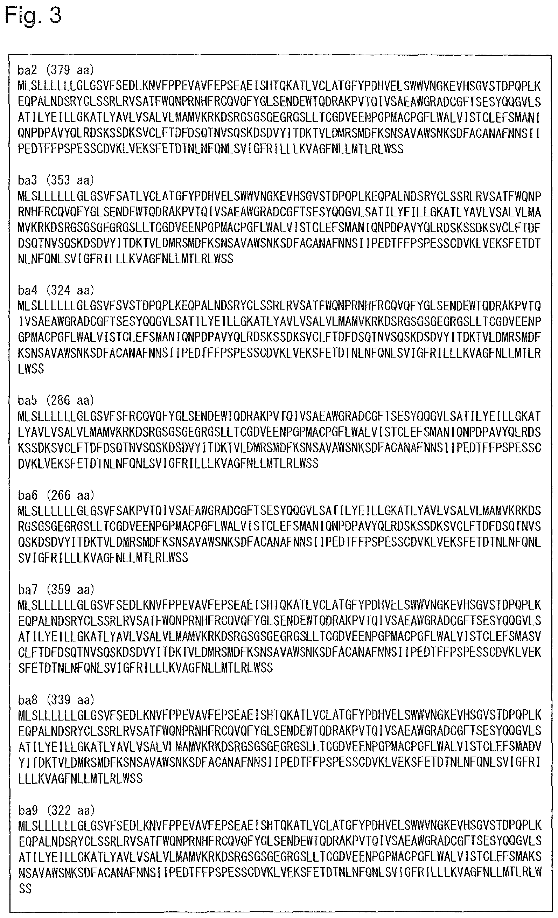

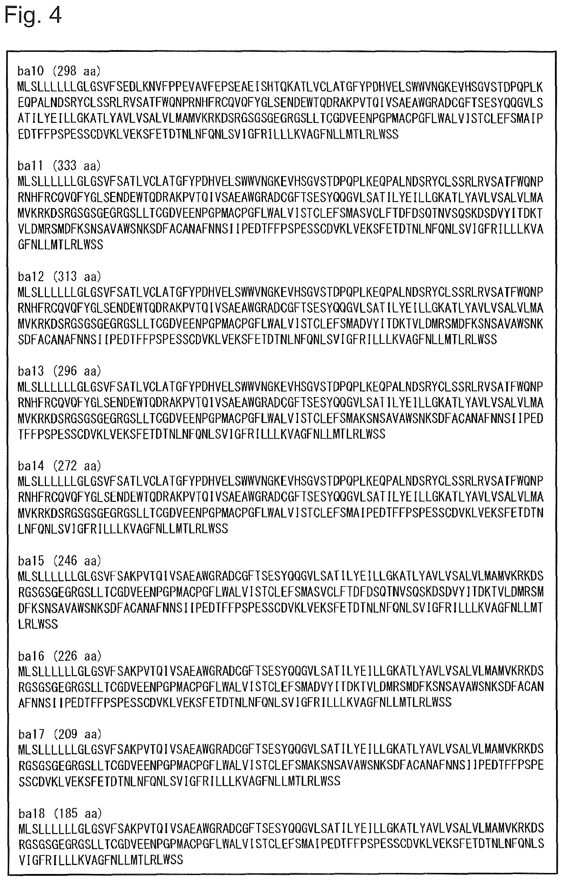

A schematic view of the above full-length and modified TCR sequences is shown in Fig. 1. The amino acid sequence information of ba1 (SEQ ID NO: 49) is shown in Fig. 2, and the amino acid sequence information of ba2 to ba9 (SEQ ID NOs: 50 to 57) is shown in Fig. 3. The amino acid sequence information of ba10 to ba18 (SEQ ID NOs: 58 to 66) is shown in Fig. 4.

A CD3 expression vector to be expressed in 293T cells was constructed based on known cDNA sequence information of CD3 subunits (CD3E: NM 000733.3; CD3G: NM_000073.2; CD3D: NM_000732.4; CD247(CD3Z): NM_198053.2). A gene obtained by linking the CD3 subunit sequences and EGFP gene with a GSG linker-T2A peptide sequence (CD3E-T2A-CD3G-T2A-CD3D-T2A-CD3Z-T2A-EGFP) was cloned in a pEF1α expression vector.

A 293T cell line which stably expresses CD3 was established. The 293T cells were obtained from DSMZ (#ACC635). For maintenance culture of the 293T cells, 293T medium [containing Dulbecco's Modified Eagle Medium (Nacalai Tesque, Inc.), 10% FBS (Access Cell Culture) and 10 µg/mL gentamicin sulfate (Nacalai Tesque, Inc.)] was used. For passage, after washing the 293T cells once with PBS (Nacalai Tesque, Inc.), the cells were detached with a Trypsin/EDTA solution (Sigma). A CD3-EGFP-stably expressing line was established by transfecting the 293T cells with the CD3 expression vector using Lipofectamine 2000 (ThermoFisher) and then repeating purification of the EGFP-positive fraction by cell sorter SH800 (SONY) and amplification culture.

Whether CD3E and CD3D molecules would be held on the cell surface by introducing the above ba vectors into the CD3-EGFP-stably expressing line was examined. After transfecting the CD3-EGFP-stably expressing line with any of the ba vectors and mock vector using Lipofectamine 2000, the cells were collected after two days. The collected cells were washed with FACS buffer [containing PBS (Nacalai Tesque, Inc.), 2% FBS (Access Cell Culture) and 1 mM EDTA (Invitrogen)]. A PE/Cy7-labeled anti-human CD3E antibody (clone: UCHT1) (BioLegend) was added to the cells for CD3E staining, and an APC-labeled anti-human CD3D antibody (clone: 7D6) (Invitrogen) was added to the cells for CD3D staining. The cells were then left to stand still in a dark place at 4°C for 30 minutes. After washing the cells with FACS buffer, the dead cells were stained by adding DAPI (Dojindo Laboratories). Cell sorter SH800 was used for the FACS analysis.

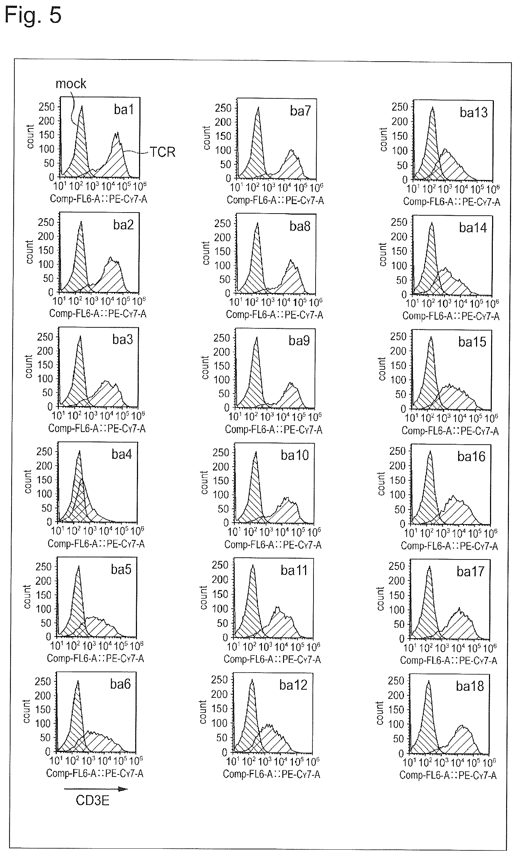

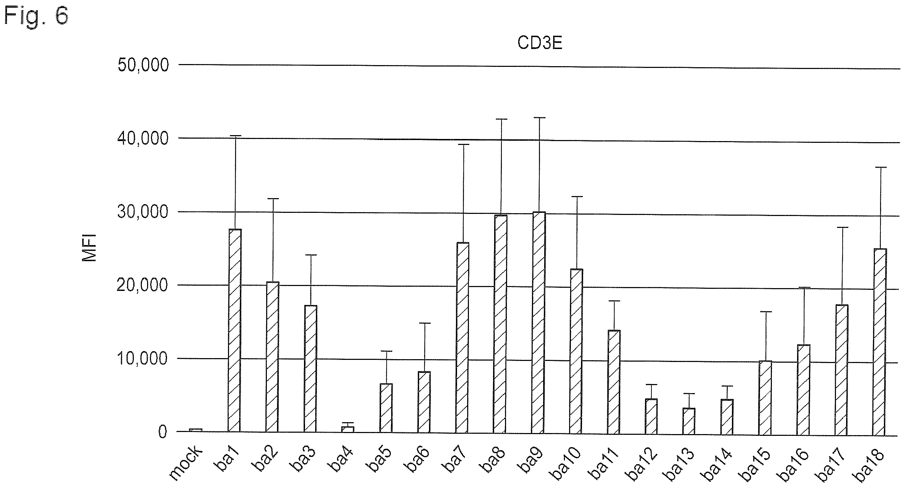

The expression patterns of CD3E in the EGFP-positive hKO1-positive DAPI-negative fractions are shown in Fig. 5, and the data quantified by MFI (Mean Fluorescence Intensity) are shown in Fig. 6. The expression of CD3E was observed in the full-length TCR (ba1)-introduced cells. Although the expression of CD3E on the cell surface was observed also when the modified TCRs of ba2 to ba18 were introduced, the expression levels varied with the kinds of modified TCR.

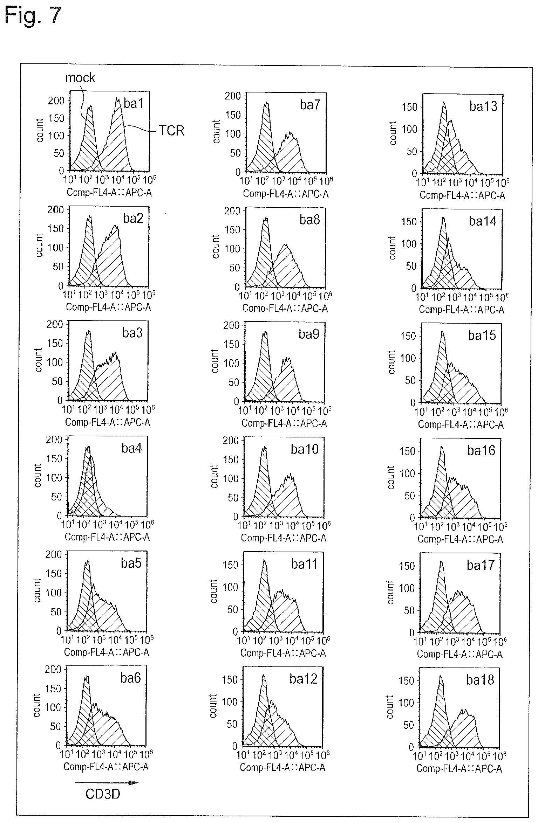

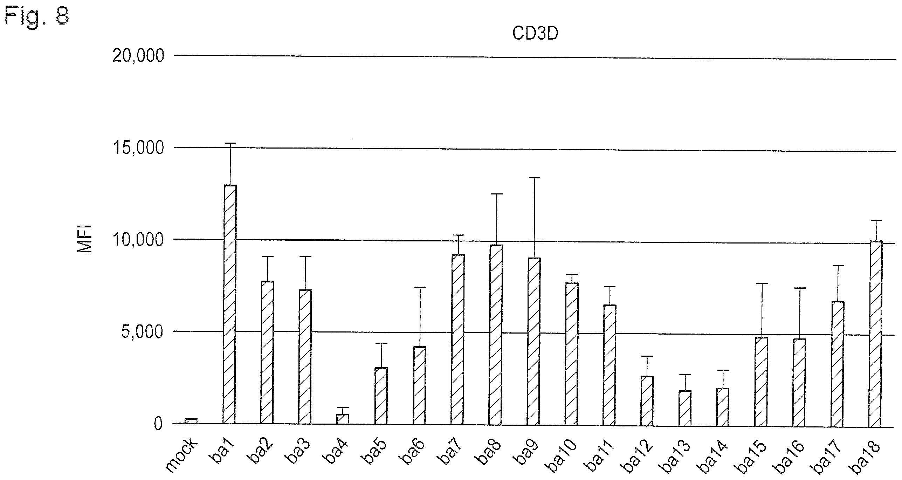

The expression patterns of CD3D are shown in Fig. 7, and the data quantified by MFI are shown in Fig. 8. The expression of CD3D was observed in the full-length TCR (ba1)-introduced cells. As in the case of CD3E, although the expression of CD3D on the cell surface was observed by introducing the modified TCRs of ba2 to ba18, the expression levels varied with the kinds of modified TCR.

From the above results, through the artificial TCR/CD3 reconstruction experiment in 293T cells, screening of modified TCRs which are capable of efficiently holding the expression of CD3 subunits on the cell surface has become possible.

As a result of the examination of ba1 to ba18, it was found that the expression levels of CD3E and CD3D on the cell surface vary with the deletion pattern of the TCR genes. Specifically, ba2, ba3, ba7, ba8, ba9, ba10, ba17 and ba18 had equivalent capabilities of holding CD3E and CD3D to that of ba1.

### [Example 2]

The capabilities of holding CD3 subunit molecules of modified TCRs were analyzed in Jurkat cells.

In order to analyze the capabilities of holding CD3 subunits of modified TCRs under more physiological conditions, Jurkat cells (#ACC282) (DSMZ), which are a cell line of T cells, were used. For maintenance culture of Jurkat cells, Jurkat medium [containing RPMI1640 (Nacalai Tesque, Inc.), 10% FBS (Access Cell Culture) and 10 µg/mL gentamicin sulfate (Nacalai Tesque, Inc.)] was used. For passage, an adequate amount of Jurkat cells were taken and suspended in fresh Jurkat medium.

Jurkat cells, which are a cell line of T cells, have an endogenous TCR gene in which the TCRα and TCRβ genes have already been reconstructed. Accordingly, when a modified TCR gene is introduced to wild-type Jurkat cells (referred to as the WT line below), a chimeric TCR molecule may be formed with the endogenous TCR gene, and the capability of holding CD3 subunits of the modified TCR cannot be determined accurately. Thus, the inventors tried to knockout both the endogenous TCRα gene and the TCRβ gene by genome editing in Jurkat cells.

Genome was edited targeting the gene encoding the TCRα chain constant region (TRAC gene) and the gene encoding the TCRβ chain constant region (TRBC gene), and the genes were knocked out by frame shifting. For the genome editing, a method for directly introducing a complex of Cas9 protein and guide RNA into the cytoplasm using Alt-R CRISPR-Cas9 System (IDT) was used. The target sequences (20 mer in the upstream of NGG) were each extracted from known documents: TRAC: gagaatcaaaatcggtgaat (SEQ ID NO: 67) (described in Osborn et al., Mol Ther, 2017); and TRBC (common to TRBC1 and TRBC2): caaacacagcgacctcgggt (SEQ ID NO: 68) (described in Legut et. al., Blood. 131(3): 311-322, 2018).

The Cas9/guide RNA complexes were formed based on the specification, and then the Cas9/guide RNA complexes were introduced into Jurkat cells by electroporation using 4D-Nucleofector (Lonza) and SE Cell Line 4D-Nucleofector X Kit L (Lonza) (introduction program: CL-120).

First, the Cas9/guide RNA complex for knocking out TRAC gene was introduced to the WT line. After amplification culture, the cells were stained with an APC-labeled anti-human TCRα/β antibody (clone: IP26) (BioLegend) and a PE/Cy7-labeled anti-human CD3E antibody (clone: UCHTl) (BioLegend), and, as a result, a TCR-negative CD3E-negative fraction appeared. The fraction was purified with a cell sorter and subjected to amplification culture, and then the Cas9/guide RNA complex for knocking out TRBC gene was introduced. After amplification culture, a vector which expresses only the full-length TCRα chain was transiently introduced into the cells, and the cells were stained using the APC-labeled anti-human TCRα/β antibody and the PE/Cy7-labeled anti-human CD3E antibody. As a result, a TCR-negative CD3E-negative fraction appeared. The fraction was considerdto be a fraction in which the TCRβ gene was successfully knocked out, and the fraction was purified with a cell sorter and subjected to amplification culture.

In the end, a vector which expresses only the full-length TCRα chain or a vector which expresses only the full-length TCRβ chain was transiently introduced into the cells, and the cells were stained with the APC-labeled anti-human TCRα/β antibody and the PE/Cy7-labeled anti-human CD3E antibody. As a result, a TCR-negative CD3E-negative fraction was observed with a purity of around 99% in both cases. In this manner, a TCRα gene- and TCRβ gene-double knockout line was established (referred to as line dKO below).

Whether CD3E and CD3D molecules would be held on the cell surface by introducing the ba vectors into line dKO was examined. After introducing any of the ba vectors and mock vector by electroporation similar to that above, cells were collected after two days. The collected cells were washed with FACS buffer. The PE/Cy7-labeled anti-human CD3E antibody was added to the cells for CD3E staining, and the APC-labeled anti-human CD3D antibody was added to the cells for CD3D staining. The cells were then left to stand still in a dark place at 4°C for 30 minutes. After washing the cells with FACS buffer, the dead cells were stained by adding DAPI. Cell sorter SH800 was used for the FACS analysis.

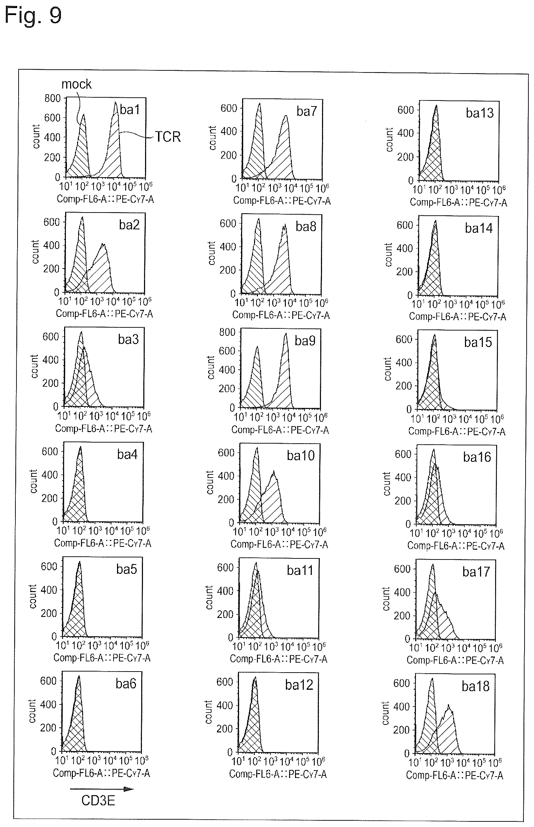

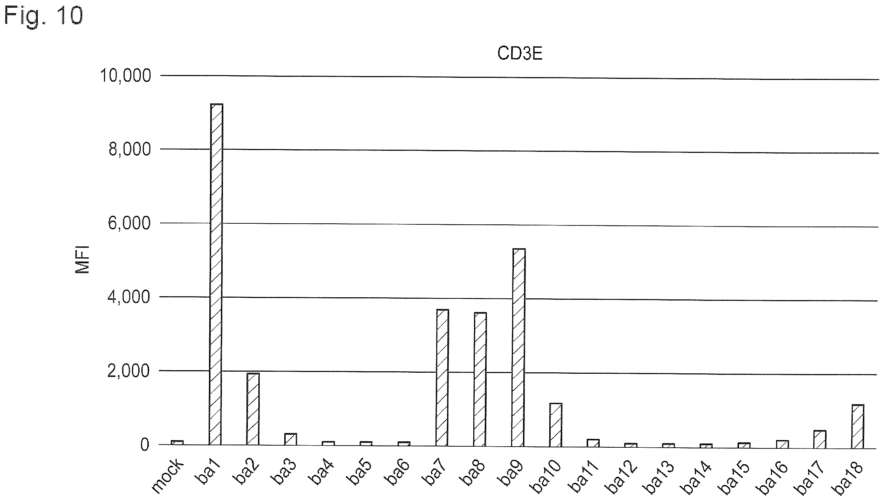

The expression patterns of CD3E in the DAPI-negative fractions are shown in Fig. 9, and the data quantified by MFI are shown in Fig. 10. The expression of CD3E was observed in the full-length TCR (ba1)-introduced cells. Although the expression of CD3E on the cell surface was observed also when the modified TCRs of ba2 to ba18 were introduced, the expression levels varied with the kinds of modified TCR.

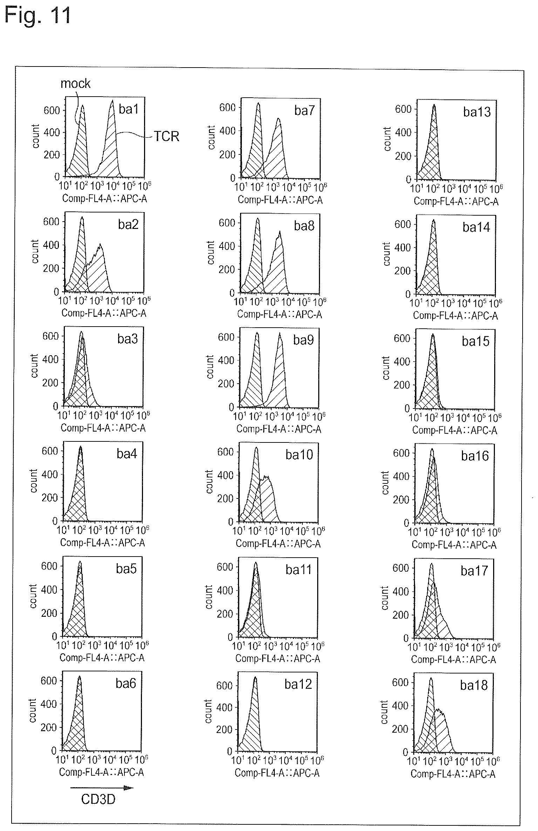

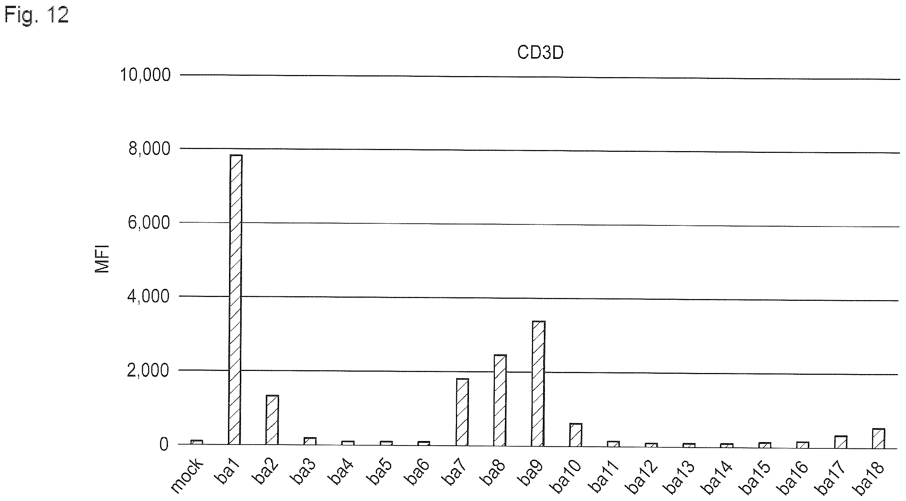

The expression patterns of CD3D are shown in Fig. 11, and the data quantified by MFI are shown in Fig. 12. The expression of CD3D was observed in the full-length TCR (ba1)-introduced cells. As in the case of CD3E, although the expression of CD3D on the cell surface was observed by introducing the modified TCRs of ba2 to ba18, the expression levels varied with the kinds of modified TCR.

From the above results, it was found that modified TCRs which are capable of efficiently holding the expression of CD3 subunits on the cell surface can be screened using line dKO, which is Jurkat cells having the properties of T cells. As a result of the examination of ba1 to ba18, it was found that the expression levels of CD3E and CD3D on the cell surface vary with the deletion patterns of the TCR genes. As a result of the examination of ba1 to ba18, it was found that the expression of CD3E and CD3D on the cell surface is supported at relatively high levels also in Jurkat cells, for example, with ba7, ba8 and ba9 although the levels are not as high as those of ba1.

### [Example 3]

The capabilities of transmitting signals of modified TCRs were analyzed using line dKO of Jurkat cells.

In Example 1 and Example 2, it was shown that the capabilities of supporting the expression of CD3 subunits on the cell surface vary with the designed modified TCRs. Next, the signal transduction to T cells through CD3 molecules presented on the cell surface by modified TCRs was examined.

CD69 is a representative cell surface marker of activated T cells, and the expression thereof is induced at an early stage when a T cell responds to TCR/CD3 complex stimulation (described in Ziegler et al., Stem Cells. 12(5): 456-65, 1994). It is known, for example, that the expression of CD69 is enhanced by stimulation with CD3/CD28 antibody-bound beads also in Jurkat cells (described in Tomkowicz et al., PLoS One, 2015). Accordingly, when CD3 molecules are held on the cell surface by a modified TCR, that the expression of CD69 is enhanced by CD3 stimulation indicates that the modified TCR is also functionally useful in the T-cell signal transduction.

Whether the expression of CD69 would be enhanced by stimulating the WT line and line dKO of Jurkat cells with OKT3, which is an anti-CD3E agonist antibody, was examined. To a 96-well plate for adherent cells, 100 µL of Ultra-LEAF Purified anti-human CD3 Antibody (clone: OKT3) (BioLegend) which was diluted to 10 µg/mL using PBS (Nacalai Tesque, Inc.) was dispensed, and the plate was left to stand still at 37°C overnight.

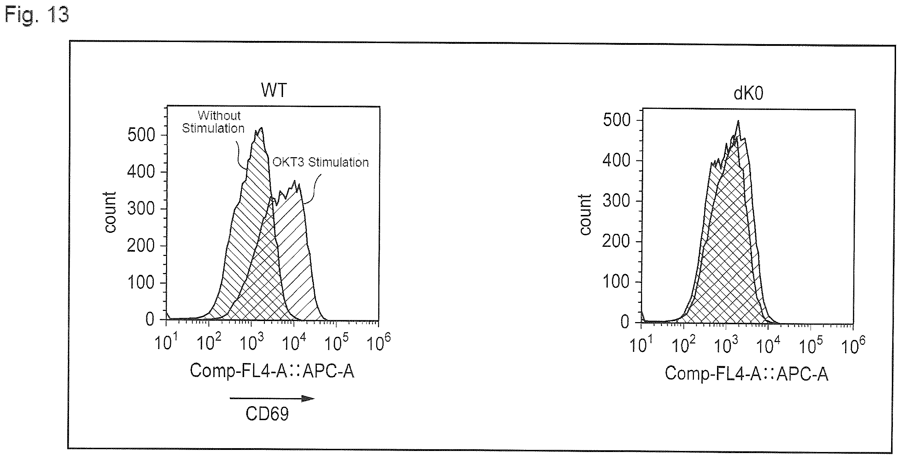

After removing the supernatants and washing twice with PBS (the plate is referred to as a solidificated OKT3 plate below), the WT line or line dKO was seeded in the plate and cultured at 37°C overnight. The cells were collected and washed with FACS buffer. An Alexa Fluor 647-labeled anti-human CD69 antibody (clone: FN50) (BioLegend) was added to the cells, and the cells were left to stand still in a dark place at 4°C for 30 minutes. After washing the cells with FACS buffer, the dead cells were stained by adding DAPI. Cell sorter SH800 was used for the FACS analysis. The results are shown in Fig. 13.

As shown in Fig. 13, while the expression of CD69 increased by OKT3 stimulation in the WT line, enhancement of the expression of CD69 was not observed in line dKO. Accordingly, it was shown that the responsiveness to CD3 stimulation is also eliminated in line dKO, in which the CD3 expression is eliminated by TCR-double knocking out.

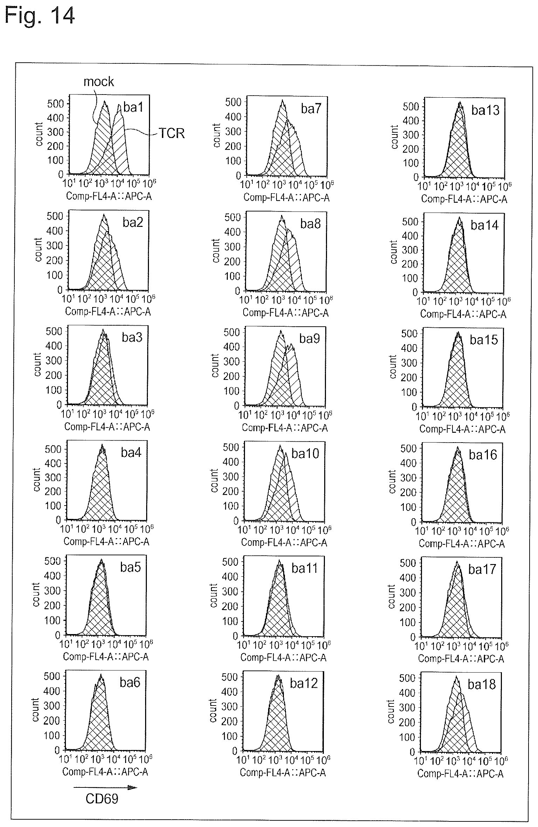

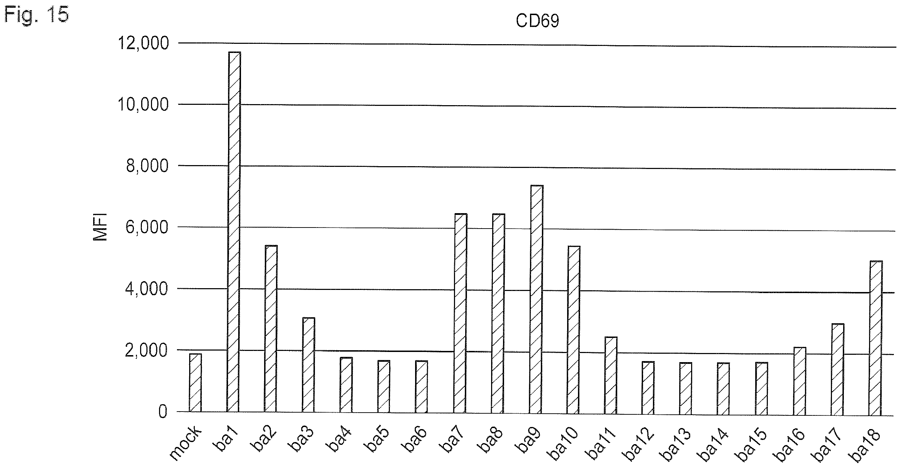

Next, whether enhancement of the expression of CD69 through OKT3 stimulation would be caused by introducing modified TCRs into line dKO was examined. As in Example 2, after introducing any of the ba vectors and mock vector into line dKO by electroporation, the cells were collected on the next day and seeded in a solidificated OKT3 plate. The cells were collected on the next day, and the expression of CD69 was analyzed by the same procedures as those described above. The expression patterns of CD69 in the DAPI-negative fractions are shown in Fig. 14, and the data quantified by MFI are shown in Fig. 15.

As shown in Fig. 14 and Fig. 15, the expression of CD69 was observed in the full-length TCR (ba1)-introduced cells. Although the expression of CD69 on the cell surface was observed also when the modified TCRs of ba2 to ba18 were introduced, the expression levels varied with the kinds of modified TCR.

From the above results, it was found that modified TCRs not only have the action of simply holding CD3 molecules on the surface of cell membrane but also have the action of transmitting TCR/CD3 complex signals into the cytoplasm by stimulating CD3 molecules presented on the cell surface with an agonist antibody or the like.

### [Example 4]

With respect to the results of the FACS analysis on CD3E protein obtained in Example 1, the CD3E-positive proportions by CD1-18 in the CD3-EGFP-stably expressing line were analyzed again. FlowJo was used as the analysis software. The average values (N=2) are shown in Table 1.

**[Table 1]**

| | CD3E-positive proportion [%] (Avg, n=2) |
|---|---|
| Mock | 0.38 |
| ba1 | 88.65 |
| ba2 | 84.50 |
| ba3 | 82.30 |
| ba4 | 10.16 |
| ba5 | 48.40 |
| ba6 | 47.65 |
| ba7 | 88.60 |
| ba8 | 90.30 |
| ba9 | 90.65 |
| ba10 | 86.50 |
| ba11 | 83.20 |
| ba12 | 51.05 |
| ba13 | 40.40 |
| ba14 | 46.40 |
| ba15 | 56.45 |
| ba16 | 70.70 |
| ba17 | 81.90 |
| ba18 | 89.50 |

### [Example 5]

Using CD3-forced expressing 293T cells, the localization of CD3 protein on the cell surface by modified TCRαs and modified TCRβs was assessed. Here, because the sensitivity of the 293T cells which constitutively expressed CD3 subunits (CD3-forced expressing 293T cells) was higher than that of the endogenous TCR-knockout Jurkat cells from the results of Example 1 and Example 2, the CD3-forced expressing 293T cells were used for the analysis in Example 5.

First, modified TCRα and TCRβ sequences were designed. Referring to known documents (described in Michael S. Kuhns et al., Immunity. 26: 357-369, 2007, Aswin Natarajan et al., Cell Reports.14: 2833-2845, 2016), the amino acids of TCRα and TCRβ which were reported to interact with CD3 protein were checked.

Moreover, the crystal structures of TCRα and TCRβ proteins were checked using PyMOL, which is an open-source molecular graphics tool. Known information (Protein Data Bank ID: 3QJF) was referred to for the crystal structures of TCRα and TCRβ proteins.

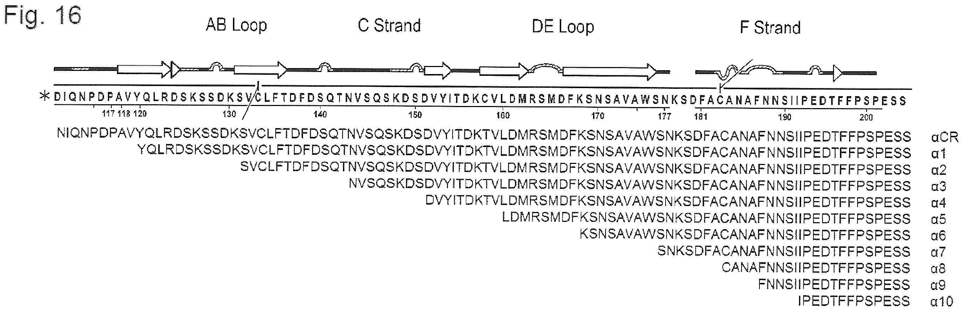

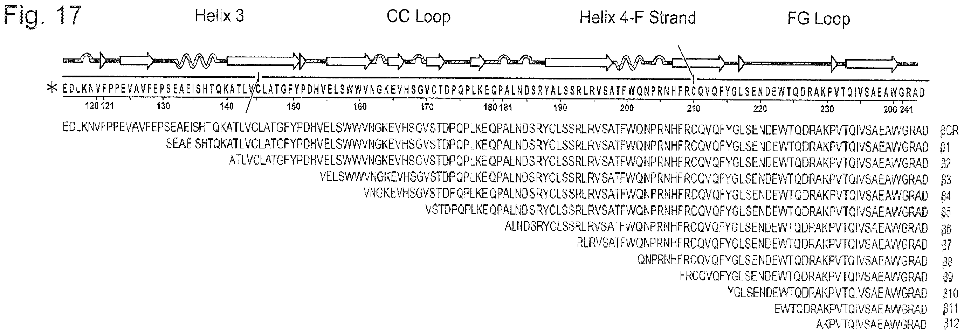

N-terminus partial sequences of the amino acid sequences of the extracellular domains of the modified TCRα and modified TCRβ proteins are shown in Fig. 16 and Fig. 17. Moreover, the amino acid sequences of the modified TCRα and the fragments thereof are shown in Table 2 and Table 8, and the amino acid sequences of the modified TCRβ and the fragments thereof are shown in Table 3 and Table 9.

**[Table 2]**

| Sequence Name | Number of Amino Acid Residues | SEQ ID NO: |
|---|---|---|
| αCR | 141 | 1 |
| α1 | 132 | 2 |
| α2 | 121 | 3 |
| α3 | 109 | 4 |
| α4 | 101 | 5 |
| α5 | 92 | 6 |
| α6 | 84 | 7 |
| α7 | 76 | 8 |
| α8 | 69 | 9 |
| α9 | 65 | 10 |
| α10 | 60 | 11 |

**[Table 3]**

| Sequence Name | Number of Amino Acid Residues | SEQ ID NO: |
|---|---|---|
| βCR | 179 | 12 |
| β1 | 163 | 13 |
| β2 | 153 | 14 |
| β3 | 139 | 15 |
| β4 | 133 | 16 |
| β5 | 124 | 17 |
| β6 | 112 | 18 |
| β7 | 101 | 19 |
| β8 | 92 | 20 |
| β9 | 86 | 21 |
| β10 | 79 | 22 |
| β11 | 72 | 23 |
| β12 | 66 | 24 |

Here, the amino acid sequences of the TCRαs and the TCRβs of ba2 to ba18 are selected from the modified TCRα and the fragments thereof, which are shown in Table 2 and Table 8, and the modified TCRβ and the fragments thereof, which are shown in Table 3 and Table 9. The correspondence between the amino acid sequences of the modified TCRα and the fragments thereof, which are shown in Table 2 and Table 8, and the modified TCRβ and the fragments thereof, which are shown in Table 3 and Table 9, and the amino acid sequences of the TCRαs and the TCRβs of ba2 to ba18 is shown in Table 4.

**[Table 4]**

| | TCRα | TCRβ |
|---|---|---|
| ba2 | αCR | βCR |
| ba3 | αCR | β2 |
| ba4 | αCR | β5 |
| ba5 | αCR | β9 |
| ba6 | αCR | β12 |
| ba7 | α2 | βCR |
| ba8 | α4 | βCR |
| ba9 | α6 | βCR |
| ba10 | α10 | βCR |
| ba11 | α2 | β2 |
| ba12 | α4 | β2 |
| ba13 | α6 | β2 |
| ba14 | α10 | β2 |
| ba15 | α2 | β12 |
| ba16 | α4 | β12 |
| ba17 | α6 | β12 |
| ba18 | α10 | β12 |

Plasmid vectors expressing the designed modified TCRas and modified TCRβs were constructed. Using ba2 as a template, Kpn1 was added to the 5' termini of αCR and βCR, and Xho1 was added to the 3' termini by amplification by PCR. Using Kpn1 and Xho1, pcDNA3.1(+) Mammalian Expression vector (Thermo Fisher Scientific) was cleaved, and the amplified DNAs were cloned. Next, using αCR and βCR as templates, α1 to 10 and β1 to 12 were produced by inverse PCR. The primers used for the inverse PCR are shown in Table 5. PrimeSTAR GXL Polymerase was used for the PCR, and the DNA sequences were determined by sequencing the obtained plasmid DNAs. The nucleotide sequences of the DNAs encoding the modified TCRas are shown in Tables 6, 10 and 11, and the nucleotide sequences of the DNAs encoding the modified TCRβs are shown in Tables 7, 12 and 13.

**[Table 5]**

| | Forward Primer | | Reverse Primer | |
|---|---|---|---|---|
| | Nucleotide Sequence | SEQ ID NO: | Nucleotide Sequence | SEQ ID NO: |
| α1 | TACCAGCTGAGAGACTCTAAATCC | 69 | AGCCATGCTAAATT CAAGACAGGTG | 91 |
| α2 | TCTGTCTGCCTATTCACCGATTTTG | 70 | | |
| α3 | AATGTGTCACAAAGTAAGGATTCTGATGTG | 71 | | |
| α4 | GATGTGTATATCACAGACAAAACTGTGCTAG | 72 | | |
| α5 | CTAGACATGAGGTCTATGGACTTCAAG | 73 | | |
| α6 | AAGAGCAACAGTGCTGTGGCC | 74 | | |
| α7 | AGCAACAAATCTGACTTTGCATGTG | 75 | | |
| α8 | TGTGCAAACGCCTTCAACAACAG | 76 | | |
| α9 | TTCAACAACAGCATTATTCCAGAAGAC | 77 | | |
| α10 | ATTCCAGAAGACACCTTCTTCCCC | 78 | | |
| β1 | TCAGAAGCAGAGATCTCCCACACCC | 79 | ACTGAACACAGAG CCTAGTCCCAG | 92 |
| β2 | GCCACACTGGTGTGCCTGGCC | 80 | | |
| β3 | GTGGAGCTGAGCTGGTGGGTG | 81 | | |
| β4 | GTGAATGGGAAGGAGGTGCACAGTG | 82 | | |
| β5 | GTCAGCACAGACCCGCAGCCCC | 83 | | |
| β6 | GCCCTCAATGACTCCAGATACTGCC | 84 | | |
| β7 | CGCCTGAGGGTCTCGGCCAC | 85 | | |
| β8 | CAGAACCCCCGCAACCACTTCC | 86 | | |
| β9 | TTCCGCTGTCAAGTCCAGTTCTACG | 87 | | |
| β10 | TACGGGCTCTCGGAGAATGACG | 88 | | |
| β11 | GAGTGGACCCAGGATAGGGCCAAACC | 89 | | |
| β12 | GCCAAACCTGTCACCCAGATCGTC | 90 | | |

**[Table 6]**

| Sequence Name | Number of Bases | SEQ ID NO: |
|---|---|---|
| αCR | 423 | 25 |
| α1 | 396 | 26 |
| α2 | 363 | 27 |
| α3 | 327 | 28 |
| α4 | 303 | 29 |
| α5 | 276 | 30 |
| α6 | 252 | 31 |
| α7 | 228 | 32 |
| α8 | 207 | 33 |
| α9 | 195 | 34 |
| α10 | 180 | 35 |

**[Table 7]**

| Sequence Name | Number of Bases | SEQ ID NO: |
|---|---|---|
| βCR | 537 | 36 |
| β1 | 489 | 37 |
| β2 | 459 | 38 |
| β3 | 417 | 39 |
| β4 | 399 | 40 |
| β5 | 372 | 41 |
| β6 | 336 | 42 |
| β7 | 303 | 43 |
| β8 | 276 | 44 |
| β9 | 258 | 45 |
| β10 | 237 | 46 |
| β11 | 216 | 47 |
| β12 | 198 | 48 |

**[Table 8]**

| Sequence Name | Number of Amino Acid Residues | Amino Acid Sequence |
|---|---|---|
| αCR | 141 | NIQNPDPAVYQLRDSKSSDKSVCLFTDFDSQTNVSQSKDSDVYITDK TVLDMRSMDFKSNSAVAWSNKSDFACANAFNNSIIPEDTFFPSPESS CDVKLVEKSFETDTNLNFQNLSVIGFRILLLKVAGFNLLMTLRLWSS |
| α1 | 132 | YQLRDSKSSDKSVCLFTDFDSQTNVSQSKDSDVYITDKTVLDMRSM DFKSNSAVAWSNKSDFACANAFNNSIIPEDTFFPSPESSCDVKLVEKS FETDTNLNFQNLSVIGFRILLLKVAGFNLLMTLRLWSS |
| α2 | 121 | SVCLFTDFDSQTNVSQSKDSDVYITDKTVLDMRSMDFKSNSAVAWS NKSDFACANAFNNSIIPEDTFFPSPESSCDVKLVEKSFETDTNLNFQN LSVIGFRILLLKVAGFNLLMTLRLWSS |
| α3 | 109 | NVSQSKDSDVYITDKTVLDMRSMDFKSNSAVAWSNKSDFACANAF NNSIIPEDTFFPSPESSCDVKLVEKSFETDTNLNFQNLSVIGFRILLLKV AGFNLLMTLRLWSS |
| α4 | 101 | DVYITDKTVLDMRSMDFKSNSAVAWSNKSDFACANAFNNSIIPEDTF FPSPESSCDVKLVEKSFETDTNLNFQNLSVIGFRILLLKVAGFNLLMTL RLWSS |
| α5 | 92 | LDMRSMDFKSNSAVAWSNKSDFACANAFNNSIIPEDTFFPSPESSCD VKLVEKSFETDTNLNFQNLSVIGFRILLLKVAGFNLLMTLRLWSS |
| α6 | 84 | KSNSAVAWSNKSDFACANAFNNSIIPEDTFFPSPESSCDVKLVEKSFE TDTNLNFQNLSVIGFRILLLKVAGFNLLMTLRLWSS |
| α7 | 76 | SNKSDFACANAFNNSIIPEDTFFPSPESSCDVKLVEKSFETDTNLNFQ NLSVIGFRILLLKVAGFNLLMTLRLWSS |
| α8 | 69 | CANAFNNSIIPEDTFFPSPESSCDVKLVEKSFETDTNLNFQNLSVIGFR ILLLKVAGFNLLMTLRLWSS |
| α9 | 65 | FNNSIIPEDTFFPSPESSCDVKLVEKSFETDTNLNFQNLSVIGFRILLLK VAGFNLLMTLRLWSS |
| α10 | 60 | IPEDTFFPSPESSCDVKLVEKSFETDTNLNFQNLSVIGFRILLLKVAGFN LLMTLRLWSS |

**[Table 9]**

| Sequence Name | Number of Amino Acid Residues | Amino Acid Sequence |
|---|---|---|
| βCR | 179 | EDLKNVFPPEVAVFEPSEAEISHTQKATLVCLATGFYPDHVELSWWV NGKEVHSGVSTDPQPLKEQPALNDSRYCLSSRLRVSATFWQNPRNH FRCQVQFYGLSENDEWTQDRAKPVTQIVSAEAWGRADCGFTSESYQ QGVLSATILYEILLGKATLYAVLVSALVLMAMVKRKDSRG |
| β1 | 163 | SEAEISHTQKATLVCLATGFYPDHVELSWWVNGKEVHSGVSTDPQPL KEQPALNDSRYCLSSRLRVSATFWQNPRNHFRCQVQFYGLSENDEW TQDRAKPVTQIVSAEAWGRADCGFTSESYQQGVLSATILYEILLGKAT LYAVLVSALVLMAMVKRKDSRG |
| β2 | 153 | ATLVCLATGFYPDHVELSWWVNGKEVHSGVSTDPQPLKEQPALNDS RYCLSSRLRVSATFWQNPRNHFRCQVQFYGLSENDEWTQDRAKPVT QIVSAEAWGRADCGFTSESYQQGVLSATILYEILLGKATLYAVLVSALV LMAMVKRKDSRG |
| β3 | 139 | VELSWWVNGKEVHSGVSTDPQPLKEQPALNDSRYCLSSRLRVSATF WQNPRNHFRCQVQFYGLSENDEWTQDRAKPVTQIVSAEAWGRADC GFTSESYQQGVLSATILYEILLGKATLYAVLVSALVLMAMVKRKDSRG |
| β4 | 133 | VNGKEVHSGVSTDPQPLKEQPALNDSRYCLSSRLRVSATFWQNPRN HFRCQVQFYGLSENDEWTQDRAKPVTQIVSAEAWGRADCGFTSESY QQGVLSATILYEILLGKATLYAVLVSALVLMAMVKRKDSRG |
| β5 | 124 | VSTDPQPLKEQPALNDSRYCLSSRLRVSATFWQNPRNHFRCQVQFY GLSENDEWTQDRAKPVTQIVSAEAWGRADCGFTSESYQQGVLSATIL YEILLGKATLYAVLVSALVLMAMVKRKDSRG |
| β6 | 112 | ALNDSRYCLSSRLRVSATFWQNPRNHFRCQVQFYGLSENDEWTQDR AKPVTQIVSAEAWGRADCGFTSESYQQGVLSATILYEILLGKATLYAVL VSALVLMAMVKRKDSRG |
| β7 | 101 | RLRVSATFWQNPRNHFRCQVQFYGLSENDEWTQDRAKPVTQIVSAE AWGRADCGFTSESYQQGVLSATILYEILLGKATLYAVLVSALVLMAMV KRKDSRG |
| β8 | 92 | QNPRNHFRCQVQFYGLSENDEWTQDRAKPVTQIVSAEAWGRADCG FTSESYQQGVLSATILYEILLGKATLYAVLVSALVLMAMVKRKDSRG |
| β9 | 86 | FRCQVQFYGLSENDEWTQDRAKPVTQIVSAEAWGRADCGFTSESYQ QGVLSATILYEILLGKATLYAVLVSALVLMAMVKRKDSRG |
| β10 | 79 | YGLSENDEWTQDRAKPVTQIVSAEAWGRADCGFTSESYQQGVLSATI LYEILLGKATLYAVLVSALVLMAMVKRKDSRG |
| β11 | 72 | EWTQDRAKPVTQIVSAEAWGRADCGFTSESYQQGVLSATILYEILLGK ATLYAVLVSALVLMAMVKRKDSRG |
| β12 | 66 | AKPVTQIVSAEAWGRADCGFTSESYQQGVLSATILYEILLGKATLYAVL VSALVLMAMVKRKDSRG |

**[Table 10]**

| Sequence Name | Number of Bases | Nucleotide Sequence |
|---|---|---|
| αCR | 423 | AATATCCAGAACCCTGACCCTGCCGTGTACCAGCTGAGAGACTCTAAATCCAGTGACAAGTCTGTCTGCCTA TTCACCGATTTTGATTCTCAAACAAATGTGTCACAAAGTAAGGATTCTGATGTGTATATCACAGACAAAACTG TGCTAGACATGAGGTCTATGGACTTCAAGAGCAACAGTGCTGTGGCCTGGAGCAACAAATCTGACTTTGCA TGTGCAAACGCCTTCAACAACAGCATTATTCCAGAAGACACCTTCTTCCCCAGCCCAGAAAGTTCCTGTGAT GTCAAGCTGGTCGAGAAAAGCTTTGAAACAGATACGAACCTAAACTTTCAAAACCTGTCAGTGATTGGGTTC CGAATCCTCCTCCTGAAAGTGGCCGGGTTTAATCTGCTCATGACGCTGCGGCTGTGGTCCAGC |
| α1 | 396 | TACCAGCTGAGAGACTCTAAATCCAGTGACAAGTCTGTCTGCCTATTCACCGATTTTGATTCTCAAACAAATG TGTCACAAAGTAAGGATTCTGATGTGTATATCACAGACAAAACTGTGCTAGACATGAGGTCTATGGACTTCA AGAGCAACAGTGCTGTGGCCTGGAGCAACAAATCTGACTTTGCATGTGCAAACGCCTTCAACAACAGCATT ATTCCAGAAGACACCTTCTTCCCCAGCCCAGAAAGTTCCTGTGATGTCAAGCTGGTCGAGAAAAGCTTTGAA ACAGATACGAACCTAAACTTTCAAAACCTGTCAGTGATTGGGTTCCGAATCCTCCTCCTGAAAGTGGCCGGG TTTAATCTGCTCATGACGCTGCGGCTGTGGTCCAGC |
| α2 | 363 | TCTGTCTGCCTATTCACCGATTTTGATTCTCAAACAAATGTGTCACAAAGTAAGGATTCTGATGTGTATATCAC AGACAAAACTGTGCTAGACATGAGGTCTATGGACTTCAAGAGCAACAGTGCTGTGGCCTGGAGCAACAAAT CTGACTTTGCATGTGCAAACGCCTTCAACAACAGCATTATTCCAGAAGACACCTTCTTCCCCAGCCCAGAAA GTTCCTGTGATGTCAAGCTGGTCGAGAAAAGCTTTGAAACAGATACGAACCTAAACTTTCAAAACCTGTCAG TGATTGGGTTCCGAATCCTCCTCCTGAAAGTGGCCGGGTTTAATCTGCTCATGACGCTGCGGCTGTGGTCCA GC |
| α3 | 327 | AATGTGTCACAAAGTAAGGATTCTGATGTGTATATCACAGACAAAACTGTGCTAGACATGAGGTCTATGGAC TTCAAGAGCAACAGTGCTGTGGCCTGGAGCAACAAATCTGACTTTGCATGTGCAAACGCCTTCAACAACAG CATTATTCCAGAAGACACCTTCTTCCCCAGCCCAGAAAGTTCCTGTGATGTCAAGCTGGTCGAGAAAAGCTT TGAAACAGATACGAACCTAAACTTTCAAAACCTGTCAGTGATTGGGTTCCGAATCCTCCTCCTGAAAGTGGC CGGGTTTAATCTGCTCATGACGCTGCGGCTGTGGTCCAGC |
| α4 | 303 | GATGTGTATATCACAGACAAAACTGTGCTAGACATGAGGTCTATGGACTTCAAGAGCAACAGTGCTGTGGC CTGGAGCAACAAATCTGACTTTGCATGTGCAAACGCCTTCAACAACAGCATTATTCCAGAAGACACCTTCTTC CCCAGCCCAGAAAGTTCCTGTGATGTCAAGCTGGTCGAGAAAAGCTTTGAAACAGATACGAACCTAAACTTT CAAAACCTGTCAGTGATTGGGTTCCGAATCCTCCTCCTGAAAGTGGCCGGGTTTAATCTGCTCATGACGCTG CGGCTGTGGTCCAGC |

**[Table 11]**

| Sequence Name | Number of Bases | Nucleotide Sequence |
|---|---|---|
| α5 | 276 | CTAGACATGAGGTCTATGGACTTCAAGAGCAACAGTGCTGTGGCCTGGAGCAACAAATCTGACTTTGCATG TGCAAACGCCTTCAACAACAGCATTATTCCAGAAGACACCTTCTTCCCCAGCCCAGAAAGTTCCTGTGATGT CAAGCTGGTCGAGAAAAGCTTTGAAACAGATACGAACCTAAACTTTCAAAACCTGTCAGTGATTGGGTTCCG AATCCTCCTCCTGAAAGTGGCCGGGTTTAATCTGCTCATGACGCTGCGGCTGTGGTCCAGC |
| α6 | 252 | AAGAGCAACAGTGCTGTGGCCTGGAGCAACAAATCTGACTTTGCATGTGCAAACGCCTTCAACAACAGCAT TATTCCAGAAGACACCTTCTTCCCCAGCCCAGAAAGTTCCTGTGATGTCAAGCTGGTCGAGAAAAGCTTTGA AACAGATACGAACCTAAACTTTCAAAACCTGTCAGTGATTGGGTTCCGAATCCTCCTCCTGAAAGTGGCCGG GTTTAATCTGCTCATGACGCTGCGGCTGTGGTCCAGC |
| α7 | 228 | AGCAACAAATCTGACTTTGCATGTGCAAACGCCTTCAACAACAGCATTATTCCAGAAGACACCTTCTTCCCCA GCCCAGAAAGTTCCTGTGATGTCAAGCTGGTCGAGAAAAGCTTTGAAACAGATACGAACCTAAACTTTCAAA ACCTGTCAGTGATTGGGTTCCGAATCCTCCTCCTGAAAGTGGCCGGGTTTAATCTGCTCATGACGCTGCGGC TGTGGTCCAGC |
| α8 | 207 | TGTGCAAACGCCTTCAACAACAGCATTATTCCAGAAGACACCTTCTTCCCCAGCCCAGAAAGTTCCTGTGAT GTCAAGCTGGTCGAGAAAAGCTTTGAAACAGATACGAACCTAAACTTTCAAAACCTGTCAGTGATTGGGTTC CGAATCCTCCTCCTGAAAGTGGCCGGGTTTAATCTGCTCATGACGCTGCGGCTGTGGTCCAGC |
| α9 | 195 | TTCAACAACAGCATTATTCCAGAAGACACCTTCTTCCCCAGCCCAGAAAGTTCCTGTGATGTCAAGCTGGTC GAGAAAAGCTTTGAAACAGATACGAACCTAAACTTTCAAAACCTGTCAGTGATTGGGTTCCGAATCCTCCTC CTGAAAGTGGCCGGGTTTAATCTGCTCATGACGCTGCGGCTGTGGTCCAGC |
| α10 | 180 | ATTCCAGAAGACACCTTCTTCCCCAGCCCAGAAAGTTCCTGTGATGTCAAGCTGGTCGAGAAAAGCTTTGAA ACAGATACGAACCTAAACTTTCAAAACCTGTCAGTGATTGGGTTCCGAATCCTCCTCCTGAAAGTGGCCGGG TTTAATCTGCTCATGACGCTGCGGCTGTGGTCCAGC |

**[Table 12]**

| Sequence Name | Number of Bases | Nucleotide Sequence |
|---|---|---|
| βCR | 537 | GAGGACCTGAAAAACGTGTTCCCACCCGAGGTCGCTGTGTTTGAGCCATCAGAAGCA GAGATCTCCCACACCCAAAAGGCCACACTGGTGTGCCTGGCCACAGGCTTCTACCCC GACCACGTGGAGCTGAGCTGGTGGGTGAATGGGAAGGAGGTGCACAGTGGGGTCAGC ACAGACCCGCAGCCCCTCAAGGAGCAGCCCGCCCTCAATGACTCCAGATACTGCCT GAGCAGCCGCCTGAGGGTCTCGGCCACCTTCTGGCAGAACCCCCGCAACCACTTCC GCTGTCAAGTCCAGTTCTACGGGCTCTCGGAGAATGACGAGTGGACCCAGGATAGGG CCAAACCTGTCACCCAGATCGTCAGCGCCGAGGCCTGGGGTAGAGCAGACTGTGGCT TCACCTCCGAGTCTTACCAGCAAGGGGTCCTGTCTGCCACCATCCTCTATGAGATCT TGCTAGGGAAGGCCACCTTGTATGCCGTGCTGGTCAGTGCCCTCGTGCTGATGGCCA TGGTCAAGAGAAAGGATTCCAGAGGC |
| β1 | 489 | TCAGAAGCAGAGATCTCCCACACCCAAAAGGCACACTGGTGTGCCTGGCCACAGG CTTCTACCCCGACCACGTGGAGCTGAGCTGGTGGGTGAATGGGAAGGAGGTGCACAG TGGGGTCAGCACAGACCCGCAGCCCCTCAAGGAGCAGCCCGCCCTCAATGACTCCA GATACTGCCTGAGCAGCCGCCTGAGGGTCTCGGCCACCTTCTGGCAGAACCCCCGCA ACCACTTCCGCTGTCAAGTCCAGTTCTACGGGCTCTCGGAGAATGACGAGTGGACCC AGGATAGGGCCAAACCTGTCACCCAGATCGTCAGCGCCGAGGCCTGGGGTAGAGCA GACTGTGGCTTCACCTCCGAGTCTTACCAGCAAGGGGTCCTGTCTGCCACCATCCTC TATGAGATCTTGCTAGGGAAGGCCACCTTGTATGCCGTGCTGGTCAGTGCCCTCGTG CTGATGGCCATGGTCAAGAGAAAGGATTCCAGAGGC |
| β2 | 459 | GCCACACTGGTGTGCCTGGCCACAGGCTTCTACCCCGACCACGTGGAGCTGAGCTGG TGGGTGAATGGGAAGGAGGTGCACAGTGGGGTCAGCACAGACCCGCAGCCCCTCAA GGAGCAGCCCGCCCTCAATGACTCCAGATACTGCCTGAGCAGCCGCCTGAGGGTCTC GGCCACCTTCTGGCAGAACCCCCGCAACCACTTCCGCTGTCAAGTCCAGTTCTACGG GCTCTCGGAGAATGACGAGTGGACCCAGGATAGGGCCAAACCTGTCACCCAGATCGT CAGCGCCGAGGCCTGGGGTAGAGCAGACTGTGGCTTCACCTCCGAGTCTTACCAGCA AGGGGTCCTGTCTGCCACCATCCTCTATGAGATCTTGCTAGGGAAGGCCACCTTGTA TGCCGTGCTGGTCAGTGCCCTCGTGCTGATGGCCATGGTCAAGAGAAAGGATTCCAG AGGC |
| β3 | 417 | GTGGAGCTGAGCTGGTGGGTGAATGGAAGGAGGTGCACAGTGGGGTCAGCACAGAC CCGCAGCCCCTCAAGGAGCAGCCCGCCCTCAATGACTCCAGATACTGCCTGAGCAG CCGCCTGAGGGTCTCGGCCACCTTCTGGCAGAACCCCCGCAACCACTTCCGCTGTCA AGTCCAGTTCTACGGGCTCTCGGAGAATGACGAGTGGACCCAGGATAGGGCCAAACC TGTCACCCAGATCGTCAGCGCCGAGGCCTGGGGTAGAGCAGACTGTGGCTTCACCTC CGAGTCTTACCAGCAAGGGGTCCTGTCTGCCACCATCCTCTATGAGATCTTGCTAGG GAAGGCCACCTTGTATGCCGTGCTGGTCAGTGCCCTCGTGCTGATGGCCATGGTCAA GAGAAAGGATTCCAGAGGC |
| β4 | 399 | GTGAATGGGAAGGAGGTGCACAGTGGGGTCAGCACAGACCCGCAGCCCCTCAAGGA GCAGCCCGCCCTCAATGACTCCAGATACTGCCTGAGCAGCCGCCTGAGGGTCTCGGC CACCTTCTGGCAGAACCCCCGCAACCACTTCCGCTGTCAAGTCCAGTTCTACGGGCT CTCGGAGAATGACGAGTGGACCCAGGATAGGGCCAAACCTGTCACCCAGATCGTCA GCGCCGAGGCCTGGGGTAGAGCAGACTGTGGCTTCACCTCCGAGTCTTACCAGCAAG GGGTCCTGTCTGCCACCATCCTCTATGAGATCTTGCTAGGGAAGGCCACCTTGTATG CCGTGCTGGTCAGTGCCCTCGTGCTGATGGCCATGGTCAAGAGAAAGGATTCCAGAG GC |

**[Table 13]**

| Sequence Name | Number of Bases | Nucleotide Sequence |
|---|---|---|
| β5 | 372 | GTCAGCACAGACCCGCAGCCCCTCAAGGAGCAGCCCGCCCTCAATGACTCCAGATA CTGCCTGAGCAGCCGCCTGAGGGTCTCGGCCACCTTCTGGCAGAACCCCCGCAACC ACTTCCGCTGTCAAGTCCAGTTCTACGGGCTCTCGGAGAATGACGAGTGGACCCAGG ATAGGGCCAAACCTGTCACCCAGATCGTCAGCGCCGAGGCCTGGGGTAGAGCAGAC TGTGGCTTCACCTCCGAGTCTTACCAGCAAGGGGTCCTGTCTGCCACCATCCTCTAT GAGATCTTGCTAGGGAAGGCCACCTTGTATGCCGTGCTGGTCAGTGCCCTCGTGCTG ATGGCCATGGTCAAGAGAAAGGATTCCAGAGGC |
| β6 | 336 | GCCCTCAATGACTCCAGATACTGCCTGAGCAGCCGCCTGAGGGTCTCGGCCACCTTC TGGCAGAACCCCCGCAACCACTTCCGCTGTCAAGTCCAGTTCTACGGGCTCTCGGAG AATGACGAGTGGACCCAGGATAGGGCCAAACCTGTCACCCAGATCGTCAGCGCCGA GGCCTGGGGTAGAGCAGACTGTGGCTTCACCTCCGAGTCTTACCAGCAAGGGGTCCT GTCTGCCACCATCCTCTATGAGATCTTGCTAGGGAAGGCCACCTTGTATGCCGTGCT GGTCAGTGCCCTCGTGCTGATGGCCATGGTCAAGAGAAAGGATTCCAGAGGC |
| β7 | 303 | CGCCTGAGGGTCTCGGCCACCTTCTGGCAGAACCCCCGCAACCACTTCCGCTGTCAA GTCCAGTTCTACGGGCTCTCGGAGAATGACGAGTGGACCCAGGATAGGGCCAAACCT GTCACCCAGATCGTCAGCGCCGAGGCCTGGGGTAGAGCAGACTGTGGCTTCACCTCC GAGTCTTACCAGCAAGGGGTCCTGTCTGCCACCATCCTCTATGAGATCTTGCTAGGG AAGGCCACCTTGTATGCCGTGCTGGTCAGTGCCCTCGTGCTGATGGCCATGGTCAAG AGAAAGGATTCCAGAGGC |
| β8 | 276 | CAGAACCCCCGCAACCACTTCCGCTGTCAAGTCCAGTTCTACGGCTCTCGGAGAAT GACGAGTGGACCCAGGATAGGGCCAAACCTGTCACCCAGATCGTCAGCGCCGAGGC CTGGGGTAGAGCAGACTGTGGCTTCACCTCCGAGTCTTACCAGCAAGGGGTCCTGTC TGCCACCATCCTCTATGAGATCTTGCTAGGGAAGGCCACCTTGTATGCCGTGCTGGT CAGTGCCCTCGTGCTGATGGCCATGGTCAAGAGAAAGGATTCCAGAGGC |
| β9 | 258 | TTCCGCTGTCAAGTCCAGTTCTACGGGCTCTCGGAGAATGACGAGTGGACCCAGGAT AGGGCCAAACCTGTCACCCAGATCGTCAGCGCCGAGGCCTGGGGTAGAGCAGACTG TGGCTTCACCTCCGAGTCTTACCAGCAAGGGGTCCTGTCTGCCACCATCCTCTATGA GATCTTGCTAGGGAAGGCCACCTTGTATGCCGTGCTGGTCAGTGCCCTCGTGCTGAT GGCCATGGTCAAGAGAAAGGATTCCAGAGGC |
| β10 | 237 | TACGGGCTCTCGGAGAATGACGAGTGGACCCAGGATAGGGCCAAACCTGTCACCCA GATCGTCAGCGCCGAGGCCTGGGGTAGAGCAGACTGTGGCTTCACCTCCGAGTCTTA CCAGCAAGGGGTCCTGTCTGCCACCATCCTCTATGAGATCTTGCTAGGGAAGGCCAC CTTGTATGCCGTGCTGGTCAGTGCCCTCGTGCTGATGGCCATGGTCAAGAGAAAGGA TTCCAGAGGC |
| β11 | 216 | GAGTGGACCCAGGATAGGGCCAAACCTGTCACCCAGATCGTCAGCGCCGAGGCCTG GGGTAGAGCAGACTGTGGCTTCACCTCCGAGTCTTACCAGCAAGGGGTCCTGTCTGC CACCATCCTCTATGAGATCTTGCTAGGGAAGGCCACCTTGTATGCCGTGCTGGTCAG TGCCCTCGTGCTGATGGCCATGGTCAAGAGAAAGGATTCCAGAGGC |
| β12 | 198 | GCCAAACCTGTCACCCAGATCGTCAGCGCCGAGGCCTGGGGTAGAGCAGACTGTGG CTTCACCTCCGAGTCTTACCAGCAAGGGGTCCTGTCTGCCACCATCCTCTATGAGAT CTTGCTAGGGAAGGCCACCTTGTATGCCGTGCTGGTCAGTGCCCTCGTGCTGATGGC CATGGTCAAGAGAAAGGATTCCAGAGGC |

Whether CD3 molecules would be held on the cell surface by introducing the modified TCR expression vectors into the CD3-EGFP-stably expressing line was examined. The CD3-EGFP-stably expressing line was transfected with combinations of the modified TCRα vectors and the modified TCRβ vectors together with pEF1α-IRES-hKO1 (mock vector in Example 1), which is a transfection marker, using Lipofectamine 2000 (Invitrogen).

A control was obtained by transfecting with Empty pcDNA3.1(+) vector and pEF1α-IRES-hKO1. The cells collected after two days were washed with FACS buffer [containing PBS (Nacalai Tesque, Inc.), 2% FBS (Access Cell Culture) and 1 mM EDTA (Invitrogen)] .

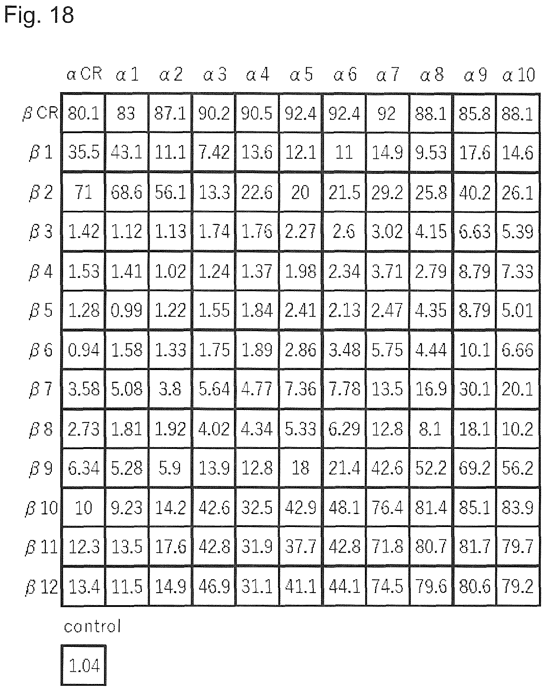

An APC-labeled anti-human CD3 antibody (clone: UCHT1) (BioLegend) was added to the cells for CD3 staining, and the cells were left to stand still in a dark place at 4°C for 30 minutes. After washing the cells with FACS buffer, the dead cells were stained by adding propidium iodide. BD LSR Fortessa was used for the FACS analysis. The CD3-positive proportions of the EGFP(+)KO(+) cells were analyzed by FlowJo9. The average values (N=3) are shown in Fig. 18.

From the results shown in Fig. 18, the findings described in (1) to (13) below were obtained.
(1) The modified TCRs of the combinations of βCR and the modified TCRαs all exhibited high CD3-recruiting activities of 80% or higher.
(2) The modified TCRs consisting of β1 and the modified TCRαs exhibited CD3-recruiting activities of 7.42-43.1 %. Of the modified TCRs, the combinations with the modified TCRαs of αCR and α1 exhibited sufficiently high CD3-recruiting activities of 20% or higher.
(3) The modified TCRs consisting of β2 and the modified TCRas exhibited CD3-recruiting activities of 13.3-71%. Of the modified TCRs, the combinations with the modified TCRαs other than α3 exhibited sufficiently high CD3-recruiting activities of 20% or higher.
(4) The modified TCRs consisting of β3 and the modified TCRas exhibited CD3-recruiting activities of 1.12-6.63%.
(5) The modified TCRs consisting of β4 and the modified TCRas exhibited CD3-recruiting activities of 1.02-8.97%.
(6) The modified TCRs consisting of β5 and the modified TCRas exhibited CD3-recruiting activities of 0.99-8.97%.
(7) The modified TCRs consisting of β6 and the modified TCRas exhibited CD3-recruiting activities of 0.94-10.1%.
(8) The modified TCRs consisting of β7 and the modified TCRas exhibited CD3-recruiting activities of 3.58-30.1%. Of the modified TCRs, the combinations with α9-10 exhibited sufficiently high CD3-recruiting activities of 20% or higher.
(9) The modified TCRs consisting of β8 and the modified TCRas exhibited CD3-recruiting activities of 1.81-18.1%.
(10) The modified TCRs consisting of β9 and the modified TCRas exhibited CD3-recruiting activities of 5.28-69.2%. Of the modified TCRs, the combinations with α8-10 exhibited sufficiently high CD3-recruiting activities of 20% or higher.
(11) The modified TCRs consisting of β10 and the modified TCRas exhibited CD3 -recruiting activities of 9.23-85.1%. Of the modified TCRs, the combinations with α3-10 exhibited sufficiently high CD3-recruiting activities of 20% or higher.
(12) The modified TCRs consisting of β11 and the modified TCRas exhibited CD3-recruiting activities of 12.3-81.7%. Of the modified TCRs, the combinations with α3-10 exhibited sufficiently high CD3-recruiting activities of 20% or higher.
(13) The modified TCRs consisting of β12 and the modified TCRas exhibited CD3-recruiting activities of 11.5-80.6%. Of the modified TCRs, the combinations with α3-10 exhibited sufficiently high CD3-recruiting activities of 20% or higher.

From the above results, recruiting of CD3 protein to the cell surface was observed in many of the assessed combinations of the modified TCRas and the modified TCRβs. It was also found that the expression level of CD3 on the cell surface varies with the deletion pattern of the TCR gene.

### [Example 6]

Retroviral vectors of ba1, ba2, ba4 and ba9, which are modified TCRs, were constructed. The inserts were cut out of ba1 vector, ba2 vector, ba4 vector and ba9 vector (pEF1α-IRES-hKO1) constructed in Example 1 using EcoRI and NotI in the upstream of IRES and subcloned into pMY-IRES-EGFP (referred to as pMY-IG below, Cell Biolabs, Inc) which was cleaved with EcoRI and NotI. To produce retroviruses, bal-pMY-IG, ba2-pMY-IG, ba4-pMY-IG, ba9-pMY-IG and Empty-pMY-IG were each transfected with VSV-G vector (Takara Bio Inc.) to GP2-293 packaging cells (Takara Bio Inc.) using Lipofectamine 2000. The cell supernatants containing the retroviruses were collected after two days, and concentrated retroviruses were produced using Retro-X Concentrator (Takara Bio Inc.).

### [Example 7]

The T-cell inducibility from iPS cells of ba1 and ba2, which are modified TCRs, was examined. First, hematopoietic progenitor cells (HPCs) cells for introducing ba1 and ba2 genes were induced from iPS cell lines (FF-WJs524 and FF-WJs527), which are derived from cord blood and have been confirmed not to be derived from T cells. For the maintenance of the iPS cells, StemFit AK02N medium (Ajinomoto) was used for feeder-free iPS cells.

iPS cells were detached using a detaching agent [obtained by diluting 1 × TrypLE Select (Thermo Fisher Scientific) with PBS at 1/2 and adding a 0.5 mol/l-EDTA solution (pH 8, Nacalai Tesque), final concentrations of 0.5× TrypLE Select and 0.75 mM EDTA] on a culture surface coated with iMatrix-511, and the cells were passed to a medium obtained by adding 10 µM Y-27632 to StemFit AK02N and cultured (37°C, 5%CO₂).

The medium was changed on the next day with StemFit AK02N. The operation was repeated once a week, and the iPS cells were maintained.

Subsequently, HPCs were produced by inducing differentiation of the iPS cells by embryoid body formation. The feeder-free iPS cells were detached with 0.5× TrypLE Select and 0.75 mM EDTA and then seeded to an ultra-low attachment 6-well plate (CORNING) at 2 to 3×10⁵ cells/well.

Using Stemfit AK02N medium containing 10 µM Y-27632 (Nacalai Tesque) and 10 µM CHIR99021 (Tocris Bioscience), the cells were cultured under low oxygen conditions (5% O₂) (Day 0). On the next day, the cells were cultured using StemPro34 (Thermo Fisher Scientific) medium (EB medium) containing 1× Insurin, Transferrin, Selenium Solution (Thermo Fisher Scientific), 1× Glutamax (Thermo Fisher Scientific), 0.2× PSG, 45 mM monothioglycerol (Wako) and 50 µg/ml PAA (Wako) to which 50 ng/ml BMP-4 (Miltenyi Biotec), 50 ng/ml VEGF-165A (Wako) and 50 ng/ml bFGF (Wako) were added (Day 1,5% O₂).

On the next day, 6 µM SB431542 (Wako) was added, and the cells were cultured (Day 2, 5% O₂). After two days, the cells were cultured in EB medium containing 50 ng/ml VEGF-165A, 50 ng/ml bFGF and 50 ng/ml SCF (Day 4,5% O₂). After two days, the cells were cultured in EB medium containing 50 ng/ml VEGF-165A, 50 ng/ml bFGF, 50 ng/ml SCF, 30 ng/ml TPO (Wako) and 10 ng/ml FLT3L (Wako) (Day 6). The medium was changed every two to three days, and the cells were cultured until Day 13 in 5% CO₂. The cells were collected and frozen with Tc protector.

After introducing modified TCRs ba1 and ba2 genes into the obtained HPCs, the cells were differentiated into CD3(+)CD45(+) T cells. Specifically, to introduce the retroviruses, the frozen HPCs were thawed and cultured in EB medium containing 50 ng/ml VEGF-165A, 50 ng/ml bFGF, 50 ng/ml SCF, 30 ng/ml TPO (Wako) and 10 ng/ml FLT3L (Wako) (Day-2).

On the next day, the cells which were thawed on the previous day were seeded in a 48-well plate coated with Retronectin (100 µg/ml, Takara Bio Inc.) at 2 to 4×10⁴ cells/well. Then, any of the retroviruses of modified TCRs ba1 and ba2 and Empty was added, and the cells were cultured in EB medium containing 50 ng/ml VEGF-165A, 50 ng/ml bFGF, 50 ng/ml SCF, 30 ng/ml TPO (Wako) and 10 ng/ml FLT3L (Wako) (Day -1).

On the next day, the cells were seeded again in a 48-well plate coated with DLL4 (5 µg/ml, R&D systems) and Retronectin (5 µg/ml, Takara Bio Inc.) and cultured in alpha-MEM medium containing 50 ng/ml SCF (Wako), 50 ng/ml IL-7 (Wako), 50 ng/ml Flt3L (Wako), 100 ng/ml TPO (Wako), 30 µM SDF-1α (Wako), 15 µM SB203580 (Tocris Bioscience), 55 µM 2-mercaptoethanol (Wako), 50 µg/ml PAA, 15% FCS and 1% PSG (Day 0).

A new plate coated with DLL4 and Retronectin was produced every week, and the cells were seeded. The medium was changed every two to three days. The cells were cultured until Day 21. The differentiated cells (Day 21) were co-stained with CD3-BV510 (BioLegend) and CD45-APC-Cy7 (BioLegend). After washing, the cells were suspended again in FACS buffer containing propidium iodide (PI). BD LSRFortessa cytometer (BD Bioscience) was used for the analysis. The CD3- and CD45-positive proportions of the PI(-)GFP(+) cell populations were analyzed with FlowJo9.

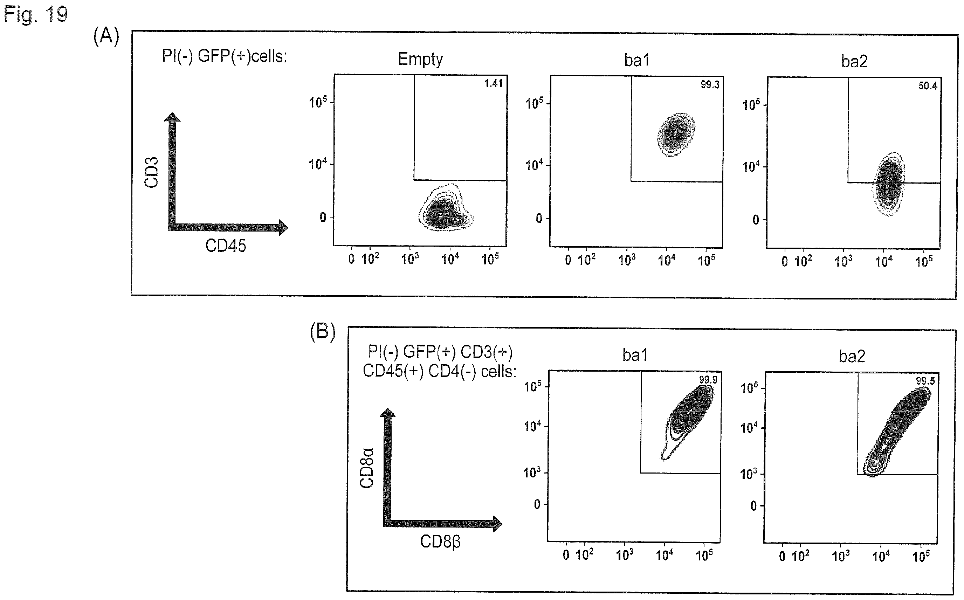

As a representative example of the results of analysis of CD3 and CD45 expression, the results of FF-WJs524 are shown in Fig. 19(A). As shown in Fig. 19(A), while the expression of CD3 was not observed in the Empty-introduced cells, the expression of CD3 was observed in the ba1-introduced cells and the ba2-introduced cells (ba1: 99.8%, ba2: 50.4%). In all of Empty, ba1 and ba2, the expression of CD45 leukocyte marker was observed.

Subsequently, CD8β(+)CD8α(+) was induced to mature T cells. Specifically, cells containing the CD3(+)CD45(+) T cells were seeded in a 48-well plate at 1 to 10×10⁴ cells/well and cultured in alpha-MEM medium containing 500 ng/ml anti-CD3 antibody OKT3 (eBioscience), 10 nM dexamethasone (Dexart R, Fuji Pharma), 10 ng/ml IL-7 (Wako), 50 µg/ml PAA, 15% FCS and 1% PSG (Day 0). After three days, the medium was changed to 10 ng/ml IL-7 (Wako), 50 µg/ml PAA, 15% FCS and 1% PSG, and the cells were cultured (Day 3). After seven days, the cells were counted by trypan blue staining (Day 10).

While the cell proliferation rates in the 10 days from Day 0 to Day 10 were 0.38 times and 0.165 times (the results of FF-WJs524 and FF-WJs527, respectively, the same applies below) in the Empty control, the rates were 5.13 times and 2.21 times in the ba1-introduced cells and 1.55 times and 4.25 times in the case of ba2.

The expression of CD8β and CD8α, which are mature T cell markers, only in the modified TCRs ba1- and ba2-introduced cells with sufficient cell numbers was analyzed. Specifically, the cells were co-stained with CD3-BV510, CD45-APC-Cy7, CD4-BV421 (BioLegend), CD8beta-PE-Cy7 (eBioscience) and CD8α-APC (BioLegend). After washing, the cells were suspended again in FACS buffer containing propidium iodide. BD LSRFortessa cytometer (BD Bioscience) was used for the analysis. The CD8β- and CD8α-positive proportions in the PI(-)GFP(+)CD3(+)CD45(+)CD4(-) cell populations were analyzed with FlowJo9.

As a representative example of the results of analysis of CD8β and CD8α expression, the results of FF-WJs524 are shown in Fig. 19(B). Both the ba1-introduced cells and the ba2-introduced cells expressed CD8β and CD8α (Ba1: 100%, Ba2: 99.5%).

From the above results, it was shown that, like ba1 consisting of the TCRα/TCRβ full-length sequences, ba2, which is a modified TCR consisting of TCRα/TCRβ without the variable region domains, is also useful for inducing mature T cells from totipotent stem cells. In particular, because mature T cells could be induced by introducing ba1 and ba2 by CD3 antibody OKT3, it was shown that modified TCRs not only have the action of simply holding CD3 molecules on the surface of cell membrane but also have the action of transmitting TCR/CD3 complex signals into the cytoplasm by stimulating CD3 molecules presented on the cell surface with an agonist antibody or the like.

### [Example 8]

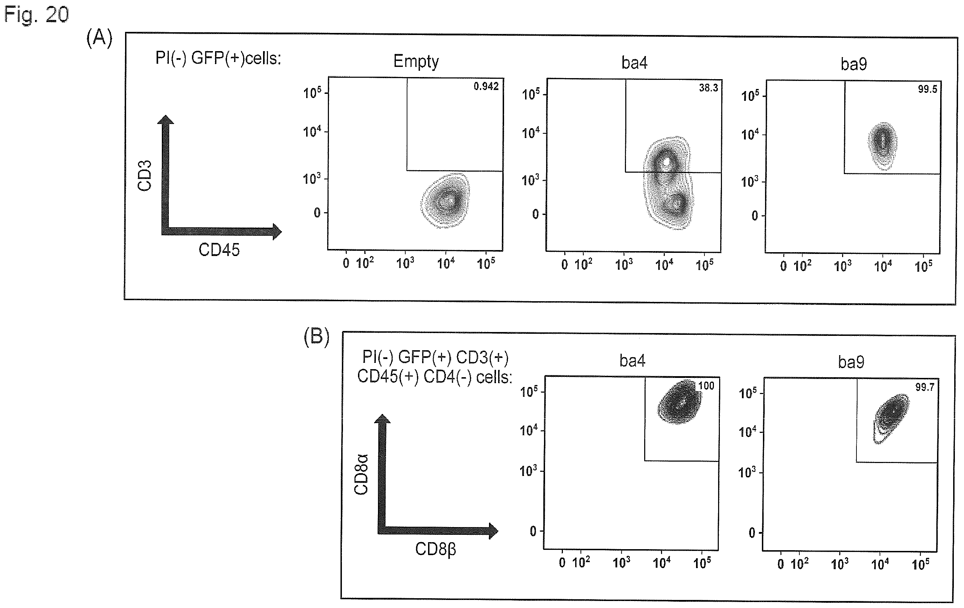

The T-cell inducibility was assessed using ba4, which is a modified TCR obtained by cutting out a part of the TCRβ constant region domain, and ba9, which is a modified TCR obtained by cutting out a part of the TCRα constant region domain. After introducing ba4 and ba9 genes into HPCs of iPS cell line FF-WJs524, differentiation was induced, and analysis was made by the same methods as those in Example 7. The results obtained after 21-day culture on DLL4 and Retronectin are shown in Fig. 20(A).

As shown in Fig. 20(A), while the expression of CD3 was not observed in the Empty-introduced cells (0.94%), the expression of CD3 was observed in the ba4-introduced cells and the ba9-introduced cells (ba4: 38.3%, ba9: 99.5%). In all of Empty, ba4 and ba9, the expression of CD45 leukocyte marker was observed.

Subsequently, differentiation into mature T cells of CD8β(+)CD8α(+) was induced.

The results of analysis of CD8β and CD8α expression are shown in Fig. 20(B). As shown in Fig. 20(B), both the ba4-introduced cells and the ba9-introduced cells expressed CD8β and CD8α (ba4: 100%, ba2: 99.7%).

From the above results, it was shown that, like ba1 and ba2, modified TCRs with deletion of a part of the constant region domains are also useful for inducing differentiation of pluripotent stem cells or totipotent stem cells into mature T cells. Moreover, ba4 showed the lowest CD3-positive proportion in the results of the re-analysis of Example 1 (Example 4) and showed the lowest-level CD3-positive proportion in additional Example 2. Nevertheless, in this Example, differentiation into mature T cells could be induced by ba4. This showed that a modified TCR which is capable of recruiting CD3 to the cell surface even at a low level is useful for inducing differentiation into mature T cells. Moreover, it was suggested that not only the modified TCRs shown in the invention but also modified TCRs having various modifications in the TCRα and TCRβ constant domains have CD3-recruiting activities.

From the above results, it was found that modified TCRs not only have the action of simply holding CD3 molecules on the surface of cell membrane but also have the action of transmitting TCR/CD3 complex signals into the cytoplasm by stimulating CD3 molecules presented on the cell surface with an agonist antibody or the like.

### Industrial Applicability

In order to efficiently induce differentiation of pluripotent stem cells or hematopoietic stem and progenitor cells into T cells, it is considered to be important that the functional TCR gene loci are rearranged at an appropriate timing during the differentiation stage and that the proliferation and the survival are promoted by stimulating thus formed TCR/CD3 complex *in vitro.* In pluripotent stem cells in which the TCR gene loci are not rearranged, however, it is expected that functional TCR is not efficiently produced during the differentiation, and this causes a significant decrease in the production efficiency of T cells. Even if the TCR gene loci are rearranged, the antigen specificity of the produced TCR molecule is at random, and thus there are still problems of not only the homogeneousness as a cell preparation but also the safeness because unpredictable alloreaction may be caused after the transplantation to a patient.

The modified TCR according to the invention can hold CD3 subunits on the cell membrane. Accordingly, when the modified TCR is expressed in the original pluripotent stem cells, the progenitor cells during the differentiation or the like during the differentiation of pluripotent stem cells into T cells, signals required for the maturation, the proliferation and the like of the T cells can be freely transmitted at any timing through stimulation through the modified TCR/CD3 complex, and as a result, the induction of the differentiation into T cells is promoted. Similarly, even after the differentiation into T cells, the T cells can also be appropriately proliferated by stimulating the modified TCR/CD3 complex. Because the antigen recognition ability of the modified TCR according to the invention is eliminated, the likelihood of the alloreaction caused after the transplantation of the cell preparation is considered to be extremely low. Therefore, using the modified TCR according to the invention, differentiation of pluripotent stem cells into homogeneous T cells can be induced efficiently.

In addition, because the size of the gene encoding the modified TCR according to the invention is compact as compared to that of the full-length TCR gene, the modified TCR is considered to be able to fit all the formats for gene introduction, such as gene introduction through an expression vector, a viral vector, an episomal vector or a transposon vector, gene insertion into a desired genome region or modification of the TCR gene loci themselves by genome editing technology and gene installation to an artificial chromosome vector.

The invention has been explained in detail using the specific embodiments, but it is obvious for one skilled in the art that various changes and modifications can be made without departing from the intension and the scope of the invention. The present application is based on a Japanese patent application filed on December 26, 2018 (patent application No. 2018-242733), which is incorporated by reference in its entirety.

## Claims

1. A modified T-cell receptor (TCR) comprising a first polypeptide and a second polypeptide,
wherein the first polypeptide is a polypeptide which comprises a constant region of human T-cell receptor α (TCRα) or a fragment of the constant region and which does not comprise a variable region of human TCRα, and
the second polypeptide is a polypeptide which comprises a constant region of human T-cell receptor β (TCRβ) or a fragment of the constant region and which does not comprise a variable region of human TCRβ.

2. The modified TCR according to claim 1, wherein the constant region of human TCRα or the fragment of the constant region is the polypeptide described in any one of (A1) to (A3) below,
(A1) a polypeptide consisting of the amino acid sequence of any one of SEQ ID NOs: 1 to 11,
(A2) a polypeptide consisting of an amino acid sequence having sequence identity of 90% or more with the amino acid sequence of any one of SEQ ID NOs: 1 to 11, and
(A3) a polypeptide consisting of an amino acid sequence in which one or some amino acids have been substituted, inserted, deleted and/or added in the amino acid sequence of any one of SEQ ID NOs: 1 to 11.

3. The modified TCR according to claim 1 or 2, wherein the constant region of human TCRβ or the fragment of the constant region is the polypeptide described in any one of (B1) to (B3) below,
(B1) a polypeptide consisting of the amino acid sequence of any one of SEQ ID NOs: 12 to 24,
(B2) a polypeptide consisting of an amino acid sequence having sequence identity of 90% or more with the amino acid sequence of any one of SEQ ID NOs: 12 to 24, and
(B3) a polypeptide consisting of an amino acid sequence in which one or some amino acids have been substituted, inserted, deleted and/or added in the amino acid sequence of any one of SEQ ID NOs: 12 to 24.

4. The modified TCR according to any one of claims 1 to 3, wherein the constant region of human TCRα or the fragment of the constant region and the constant region of human TCRβ or the fragment of the constant region are the polypeptides described in any one of (a1) to (a8) below:
(a1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
(a2) the constant region of human TCRβ or the fragment of the constant region is a polypeptide consisting of the amino acid sequence of SEQ ID NO: 13, a polypeptide consisting of an amino acid sequence having sequence identity of 90% or more with the amino acid sequence of SEQ ID NO: 13 or a polypeptide consisting of an amino acid sequence in which one or some amino acids have been substituted, inserted, deleted and/or added in the amino acid sequence of SEQ ID NO: 13, and the constant region of human TCRα or the fragment of the constant region is a polypeptide consisting of the amino acid sequence of SEQ ID NO: 1 or 2, a polypeptide consisting of an amino acid sequence having sequence identity of 90% or more with the amino acid sequence of SEQ ID NO: 1 or 2 or a polypeptide consisting of an amino acid sequence in which one or some amino acids have been substituted, inserted, deleted and/or added in the amino acid sequence of SEQ ID NO: 1 or 2;
(a3) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of any one of SEQ ID NOs: 1 to 3 and 5 to 11, a polypeptide consisting of an amino acid sequence having sequence identity of 90% or more with the amino acid sequence of any one of SEQ ID NOs: 1 to 3 and 5 to 11 or a polypeptide consisting of an amino acid sequence in which one or some amino acids have been substituted, inserted, deleted and/or added in the amino acid sequence of any one of SEQ ID NOs: 1 to 3 and 5 to 11;
(a4) the constant region of human TCRβ or the fragment of the constant region is a polypeptide consisting of the amino acid sequence of SEQ ID NO: 19, a polypeptide consisting of an amino acid sequence having sequence identity of 90% or more with the amino acid sequence of SEQ ID NO: 19 or a polypeptide consisting of an amino acid sequence in which one or some amino acids have been substituted, inserted, deleted and/or added in the amino acid sequence of SEQ ID NO: 19, and the constant region of human TCRα or the fragment of the constant region is a polypeptide consisting of the amino acid sequence of SEQ ID NO: 10 or 11, a polypeptide consisting of an amino acid sequence having sequence identity of 90% or more with the amino acid sequence of SEQ ID NO: 10 or 11 or a polypeptide consisting of an amino acid sequence in which one or some amino acids have been substituted, inserted, deleted and/or added in the amino acid sequence of SEQ ID NO: 10 or 11;
(a5) the constant region of human TCRβ or the fragment of the constant region is a polypeptide consisting of the amino acid sequence of SEQ ID NO: 21, a polypeptide consisting of an amino acid sequence having sequence identity of 90% or more with the amino acid sequence of SEQ ID NO: 21 or a polypeptide consisting of an amino acid sequence in which one or some amino acids have been substituted, inserted, deleted and/or added in the amino acid sequence of SEQ ID NO: 21, and the constant region of human TCRα or the fragment of the constant region is a polypeptide consisting of the amino acid sequence of any one of SEQ ID NOs: 7 to 11, a polypeptide consisting of an amino acid sequence having sequence identity of 90% or more with the amino acid sequence of any one of SEQ ID NOs: 7 to 11 or a polypeptide consisting of an amino acid sequence in which one or some amino acids have been substituted, inserted, deleted and/or added in the amino acid sequence of any one of SEQ ID NOs: 7 to 11;
(a6) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11;
(a7) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11; and
(a8) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 4 to 11, a polypeptide consisting of an amino acid sequence having sequence identity of 90% or more with the amino acid sequence of any one of SEQ ID NOs: 4 to 11 or a polypeptide consisting of an amino acid sequence in which one or some amino acids have been substituted, inserted, deleted and/or added in the amino acid sequence of any one of SEQ ID NOs: 4 to 11.

5. The modified TCR according to any one of claims 1 to 4, wherein the constant region of human TCRα or the fragment of the constant region and the constant region of human TCRβ or the fragment of the constant region are the polypeptides described in any one of (b1) to (b7) below:
(b1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
(b2) the constant region of human TCRβ or the fragment of the constant region is a polypeptide consisting of the amino acid sequence of SEQ ID NO: 13, a polypeptide consisting of an amino acid sequence having sequence identity of 90% or more with the amino acid sequence of SEQ ID NO: 13 or a polypeptide consisting of an amino acid sequence in which one or some amino acids have been substituted, inserted, deleted and/or added in the amino acid sequence of SEQ ID NO: 13, and the constant region of human TCRα or the fragment of the constant region is a polypeptide consisting of the amino acid sequence of SEQ ID NO: 2, a polypeptide consisting of an amino acid sequence having sequence identity of 90% or more with the amino acid sequence of SEQ ID NO: 2 or a polypeptide consisting of an amino acid sequence in which one or some amino acids have been substituted, inserted, deleted and/or added in the amino acid sequence of SEQ ID NO: 2;
(b3) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of any one of SEQ ID NOs: 1 to 3 and 10, a polypeptide consisting of an amino acid sequence having sequence identity of 90% or more with the amino acid sequence of any one of SEQ ID NOs: 1 to 3 and 10 or a polypeptide consisting of an amino acid sequence in which one or some amino acids have been substituted, inserted, deleted and/or added in the amino acid sequence of any one of SEQ ID NOs: 1 to 3 and 10;
(b4) the constant region of human TCRβ or the fragment of the constant region is a polypeptide consisting of the amino acid sequence of SEQ ID NO: 21, a polypeptide consisting of an amino acid sequence having sequence identity of 90% or more with the amino acid sequence of SEQ ID NO: 21 or a polypeptide consisting of an amino acid sequence in which one or some amino acids have been substituted, inserted, deleted and/or added in the amino acid sequence of SEQ ID NO: 21, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11;
(b5) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 4 and 6 to 11, a polypeptide consisting of an amino acid sequence having sequence identity of 90% or more with the amino acid sequence of any one of SEQ ID NOs: 4 and 6 to 11 or a polypeptide consisting of an amino acid sequence in which one or some amino acids have been substituted, inserted, deleted and/or added in the amino acid sequence of any one of SEQ ID NOs: 4 and 6 to 11;
(b6) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 4 and 7 to 11, a polypeptide consisting of an amino acid sequence having sequence identity of 90% or more with the amino acid sequence of any one of SEQ ID NOs: 4 and 7 to 11 or a polypeptide consisting of an amino acid sequence in which one or some amino acids have been substituted, inserted, deleted and/or added in the amino acid sequence of any one of SEQ ID NOs: 4 and 7 to 11; and
(b7) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 4 and 6 to 11, a polypeptide consisting of an amino acid sequence having sequence identity of 90% or more with the amino acid sequence of any one of SEQ ID NOs: 4 and 6 to 11 or a polypeptide consisting of an amino acid sequence in which one or some amino acids have been substituted, inserted, deleted and/or added in the amino acid sequence of any one of SEQ ID NOs: 4 and 6 to 11.

6. The modified TCR according to any one of claims 1 to 5, wherein the constant region of human TCRα or the fragment of the constant region and the constant region of human TCRβ or the fragment of the constant region are the polypeptides described in any one of (c1) to (c5) below:
(c1) the constant region of human TCRβ or the fragment of the constant region is a polypeptide consisting of the amino acid sequence of SEQ ID NO: 12, a polypeptide consisting of an amino acid sequence having sequence identity of 90% or more with the amino acid sequence of SEQ ID NO: 12 or a polypeptide consisting of an amino acid sequence in which one or some amino acids have been substituted, inserted, deleted and/or added in the amino acid sequence of SEQ ID NO: 12, and the constant region of human TCRα or the fragment of the constant region is a polypeptide consisting of the amino acid sequence of any one of SEQ ID NOs: 1 to 11, a polypeptide consisting of an amino acid sequence having sequence identity of 90% or more with the amino acid sequence of any one of SEQ ID NOs: 1 to 11 or a polypeptide consisting of an amino acid sequence in which one or some amino acids have been substituted, inserted, deleted and/or added in the amino acid sequence of any one of SEQ ID NOs: 1 to 11;
(c2) the constant region of human TCRβ or the fragment of the constant region is a polypeptide consisting of the amino acid sequence of SEQ ID NO: 14, a polypeptide consisting of an amino acid sequence having sequence identity of 90% or more with the amino acid sequence of SEQ ID NO: 14 or a polypeptide consisting of an amino acid sequence in which one or some amino acids have been substituted, inserted, deleted and/or added in the amino acid sequence of SEQ ID NO: 14, and the constant region of human TCRα or the fragment of the constant region is a polypeptide consisting of the amino acid sequence of SEQ ID NO: 1, a polypeptide consisting of an amino acid sequence having sequence identity of 90% or more with the amino acid sequence of SEQ ID NO: 1 or a polypeptide consisting of an amino acid sequence in which one or some amino acids have been substituted, inserted, deleted and/or added in the amino acid sequence of SEQ ID NO: 1;
(c3) the constant region of human TCRβ or the fragment of the constant region is a polypeptide consisting of the amino acid sequence of SEQ ID NO: 22, a polypeptide consisting of an amino acid sequence having sequence identity of 90% or more with the amino acid sequence of SEQ ID NO: 22 or a polypeptide consisting of an amino acid sequence in which one or some amino acids have been substituted, inserted, deleted and/or added in the amino acid sequence of SEQ ID NO: 22, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11;
(c4) the constant region of human TCRβ or the fragment of the constant region is a polypeptide consisting of the amino acid sequence of SEQ ID NO: 23, a polypeptide consisting of an amino acid sequence having sequence identity of 90% or more with the amino acid sequence of SEQ ID NO: 23 or a polypeptide consisting of an amino acid sequence in which one or some amino acids have been substituted, inserted, deleted and/or added in the amino acid sequence of SEQ ID NO: 23, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11; and
(c5) the constant region of human TCRβ or the fragment of the constant region is a polypeptide consisting of the amino acid sequence of SEQ ID NO: 24, a polypeptide consisting of an amino acid sequence having sequence identity of 90% or more with the amino acid sequence of SEQ ID NO: 24 or a polypeptide consisting of an amino acid sequence in which one or some amino acids have been substituted, inserted, deleted and/or added in the amino acid sequence of SEQ ID NO: 24, and the constant region of human TCRα or the fragment of the constant region is a polypeptide consisting of the amino acid sequence of any one of SEQ ID NOs: 8 to 11, a polypeptide consisting of an amino acid sequence having sequence identity of 90% or more with the amino acid sequence of any one of SEQ ID NOs: 8 to 11 or a polypeptide consisting of an amino acid sequence in which one or some amino acids have been substituted, inserted, deleted and/or added in the amino acid sequence of any one of SEQ ID NOs: 8 to 11.

7. The modified TCR according to any one of claims 1 to 6, wherein the first polypeptide comprises at least one selected from AB loop, C strand, DE loop and F strand in the constant region of human TCRα.

8. The modified TCR according to any one of claims 1 to 7, wherein the second polypeptide comprises at least one selected from Helix3, CC loop, Helix4 F strand and FG loop in the constant region of human TCRβ.

9. The modified TCR according to any one of claims 1 to 8, wherein the first polypeptide comprises at least a part of the extracellular domain, the transmembrane domain and the intracellular domain of the constant region of human TCRα, and the second polypeptide comprises at least a part of the extracellular domain, the transmembrane domain and the intracellular domain of the constant region of human TCRβ.

10. A modified TCR comprising a first polypeptide and a second polypeptide,
wherein the first polypeptide is a polypeptide which comprises a constant region of human TCRα or a fragment of the constant region and which does not comprise a variable region of human TCRα, and
the second polypeptide is a polypeptide which comprises a constant region of human TCRβ or a fragment of the constant region and which does not comprise a variable region of human TCRβ, and
wherein the proportion of CD3-positive cells in pluripotent stem cells, hematopoietic stem and progenitor cells or endogenous TCRα gene- and endogenous TCRβ gene-knockdown or knockout T cells into which the modified TCR has been introduced is 1% or more.

11. The modified TCR according to any one of claims 1 to 10, wherein the proportion of CD3-positive cells in pluripotent stem cells, hematopoietic stem and progenitor cells or endogenous TCRα gene- and endogenous TCRβ gene-knockdown or knockout T cells into which the modified TCR has been introduced is two times or more higher than the proportion of CD3-positive cells in corresponding cells into which the modified TCR has not been introduced.

12. The modified TCR according to any one of claims 1 to 11, wherein a pluripotent stem cell or a hematopoietic stem and progenitor cell into which the modified TCR has been introduced exhibits an equivalent or higher T-cell differentiation capacity as compared to that of a corresponding cell into which the full-length TCR has been introduced.

13. The modified TCR according to any one of claims 1 to 12, wherein the alloreactivity of a non-T cell-derived pluripotent stem cell, a hematopoietic stem and progenitor cell or an endogenous TCRα gene- and endogenous TCRβ gene-knockdown or knockout T cell into which the modified TCR has been introduced is lower than that of a corresponding cell into which the full-length TCR has been introduced.

14. The modified TCR according to any one of claims 1 to 13 which is capable of holding a CD3 subunit on cell membrane.

15. The modified TCR according to claim 14 which is capable of transmitting a TCR/CD3 complex-related signal into cytoplasm.

16. The modified TCR according to claim 14 or 15 which is capable of transmitting a TCR/CD3 complex-related signal into cytoplasm through the CD3 subunit held on the cell membrane.

17. The modified TCR according to claim 16 which transmits a TCR/CD3 complex-related signal into cytoplasm through the CD3 subunit held on the cell membrane and thus activates a T cell.

18. The modified TCR according to any one of claims 1 to 17 for the expression in a pluripotent stem cell, a hematopoietic stem and progenitor cell or an endogenous TCRα gene- and endogenous TCRβ gene-knockdown or knockout T cell.

19. The modified TCR according to claim 18 for the expression in a pluripotent stem cell or a hematopoietic stem and progenitor cell to differentiate the cell into a T cell.

20. The modified TCR according to claim 18 for the expression in a pluripotent stem cell, a hematopoietic stem and progenitor cell or an endogenous TCRα gene- and endogenous TCRβ gene-knockdown or knockout T cell to produce a T cell which is capable of responding to stimulation through a complex of the modified TCR and CD3.

21. The modified TCR according to any one of claims 1 to 20, wherein the pluripotent stem cell is a non-T cell-derived pluripotent stem cell or an endogenous TCRα gene- and endogenous TCRβ gene-knockdown or knockout T cell-derived pluripotent stem cell.

22. A cell which expresses the modified TCR according to any one of claims 1 to 21.

23. The cell according to claim 22, wherein the cell which expresses the modified TCR is a T cell.

24. A pharmaceutical composition comprising a cell which expresses the modified TCR according to any one of claims 1 to 21.

25. A production method of a cell which expresses a modified TCR, wherein the modified TCR comprises a first polypeptide and a second polypeptide, and the production method comprises the following steps (a) to (c):
(a) a step of preparing a cell;
(b) a step of preparing an expression vector comprising a polynucleotide encoding the first polypeptide which comprises a constant region of human TCRα or a fragment of the constant region and which does not comprise a variable region of human TCRα and an expression vector comprising a polynucleotide encoding the second polypeptide which comprises a constant region of human TCRβ or a fragment of the constant region and which does not comprise a variable region of human TCRβ; and
(c) a step of transforming the cell prepared in the step (a) using the expression vectors prepared in the step (b).

26. A production method of a cell which expresses a modified TCR, wherein the modified TCR comprises a first polypeptide and a second polypeptide, and the production method comprises the following steps (a') to (c'):
(a') a step of preparing a cell;
(b') a step of preparing an expression vector comprising a polynucleotide encoding the first polypeptide which comprises a constant region of human TCRα or a fragment of the constant region and which does not comprise a variable region of human TCRα and a polynucleotide encoding the second polypeptide which comprises a constant region of human TCRβ or a fragment of the constant region and which does not comprise a variable region of human TCRβ; and
(c') a step of transforming the cell prepared in the step (a') using the expression vector prepared in the step (b').

27. The production method according to claim 25 or 26, wherein the cell in the step (a) or (a') is a pluripotent stem cell, a hematopoietic stem and progenitor cell or a TCRα and TCRβ genes-knockdown or knockout T cell.

28. The production method according to any one of claims 25 to 27, wherein the cell which expresses the modified TCR is a T cell for an immune cell therapy.

29. A method for producing a T cell comprising a step of expressing the modified TCR according to any one of claims 1 to 21 in a pluripotent stem cell or a hematopoietic stem and progenitor cell and differentiating the cell into a T cell.

30. A method for producing a T cell comprising a step of expressing the modified TCR according to any one of claims 1 to 21 in a pluripotent stem cell, a hematopoietic stem and progenitor cell or an endogenous TCRα gene- and endogenous TCRβ gene-knockdown or knockout T cell, wherein the obtained T cell is a T cell which is capable of responding to stimulation through a complex of the modified TCR and CD3.

31. A method of immune cell therapy comprising a step of expressing the modified TCR according to any one of claims 1 to 21 in a pluripotent stem cell and differentiating the cell into a T cell and a step of administering the obtained T cell to a subject.

32. A method of immune cell therapy comprising a step of expressing the modified TCR according to any one of claims 1 to 21 in an endogenous TCRα gene- and endogenous TCRβ gene-knockdown or knockout T cell and administering the obtained T cell to a subject.

33. A method of immune cell therapycomprising a step of expressing the modified TCR according to any one of claims 1 to 21 in a hematopoietic stem and progenitor cell and differentiating the cell into a T cell and a step of administering the obtained T cell to a subject.

34. The modified TCR according to any one of claims 1 to 21 for use in an immune cell therapy.

35. A pluripotent stem cell-derived T cell for use in an immune cell therapy which is obtained by expressing the modified TCR according to any one of claims 1 to 21 in a pluripotent stem cell and differentiating the cell into the T cell.

36. A T cell for use in an immune cell therapy which is obtained by expressing the modified TCR according to any one of claims 1 to 21 in an endogenous TCRα gene- and endogenous TCRβ gene-knockdown or knockout T cell.

37. A hematopoietic stem and progenitor cell-derived T cell for use in an immune cell therapy which is obtained by expressing the modified TCR according to any one of claims 1 to 21 in a hematopoietic stem and progenitor cell and differentiating the cell into the T cell.

38. Use of the modified TCR according to any one of claims 1 to 21 for the manufacture of a composition for an immune cell therapy.

39. Use of a pluripotent stem cell-derived T cell for the manufacture of a composition for an immune cell therapy, wherein the pluripotent stem cell-derived T cell is obtained by expressing the modified TCR according to any one of claims 1 to 21 in a pluripotent stem cell and differentiating the cell into a T cell.

40. Use of a T cell for the manufacture of a composition for an immune cell therapy, wherein the T cell is obtained by expressing the modified TCR according to any one of 1 to 21 above in an endogenous TCRα gene- and endogenous TCRβ gene-knockdown or knockout T cell.

41. Use of a T cell for the manufacture of a composition for an immune cell therapy, wherein the T cell is obtained by expressing the modified TCR according to any one of 1 to 21 above in a hematopoietic stem and progenitor cell and differentiating the cell into the T cell.

42. A nucleic acid encoding the modified TCR according to any one of claims 1 to 21.

43. A vector comprising the nucleic acid according to claim 42.

44. A production method of a pharmaceutical composition using the nucleic acid according to claim 42 or the vector according to claim 43.

45. A pharmaceutical composition comprising the nucleic acid according to claim 42 or the vector according to claim 43.


SEQUENCE LISTING

<110>  KIRIN HOLDINGS KABUSHIKI KAISHA
       Kyoto University

<120>  Modified TCR and production method thereof

<130>  W527533

<150>  JP2018-242733
<151>  2018-12-26

<160>  92

<170>  PatentIn version 3.5

<210>  1
<211>  141
<212>  PRT
<213>  Homo sapiens

<400>  1

Asn Ile Gln Asn Pro Asp Pro Ala Val Tyr Gln Leu Arg Asp Ser Lys
1               5                   10                  15

Ser Ser Asp Lys Ser Val Cys Leu Phe Thr Asp Phe Asp Ser Gln Thr
            20                  25                  30

Asn Val Ser Gln Ser Lys Asp Ser Asp Val Tyr Ile Thr Asp Lys Thr
        35                  40                  45

Val Leu Asp Met Arg Ser Met Asp Phe Lys Ser Asn Ser Ala Val Ala
    50                  55                  60

Trp Ser Asn Lys Ser Asp Phe Ala Cys Ala Asn Ala Phe Asn Asn Ser
65                  70                  75                  80

Ile Ile Pro Glu Asp Thr Phe Phe Pro Ser Pro Glu Ser Ser Cys Asp
                85                  90                  95

Val Lys Leu Val Glu Lys Ser Phe Glu Thr Asp Thr Asn Leu Asn Phe
            100                 105                 110

Gln Asn Leu Ser Val Ile Gly Phe Arg Ile Leu Leu Leu Lys Val Ala
        115                 120                 125

Gly Phe Asn Leu Leu Met Thr Leu Arg Leu Trp Ser Ser
    130                 135                 140

<210>  2
<211>  132
<212>  PRT
<213>  Homo sapiens

<400>  2

Tyr Gln Leu Arg Asp Ser Lys Ser Ser Asp Lys Ser Val Cys Leu Phe
1               5                   10                  15

Thr Asp Phe Asp Ser Gln Thr Asn Val Ser Gln Ser Lys Asp Ser Asp
            20                  25                  30

Val Tyr Ile Thr Asp Lys Thr Val Leu Asp Met Arg Ser Met Asp Phe
        35                  40                  45

Lys Ser Asn Ser Ala Val Ala Trp Ser Asn Lys Ser Asp Phe Ala Cys
    50                  55                  60

Ala Asn Ala Phe Asn Asn Ser Ile Ile Pro Glu Asp Thr Phe Phe Pro
65                  70                  75                  80

Ser Pro Glu Ser Ser Cys Asp Val Lys Leu Val Glu Lys Ser Phe Glu
                85                  90                  95

Thr Asp Thr Asn Leu Asn Phe Gln Asn Leu Ser Val Ile Gly Phe Arg
            100                 105                 110

Ile Leu Leu Leu Lys Val Ala Gly Phe Asn Leu Leu Met Thr Leu Arg
        115                 120                 125

Leu Trp Ser Ser
    130


<210>  3
<211>  121
<212>  PRT
<213>  Homo sapiens

<400>  3

Ser Val Cys Leu Phe Thr Asp Phe Asp Ser Gln Thr Asn Val Ser Gln
1               5                   10                  15

Ser Lys Asp Ser Asp Val Tyr Ile Thr Asp Lys Thr Val Leu Asp Met
            20                  25                  30

Arg Ser Met Asp Phe Lys Ser Asn Ser Ala Val Ala Trp Ser Asn Lys
        35                  40                  45

Ser Asp Phe Ala Cys Ala Asn Ala Phe Asn Asn Ser Ile Ile Pro Glu
    50                  55                  60

Asp Thr Phe Phe Pro Ser Pro Glu Ser Ser Cys Asp Val Lys Leu Val

65                  70                  75                  80

Glu Lys Ser Phe Glu Thr Asp Thr Asn Leu Asn Phe Gln Asn Leu Ser
                85                  90                  95

Val Ile Gly Phe Arg Ile Leu Leu Leu Lys Val Ala Gly Phe Asn Leu
            100                 105                 110

Leu Met Thr Leu Arg Leu Trp Ser Ser
        115                 120

<210>  4
<211>  109
<212>  PRT
<213>  Homo sapiens

<400>  4

Asn Val Ser Gln Ser Lys Asp Ser Asp Val Tyr Ile Thr Asp Lys Thr
1               5                   10                  15

Val Leu Asp Met Arg Ser Met Asp Phe Lys Ser Asn Ser Ala Val Ala
            20                  25                  30

Trp Ser Asn Lys Ser Asp Phe Ala Cys Ala Asn Ala Phe Asn Asn Ser
        35                  40                  45

Ile Ile Pro Glu Asp Thr Phe Phe Pro Ser Pro Glu Ser Ser Cys Asp
    50                  55                  60

Val Lys Leu Val Glu Lys Ser Phe Glu Thr Asp Thr Asn Leu Asn Phe
65                  70                  75                  80

Gln Asn Leu Ser Val Ile Gly Phe Arg Ile Leu Leu Leu Lys Val Ala
                85                  90                  95

Gly Phe Asn Leu Leu Met Thr Leu Arg Leu Trp Ser Ser
            100                 105

<210>  5
<211>  101
<212>  PRT
<213>  Homo sapiens

<400>  5

Asp Val Tyr Ile Thr Asp Lys Thr Val Leu Asp Met Arg Ser Met Asp
1               5                   10                  15

Phe Lys Ser Asn Ser Ala Val Ala Trp Ser Asn Lys Ser Asp Phe Ala
            20                  25                  30

Cys Ala Asn Ala Phe Asn Asn Ser Ile Ile Pro Glu Asp Thr Phe Phe
        35                  40                  45

Pro Ser Pro Glu Ser Ser Cys Asp Val Lys Leu Val Glu Lys Ser Phe
    50                  55                  60

Glu Thr Asp Thr Asn Leu Asn Phe Gln Asn Leu Ser Val Ile Gly Phe
65                  70                  75                  80

Arg Ile Leu Leu Leu Lys Val Ala Gly Phe Asn Leu Leu Met Thr Leu
                85                  90                  95

Arg Leu Trp Ser Ser
            100

<210>  6
<211>  92
<212>  PRT
<213>  Homo sapiens

<400>  6

Leu Asp Met Arg Ser Met Asp Phe Lys Ser Asn Ser Ala Val Ala Trp
1               5                   10                  15

Ser Asn Lys Ser Asp Phe Ala Cys Ala Asn Ala Phe Asn Asn Ser Ile
            20                  25                  30

Ile Pro Glu Asp Thr Phe Phe Pro Ser Pro Glu Ser Ser Cys Asp Val
        35                  40                  45

Lys Leu Val Glu Lys Ser Phe Glu Thr Asp Thr Asn Leu Asn Phe Gln
    50                  55                  60

Asn Leu Ser Val Ile Gly Phe Arg Ile Leu Leu Leu Lys Val Ala Gly
65                  70                  75                  80

Phe Asn Leu Leu Met Thr Leu Arg Leu Trp Ser Ser
                85                  90

<210>  7
<211>  84
<212>  PRT
<213>  Homo sapiens

<400>  7

Lys Ser Asn Ser Ala Val Ala Trp Ser Asn Lys Ser Asp Phe Ala Cys
1               5                   10                  15

Ala Asn Ala Phe Asn Asn Ser Ile Ile Pro Glu Asp Thr Phe Phe Pro
            20                  25                  30

Ser Pro Glu Ser Ser Cys Asp Val Lys Leu Val Glu Lys Ser Phe Glu
        35                  40                  45

Thr Asp Thr Asn Leu Asn Phe Gln Asn Leu Ser Val Ile Gly Phe Arg
    50                  55                  60

Ile Leu Leu Leu Lys Val Ala Gly Phe Asn Leu Leu Met Thr Leu Arg
65                  70                  75                  80

Leu Trp Ser Ser

<210>  8
<211>  76
<212>  PRT
<213>  Homo sapiens

<400>  8

Ser Asn Lys Ser Asp Phe Ala Cys Ala Asn Ala Phe Asn Asn Ser Ile
1               5                   10                  15

Ile Pro Glu Asp Thr Phe Phe Pro Ser Pro Glu Ser Ser Cys Asp Val
            20                  25                  30

Lys Leu Val Glu Lys Ser Phe Glu Thr Asp Thr Asn Leu Asn Phe Gln
        35                  40                  45

Asn Leu Ser Val Ile Gly Phe Arg Ile Leu Leu Leu Lys Val Ala Gly
    50                  55                  60

Phe Asn Leu Leu Met Thr Leu Arg Leu Trp Ser Ser
65                  70                  75

<210>  9
<211>  69
<212>  PRT
<213>  Homo sapiens

<400>  9

Cys Ala Asn Ala Phe Asn Asn Ser Ile Ile Pro Glu Asp Thr Phe Phe
1               5                   10                  15

Pro Ser Pro Glu Ser Ser Cys Asp Val Lys Leu Val Glu Lys Ser Phe
            20                  25                  30

Glu Thr Asp Thr Asn Leu Asn Phe Gln Asn Leu Ser Val Ile Gly Phe
        35                  40                  45

Arg Ile Leu Leu Leu Lys Val Ala Gly Phe Asn Leu Leu Met Thr Leu
    50                  55                  60

Arg Leu Trp Ser Ser
65

<210>  10
<211>  65
<212>  PRT
<213>  Homo sapiens

<400>  10

Phe Asn Asn Ser Ile Ile Pro Glu Asp Thr Phe Phe Pro Ser Pro Glu
1               5                   10                  15

Ser Ser Cys Asp Val Lys Leu Val Glu Lys Ser Phe Glu Thr Asp Thr
            20                  25                  30

Asn Leu Asn Phe Gln Asn Leu Ser Val Ile Gly Phe Arg Ile Leu Leu
        35                  40                  45

Leu Lys Val Ala Gly Phe Asn Leu Leu Met Thr Leu Arg Leu Trp Ser
    50                  55                  60

Ser
65

<210>  11
<211>  60
<212>  PRT
<213>  Homo sapiens

<400>  11

Ile Pro Glu Asp Thr Phe Phe Pro Ser Pro Glu Ser Ser Cys Asp Val
1               5                   10                  15

Lys Leu Val Glu Lys Ser Phe Glu Thr Asp Thr Asn Leu Asn Phe Gln
            20                  25                  30

Asn Leu Ser Val Ile Gly Phe Arg Ile Leu Leu Leu Lys Val Ala Gly
        35                  40                  45

Phe Asn Leu Leu Met Thr Leu Arg Leu Trp Ser Ser
    50                  55                  60

<210>  12

<211>  179
<212>  PRT
<213>  Homo sapiens

<400>  12

Glu Asp Leu Lys Asn Val Phe Pro Pro Glu Val Ala Val Phe Glu Pro
1               5                   10                  15

Ser Glu Ala Glu Ile Ser His Thr Gln Lys Ala Thr Leu Val Cys Leu
            20                  25                  30

Ala Thr Gly Phe Tyr Pro Asp His Val Glu Leu Ser Trp Trp Val Asn
        35                  40                  45

Gly Lys Glu Val His Ser Gly Val Ser Thr Asp Pro Gln Pro Leu Lys
    50                  55                  60

Glu Gln Pro Ala Leu Asn Asp Ser Arg Tyr Cys Leu Ser Ser Arg Leu
65                  70                  75                  80

Arg Val Ser Ala Thr Phe Trp Gln Asn Pro Arg Asn His Phe Arg Cys
                85                  90                  95

Gln Val Gln Phe Tyr Gly Leu Ser Glu Asn Asp Glu Trp Thr Gln Asp
            100                 105                 110

Arg Ala Lys Pro Val Thr Gln Ile Val Ser Ala Glu Ala Trp Gly Arg
        115                 120                 125

Ala Asp Cys Gly Phe Thr Ser Glu Ser Tyr Gln Gln Gly Val Leu Ser
    130                 135                 140

Ala Thr Ile Leu Tyr Glu Ile Leu Leu Gly Lys Ala Thr Leu Tyr Ala
145                 150                 155                 160

Val Leu Val Ser Ala Leu Val Leu Met Ala Met Val Lys Arg Lys Asp
                165                 170                 175

Ser Arg Gly



<210>  13
<211>  163
<212>  PRT
<213>  Homo sapiens

<400>  13

Ser Glu Ala Glu Ile Ser His Thr Gln Lys Ala Thr Leu Val Cys Leu
1               5                   10                  15

Ala Thr Gly Phe Tyr Pro Asp His Val Glu Leu Ser Trp Trp Val Asn
            20                  25                  30

Gly Lys Glu Val His Ser Gly Val Ser Thr Asp Pro Gln Pro Leu Lys
        35                  40                  45

Glu Gln Pro Ala Leu Asn Asp Ser Arg Tyr Cys Leu Ser Ser Arg Leu
    50                  55                  60

Arg Val Ser Ala Thr Phe Trp Gln Asn Pro Arg Asn His Phe Arg Cys
65                  70                  75                  80

Gln Val Gln Phe Tyr Gly Leu Ser Glu Asn Asp Glu Trp Thr Gln Asp
                85                  90                  95

Arg Ala Lys Pro Val Thr Gln Ile Val Ser Ala Glu Ala Trp Gly Arg
            100                 105                 110

Ala Asp Cys Gly Phe Thr Ser Glu Ser Tyr Gln Gln Gly Val Leu Ser
        115                 120                 125

Ala Thr Ile Leu Tyr Glu Ile Leu Leu Gly Lys Ala Thr Leu Tyr Ala
    130                 135                 140

Val Leu Val Ser Ala Leu Val Leu Met Ala Met Val Lys Arg Lys Asp
145                 150                 155                 160

Ser Arg Gly

<210> 14
<211> 153
<212> PRT
<213> Homo sapiens

<400> 14

Ala Thr Leu Val Cys Leu Ala Thr Gly Phe Tyr Pro Asp His Val Glu
1               5                   10                  15

Leu Ser Trp Trp Val Asn Gly Lys Glu Val His Ser Gly Val Ser Thr
            20                  25                  30

Asp Pro Gln Pro Leu Lys Glu Gln Pro Ala Leu Asn Asp Ser Arg Tyr
        35                  40                  45

Cys Leu Ser Ser Arg Leu Arg Val Ser Ala Thr Phe Trp Gln Asn Pro
    50                  55                  60

Arg Asn His Phe Arg Cys Gln Val Gln Phe Tyr Gly Leu Ser Glu Asn
65                  70                  75                  80

Asp Glu Trp Thr Gln Asp Arg Ala Lys Pro Val Thr Gln Ile Val Ser
                85                  90                  95

Ala Glu Ala Trp Gly Arg Ala Asp Cys Gly Phe Thr Ser Glu Ser Tyr
            100                 105                 110

Gln Gln Gly Val Leu Ser Ala Thr Ile Leu Tyr Glu Ile Leu Leu Gly
        115                 120                 125

Lys Ala Thr Leu Tyr Ala Val Leu Val Ser Ala Leu Val Leu Met Ala
    130                 135                 140

Met Val Lys Arg Lys Asp Ser Arg Gly
145                 150

<210>  15
<211>  139
<212>  PRT
<213>  Homo sapiens

<400>  15

Val Glu Leu Ser Trp Trp Val Asn Gly Lys Glu Val His Ser Gly Val
1               5                   10                  15

Ser Thr Asp Pro Gln Pro Leu Lys Glu Gln Pro Ala Leu Asn Asp Ser
            20                  25                  30

Arg Tyr Cys Leu Ser Ser Arg Leu Arg Val Ser Ala Thr Phe Trp Gln
        35                  40                  45

Asn Pro Arg Asn His Phe Arg Cys Gln Val Gln Phe Tyr Gly Leu Ser
    50                  55                  60

Glu Asn Asp Glu Trp Thr Gln Asp Arg Ala Lys Pro Val Thr Gln Ile
65                  70                  75                  80

Val Ser Ala Glu Ala Trp Gly Arg Ala Asp Cys Gly Phe Thr Ser Glu
                85                  90                  95

Ser Tyr Gln Gln Gly Val Leu Ser Ala Thr Ile Leu Tyr Glu Ile Leu
            100                 105                 110

Leu Gly Lys Ala Thr Leu Tyr Ala Val Leu Val Ser Ala Leu Val Leu
        115                 120                 125

Met Ala Met Val Lys Arg Lys Asp Ser Arg Gly
    130                 135

<210>  16
<211>  133
<212>  PRT
<213>  Homo sapiens

<400>  16

Val Asn Gly Lys Glu Val His Ser Gly Val Ser Thr Asp Pro Gln Pro
1               5                   10                  15

Leu Lys Glu Gln Pro Ala Leu Asn Asp Ser Arg Tyr Cys Leu Ser Ser
            20                  25                  30

Arg Leu Arg Val Ser Ala Thr Phe Trp Gln Asn Pro Arg Asn His Phe
        35                  40                  45

Arg Cys Gln Val Gln Phe Tyr Gly Leu Ser Glu Asn Asp Glu Trp Thr
    50                  55                  60

Gln Asp Arg Ala Lys Pro Val Thr Gln Ile Val Ser Ala Glu Ala Trp
65                  70                  75                  80

Gly Arg Ala Asp Cys Gly Phe Thr Ser Glu Ser Tyr Gln Gln Gly Val
                85                  90                  95

Leu Ser Ala Thr Ile Leu Tyr Glu Ile Leu Leu Gly Lys Ala Thr Leu
            100                 105                 110

Tyr Ala Val Leu Val Ser Ala Leu Val Leu Met Ala Met Val Lys Arg
        115                 120                 125

Lys Asp Ser Arg Gly
    130

<210>  17
<211>  124
<212>  PRT
<213>  Homo sapiens

<400>  17

Val Ser Thr Asp Pro Gln Pro Leu Lys Glu Gln Pro Ala Leu Asn Asp
1               5                   10                  15

Ser Arg Tyr Cys Leu Ser Ser Arg Leu Arg Val Ser Ala Thr Phe Trp
            20                  25                  30

Gln Asn Pro Arg Asn His Phe Arg Cys Gln Val Gln Phe Tyr Gly Leu
        35                  40                  45

Ser Glu Asn Asp Glu Trp Thr Gln Asp Arg Ala Lys Pro Val Thr Gln
    50                  55                  60

Ile Val Ser Ala Glu Ala Trp Gly Arg Ala Asp Cys Gly Phe Thr Ser
65                  70                  75                  80

Glu Ser Tyr Gln Gln Gly Val Leu Ser Ala Thr Ile Leu Tyr Glu Ile
                85                  90                  95

Leu Leu Gly Lys Ala Thr Leu Tyr Ala Val Leu Val Ser Ala Leu Val
            100                 105                 110

Leu Met Ala Met Val Lys Arg Lys Asp Ser Arg Gly
        115                 120

<210>  18
<211>  112
<212>  PRT
<213>  Homo sapiens

<400>  18

Ala Leu Asn Asp Ser Arg Tyr Cys Leu Ser Ser Arg Leu Arg Val Ser
1               5                   10                  15

Ala Thr Phe Trp Gln Asn Pro Arg Asn His Phe Arg Cys Gln Val Gln
            20                  25                  30

Phe Tyr Gly Leu Ser Glu Asn Asp Glu Trp Thr Gln Asp Arg Ala Lys
        35                  40                  45

Pro Val Thr Gln Ile Val Ser Ala Glu Ala Trp Gly Arg Ala Asp Cys
    50                  55                  60

Gly Phe Thr Ser Glu Ser Tyr Gln Gln Gly Val Leu Ser Ala Thr Ile
65                  70                  75                  80

Leu Tyr Glu Ile Leu Leu Gly Lys Ala Thr Leu Tyr Ala Val Leu Val
                85                  90                  95

Ser Ala Leu Val Leu Met Ala Met Val Lys Arg Lys Asp Ser Arg Gly
            100                 105                 110

<210>  19
<211>  101
<212>  PRT

<213>  Homo sapiens

<400>  19

Arg Leu Arg Val Ser Ala Thr Phe Trp Gln Asn Pro Arg Asn His Phe
1               5                   10                  15

Arg Cys Gln Val Gln Phe Tyr Gly Leu Ser Glu Asn Asp Glu Trp Thr
            20                  25                  30

Gln Asp Arg Ala Lys Pro Val Thr Gln Ile Val Ser Ala Glu Ala Trp
        35                  40                  45

Gly Arg Ala Asp Cys Gly Phe Thr Ser Glu Ser Tyr Gln Gln Gly Val
    50                  55                  60

Leu Ser Ala Thr Ile Leu Tyr Glu Ile Leu Leu Gly Lys Ala Thr Leu
65                  70                  75                  80

Tyr Ala Val Leu Val Ser Ala Leu Val Leu Met Ala Met Val Lys Arg
                85                  90                  95

Lys Asp Ser Arg Gly
            100

<210>  20
<211>  92
<212>  PRT
<213>  Homo sapiens

<400>  20

Gln Asn Pro Arg Asn His Phe Arg Cys Gln Val Gln Phe Tyr Gly Leu
1               5                   10                  15

Ser Glu Asn Asp Glu Trp Thr Gln Asp Arg Ala Lys Pro Val Thr Gln
            20                  25                  30

Ile Val Ser Ala Glu Ala Trp Gly Arg Ala Asp Cys Gly Phe Thr Ser
        35                  40                  45

Glu Ser Tyr Gln Gln Gly Val Leu Ser Ala Thr Ile Leu Tyr Glu Ile
    50                  55                  60

Leu Leu Gly Lys Ala Thr Leu Tyr Ala Val Leu Val Ser Ala Leu Val
65                  70                  75                  80

Leu Met Ala Met Val Lys Arg Lys Asp Ser Arg Gly
                85                  90

<210>  21
<211>  86
<212>  PRT
<213>  Homo sapiens

<400>  21

Phe Arg Cys Gln Val Gln Phe Tyr Gly Leu Ser Glu Asn Asp Glu Trp
1               5                   10                  15

Thr Gln Asp Arg Ala Lys Pro Val Thr Gln Ile Val Ser Ala Glu Ala
            20                  25                  30

Trp Gly Arg Ala Asp Cys Gly Phe Thr Ser Glu Ser Tyr Gln Gln Gly
        35                  40                  45

Val Leu Ser Ala Thr Ile Leu Tyr Glu Ile Leu Leu Gly Lys Ala Thr
    50                  55                  60

Leu Tyr Ala Val Leu Val Ser Ala Leu Val Leu Met Ala Met Val Lys
65                  70                  75                  80

Arg Lys Asp Ser Arg Gly
                85


<210>  22
<211>  79
<212>  PRT
<213>  Homo sapiens

<400>  22

Tyr Gly Leu Ser Glu Asn Asp Glu Trp Thr Gln Asp Arg Ala Lys Pro
1               5                   10                  15

Val Thr Gln Ile Val Ser Ala Glu Ala Trp Gly Arg Ala Asp Cys Gly
            20                  25                  30

Phe Thr Ser Glu Ser Tyr Gln Gln Gly Val Leu Ser Ala Thr Ile Leu
        35                  40                  45

Tyr Glu Ile Leu Leu Gly Lys Ala Thr Leu Tyr Ala Val Leu Val Ser
    50                  55                  60

Ala Leu Val Leu Met Ala Met Val Lys Arg Lys Asp Ser Arg Gly
65                  70                  75


<210>  23
<211>  72
<212>  PRT
<213>  Homo sapiens

<400>  23

Glu Trp Thr Gln Asp Arg Ala Lys Pro Val Thr Gln Ile Val Ser Ala
1               5                   10                  15

Glu Ala Trp Gly Arg Ala Asp Cys Gly Phe Thr Ser Glu Ser Tyr Gln
            20                  25                  30

Gln Gly Val Leu Ser Ala Thr Ile Leu Tyr Glu Ile Leu Leu Gly Lys
        35                  40                  45

Ala Thr Leu Tyr Ala Val Leu Val Ser Ala Leu Val Leu Met Ala Met
    50                  55                  60

Val Lys Arg Lys Asp Ser Arg Gly
65                  70

<210>  24
<211>  66
<212>  PRT
<213>  Homo sapiens

<400>  24

Ala Lys Pro Val Thr Gln Ile Val Ser Ala Glu Ala Trp Gly Arg Ala
1               5                   10                  15

Asp Cys Gly Phe Thr Ser Glu Ser Tyr Gln Gln Gly Val Leu Ser Ala
            20                  25                  30

Thr Ile Leu Tyr Glu Ile Leu Leu Gly Lys Ala Thr Leu Tyr Ala Val
        35                  40                  45

Leu Val Ser Ala Leu Val Leu Met Ala Met Val Lys Arg Lys Asp Ser
    50                  55                  60

Arg Gly
65

<210>  25
<211>  423
<212>  DNA
<213>  Homo sapiens

<400>  25
aatatccaga accctgaccc tgccgtgtac cagctgagag actctaaatc cagtgacaag      60

tctgtctgcc tattcaccga ttttgattct caaacaaatg tgtcacaaag taaggattct     120

gatgtgtata tcacagacaa aactgtgcta gacatgaggt ctatggactt caagagcaac     180

agtgctgtgg cctggagcaa caaatctgac tttgcatgtg caaacgcctt caacaacagc     240

attattccag aagacacctt cttccccagc ccagaaagtt cctgtgatgt caagctggtc 300

gagaaaagct ttgaaacaga tacgaaccta aactttcaaa acctgtcagt gattgggttc 360

cgaatcctcc tcctgaaagt ggccgggttt aatctgctca tgacgctgcg gctgtggtcc 420

agc 423

<210> 26
<211> 396
<212> DNA
<213> Homo sapiens

<400> 26
taccagctga gagactctaa atccagtgac aagtctgtct gcctattcac cgattttgat 60

tctcaaacaa atgtgtcaca aagtaaggat tctgatgtgt atatcacaga caaaactgtg 120

ctagacatga ggtctatgga cttcaagagc aacagtgctg tggcctggag caacaaatct 180

gactttgcat gtgcaaacgc cttcaacaac agcattattc cagaagacac cttcttcccc 240

agcccagaaa gttcctgtga tgtcaagctg gtcgagaaaa gctttgaaac agatacgaac 300

ctaaactttc aaaacctgtc agtgattggg ttccgaatcc tcctcctgaa agtggccggg 360

tttaatctgc tcatgacgct gcggctgtgg tccagc 396

<210> 27
<211> 363
<212> DNA
<213> Homo sapiens

<400> 27
tctgtctgcc tattcaccga ttttgattct caaacaaatg tgtcacaaag taaggattct 60

gatgtgtata tcacagacaa aactgtgcta gacatgaggt ctatggactt caagagcaac 120

agtgctgtgg cctggagcaa caaatctgac tttgcatgtg caaacgcctt caacaacagc 180

attattccag aagacacctt cttccccagc ccagaaagtt cctgtgatgt caagctggtc 240

gagaaaagct ttgaaacaga tacgaaccta aactttcaaa acctgtcagt gattgggttc 300

cgaatcctcc tcctgaaagt ggccgggttt aatctgctca tgacgctgcg gctgtggtcc 360

agc 363

<210> 28
<211> 327
<212> DNA
<213> Homo sapiens

<400> 28
aatgtgtcac aaagtaagga ttctgatgtg tatatcacag acaaaactgt gctagacatg 60

aggtctatgg acttcaagag caacagtgct gtggcctgga gcaacaaatc tgactttgca 120

tgtgcaaacg ccttcaacaa cagcattatt ccagaagaca ccttcttccc cagcccagaa 180

agttcctgtg atgtcaagct ggtcgagaaa agctttgaaa cagatacgaa cctaaacttt 240

caaaacctgt cagtgattgg gttccgaatc ctcctcctga aagtggccgg gtttaatctg 300

ctcatgacgc tgcggctgtg gtccagc 327

<210> 29
<211> 303
<212> DNA
<213> Homo sapiens

<400> 29
gatgtgtata tcacagacaa aactgtgcta gacatgaggt ctatggactt caagagcaac 60

agtgctgtgg cctggagcaa caaatctgac tttgcatgtg caaacgcctt caacaacagc 120

attattccag aagacacctt cttccccagc ccagaaagtt cctgtgatgt caagctggtc 180

gagaaaagct ttgaaacaga tacgaaccta aactttcaaa acctgtcagt gattgggttc 240

cgaatcctcc tcctgaaagt ggccgggttt aatctgctca tgacgctgcg gctgtggtcc 300

agc 303

<210> 30
<211> 276
<212> DNA
<213> Homo sapiens

<400> 30
ctagacatga ggtctatgga cttcaagagc aacagtgctg tggcctggag caacaaatct 60

gactttgcat gtgcaaacgc cttcaacaac agcattattc cagaagacac cttcttcccc 120

agcccagaaa gttcctgtga tgtcaagctg gtcgagaaaa gctttgaaac agatacgaac 180

ctaaactttc aaaacctgtc agtgattggg ttccgaatcc tcctcctgaa agtggccggg 240

tttaatctgc tcatgacgct gcggctgtgg tccagc 276

<210> 31
<211> 252
<212> DNA
<213> Homo sapiens

<400> 31
aagagcaaca gtgctgtggc ctggagcaac aaatctgact ttgcatgtgc aaacgccttc 60

aacaacagca ttattccaga agacaccttc ttccccagcc cagaaagttc ctgtgatgtc 120

aagctggtcg agaaaagctt tgaaacagat acgaacctaa actttcaaaa cctgtcagtg 180

attgggttcc gaatcctcct cctgaaagtg gccgggttta atctgctcat gacgctgcgg 240

ctgtggtcca gc 252

<210> 32
<211> 228
<212> DNA

<213>  Homo sapiens

<400>  32
agcaacaaat ctgactttgc atgtgcaaac gccttcaaca acagcattat tccagaagac      60

accttcttcc ccagcccaga aagttcctgt gatgtcaagc tggtcgagaa aagctttgaa     120

acagatacga acctaaactt tcaaaacctg tcagtgattg ggttccgaat cctcctcctg     180

aaagtggccg ggtttaatct gctcatgacg ctgcggctgt ggtccagc                  228


<210>  33
<211>  207
<212>  DNA
<213>  Homo sapiens

<400>  33
tgtgcaaacg ccttcaacaa cagcattatt ccagaagaca ccttcttccc cagcccagaa      60

agttcctgtg atgtcaagct ggtcgagaaa agctttgaaa cagatacgaa cctaaacttt     120

caaaacctgt cagtgattgg gttccgaatc ctcctcctga aagtggccgg gtttaatctg     180

ctcatgacgc tgcggctgtg gtccagc                                         207


<210>  34
<211>  195
<212>  DNA
<213>  Homo sapiens

<400>  34
ttcaacaaca gcattattcc agaagacacc ttcttcccca gcccagaaag ttcctgtgat      60

gtcaagctgg tcgagaaaag ctttgaaaca gatacgaacc taaactttca aaacctgtca     120

gtgattgggt tccgaatcct cctcctgaaa gtggccgggt ttaatctgct catgacgctg     180

cggctgtggt ccagc                                                      195


<210>  35
<211>  180
<212>  DNA
<213>  Homo sapiens

<400>  35
attccagaag acaccttctt ccccagccca gaaagttcct gtgatgtcaa gctggtcgag      60

aaaagctttg aaacagatac gaacctaaac tttcaaaacc tgtcagtgat tgggttccga     120

atcctcctcc tgaaagtggc cgggtttaat ctgctcatga cgctgcggct gtggtccagc     180


<210>  36
<211>  537
<212>  DNA
<213>  Homo sapiens

<400>  36
gaggacctga aaaacgtgtt cccacccgag gtcgctgtgt ttgagccatc agaagcagag      60

atctcccaca cccaaaaggc cacactggtg tgcctggcca caggcttcta ccccgaccac 120

gtggagctga gctggtgggt gaatgggaag gaggtgcaca gtggggtcag cacagacccg 180

cagcccctca aggagcagcc cgccctcaat gactccagat actgcctgag cagccgcctg 240

agggtctcgg ccaccttctg gcagaacccc cgcaaccact tccgctgtca agtccagttc 300

tacgggctct cggagaatga cgagtggacc caggataggg ccaaacctgt cacccagatc 360

gtcagcgccg aggcctgggg tagagcagac tgtggcttca cctccgagtc ttaccagcaa 420

ggggtcctgt ctgccaccat cctctatgag atcttgctag ggaaggccac cttgtatgcc 480

gtgctggtca gtgccctcgt gctgatggcc atggtcaaga gaaaggattc cagaggc 537

<210> 37
<211> 489
<212> DNA
<213> Homo sapiens

<400> 37
tcagaagcag agatctccca cacccaaaag gccacactgg tgtgcctggc cacaggcttc 60

taccccgacc acgtggagct gagctggtgg gtgaatggga aggaggtgca cagtggggtc 120

agcacagacc cgcagcccct caaggagcag cccgccctca atgactccag atactgcctg 180

agcagccgcc tgagggtctc ggccaccttc tggcagaacc cccgcaacca cttccgctgt 240

caagtccagt tctacgggct ctcggagaat gacgagtgga cccaggatag ggccaaacct 300

gtcacccaga tcgtcagcgc cgaggcctgg ggtagagcag actgtggctt cacctccgag 360

tcttaccagc aaggggtcct gtctgccacc atcctctatg agatcttgct agggaaggcc 420

accttgtatg ccgtgctggt cagtgccctc gtgctgatgg ccatggtcaa gagaaaggat 480

tccagaggc 489

<210> 38
<211> 459
<212> DNA
<213> Homo sapiens

<400> 38
gccacactgg tgtgcctggc cacaggcttc taccccgacc acgtggagct gagctggtgg 60

gtgaatggga aggaggtgca cagtggggtc agcacagacc cgcagcccct caaggagcag 120

cccgccctca atgactccag atactgcctg agcagccgcc tgagggtctc ggccaccttc 180

tggcagaacc cccgcaacca cttccgctgt caagtccagt tctacgggct ctcggagaat 240

gacgagtgga cccaggatag ggccaaacct gtcacccaga tcgtcagcgc cgaggcctgg 300

ggtagagcag actgtggctt cacctccgag tcttaccagc aaggggtcct gtctgccacc 360

atcctctatg agatcttgct agggaaggcc accttgtatg ccgtgctggt cagtgccctc 420

gtgctgatgg ccatggtcaa gagaaaggat tccagaggc 459

<210>  39
<211>  417
<212>  DNA
<213>  Homo sapiens

<400>  39
gtggagctga gctggtgggt gaatgggaag gaggtgcaca gtggggtcag cacagacccg      60

cagcccctca aggagcagcc cgccctcaat gactccagat actgcctgag cagccgcctg     120

agggtctcgg ccaccttctg gcagaacccc cgcaaccact tccgctgtca agtccagttc     180

tacgggctct cggagaatga cgagtggacc caggataggg ccaaacctgt cacccagatc     240

gtcagcgccg aggcctgggg tagagcagac tgtggcttca cctccgagtc ttaccagcaa     300

ggggtcctgt ctgccaccat cctctatgag atcttgctag ggaaggccac cttgtatgcc     360

gtgctggtca gtgccctcgt gctgatggcc atggtcaaga gaaaggattc cagaggc        417


<210>  40
<211>  399
<212>  DNA
<213>  Homo sapiens

<400>  40
gtgaatggga aggaggtgca cagtggggtc agcacagacc cgcagcccct caaggagcag      60

cccgccctca atgactccag atactgcctg agcagccgcc tgagggtctc ggccaccttc     120

tggcagaacc cccgcaacca cttccgctgt caagtccagt tctacgggct ctcggagaat     180

gacgagtgga cccaggatag ggccaaacct gtcacccaga tcgtcagcgc cgaggcctgg     240

ggtagagcag actgtggctt cacctccgag tcttaccagc aaggggtcct gtctgccacc     300

atcctctatg agatcttgct agggaaggcc accttgtatg ccgtgctggt cagtgccctc     360

gtgctgatgg ccatggtcaa gagaaaggat tccagaggc                            399


<210>  41
<211>  372
<212>  DNA
<213>  Homo sapiens

<400>  41
gtcagcacag acccgcagcc cctcaaggag cagcccgccc tcaatgactc cagatactgc      60

ctgagcagcc gcctgagggt ctcggccacc ttctggcaga acccccgcaa ccacttccgc     120

tgtcaagtcc agttctacgg gctctcggag aatgacgagt ggacccagga tagggccaaa     180

cctgtcaccc agatcgtcag cgccgaggcc tggggtagag cagactgtgg cttcacctcc     240

gagtcttacc agcaaggggt cctgtctgcc accatcctct atgagatctt gctagggaag     300

gccaccttgt atgccgtgct ggtcagtgcc ctcgtgctga tggccatggt caagagaaag     360

gattccagag gc                                                         372

<210>  42
<211>  336
<212>  DNA
<213>  Homo sapiens

<400>  42
gccctcaatg actccagata ctgcctgagc agccgcctga gggtctcggc caccttctgg      60

cagaaccccc gcaaccactt ccgctgtcaa gtccagttct acgggctctc ggagaatgac     120

gagtggaccc aggatagggc caaacctgtc acccagatcg tcagcgccga ggcctggggt     180

agagcagact gtggcttcac ctccgagtct taccagcaag gggtcctgtc tgccaccatc     240

ctctatgaga tcttgctagg gaaggccacc ttgtatgccg tgctggtcag tgccctcgtg     300

ctgatggcca tggtcaagag aaaggattcc agaggc                               336


<210>  43
<211>  303
<212>  DNA
<213>  Homo sapiens

<400>  43
cgcctgaggg tctcggccac cttctggcag aacccccgca accacttccg ctgtcaagtc      60

cagttctacg ggctctcgga gaatgacgag tggacccagg atagggccaa acctgtcacc     120

cagatcgtca gcgccgaggc ctggggtaga gcagactgtg gcttcacctc cgagtcttac     180

cagcaagggg tcctgtctgc caccatcctc tatgagatct tgctagggaa ggccaccttg     240

tatgccgtgc tggtcagtgc cctcgtgctg atggccatgg tcaagagaaa ggattccaga     300

ggc                                                                   303


<210>  44
<211>  276
<212>  DNA
<213>  Homo sapiens

<400>  44
cagaaccccc gcaaccactt ccgctgtcaa gtccagttct acgggctctc ggagaatgac      60

gagtggaccc aggatagggc caaacctgtc acccagatcg tcagcgccga ggcctggggt     120

agagcagact gtggcttcac ctccgagtct taccagcaag gggtcctgtc tgccaccatc     180

ctctatgaga tcttgctagg gaaggccacc ttgtatgccg tgctggtcag tgccctcgtg     240

ctgatggcca tggtcaagag aaaggattcc agaggc                               276


<210>  45
<211>  258
<212>  DNA
<213>  Homo sapiens

<400>  45

ttccgctgtc aagtccagtt ctacgggctc tcggagaatg acgagtggac ccaggatagg 60

gccaaacctg tcacccagat cgtcagcgcc gaggcctggg gtagagcaga ctgtggcttc 120

acctccgagt cttaccagca aggggtcctg tctgccacca tcctctatga gatcttgcta 180

gggaaggcca ccttgtatgc cgtgctggtc agtgccctcg tgctgatggc catggtcaag 240

agaaaggatt ccagaggc 258

<210> 46
<211> 237
<212> DNA
<213> Homo sapiens

<400> 46
tacgggctct cggagaatga cgagtggacc caggataggg ccaaacctgt cacccagatc 60

gtcagcgccg aggcctgggg tagagcagac tgtggcttca cctccgagtc ttaccagcaa 120

ggggtcctgt ctgccaccat cctctatgag atcttgctag ggaaggccac cttgtatgcc 180

gtgctggtca gtgccctcgt gctgatggcc atggtcaaga gaaaggattc cagaggc 237

<210> 47
<211> 216
<212> DNA
<213> Homo sapiens

<400> 47
gagtggaccc aggatagggc caaacctgtc acccagatcg tcagcgccga ggcctggggt 60

agagcagact gtggcttcac ctccgagtct taccagcaag gggtcctgtc tgccaccatc 120

ctctatgaga tcttgctagg gaaggccacc ttgtatgccg tgctggtcag tgccctcgtg 180

ctgatggcca tggtcaagag aaaggattcc agaggc 216

<210> 48
<211> 198
<212> DNA
<213> Homo sapiens

<400> 48
gccaaacctg tcacccagat cgtcagcgcc gaggcctggg gtagagcaga ctgtggcttc 60

acctccgagt cttaccagca aggggtcctg tctgccacca tcctctatga gatcttgcta 120

gggaaggcca ccttgtatgc cgtgctggtc agtgccctcg tgctgatggc catggtcaag 180

agaaaggatt ccagaggc 198

<210> 49
<211> 606
<212> PRT
<213> Artificial Sequence

<220>
<223> Amino acid sequence of ba1

<400>  49

Met Leu Ser Leu Leu Leu Leu Leu Leu Gly Leu Gly Ser Val Phe Ser
1               5                   10                  15

Ala Val Ile Ser Gln Lys Pro Ser Arg Asp Ile Cys Gln Arg Gly Thr
            20                  25                  30

Ser Leu Thr Ile Gln Cys Gln Val Asp Ser Gln Val Thr Met Met Phe
        35                  40                  45

Trp Tyr Arg Gln Gln Pro Gly Gln Ser Leu Thr Leu Ile Ala Thr Ala
    50                  55                  60

Asn Gln Gly Ser Glu Ala Thr Tyr Glu Ser Gly Phe Val Ile Asp Lys
65                  70                  75                  80

Phe Pro Ile Ser Arg Pro Asn Leu Thr Phe Ser Thr Leu Thr Val Ser
                85                  90                  95

Asn Met Ser Pro Glu Asp Ser Ser Ile Tyr Leu Cys Ser Val Asp Gly
            100                 105                 110

Gln Gly Asn Thr Gly Glu Leu Phe Phe Gly Glu Gly Ser Arg Leu Thr
        115                 120                 125

Val Leu Glu Asp Leu Lys Asn Val Phe Pro Pro Glu Val Ala Val Phe
    130                 135                 140

Glu Pro Ser Glu Ala Glu Ile Ser His Thr Gln Lys Ala Thr Leu Val
145                 150                 155                 160

Cys Leu Ala Thr Gly Phe Tyr Pro Asp His Val Glu Leu Ser Trp Trp
                165                 170                 175

Val Asn Gly Lys Glu Val His Ser Gly Val Ser Thr Asp Pro Gln Pro
            180                 185                 190

Leu Lys Glu Gln Pro Ala Leu Asn Asp Ser Arg Tyr Cys Leu Ser Ser
        195                 200                 205

Arg Leu Arg Val Ser Ala Thr Phe Trp Gln Asn Pro Arg Asn His Phe
    210                 215                 220

Arg Cys Gln Val Gln Phe Tyr Gly Leu Ser Glu Asn Asp Glu Trp Thr
225                 230                 235                 240

Gln Asp Arg Ala Lys Pro Val Thr Gln Ile Val Ser Ala Glu Ala Trp
                245                 250                 255

Gly Arg Ala Asp Cys Gly Phe Thr Ser Glu Ser Tyr Gln Gln Gly Val
            260                 265                 270

Leu Ser Ala Thr Ile Leu Tyr Glu Ile Leu Leu Gly Lys Ala Thr Leu
        275                 280                 285

Tyr Ala Val Leu Val Ser Ala Leu Val Leu Met Ala Met Val Lys Arg
    290                 295                 300

Lys Asp Ser Arg Gly Ser Gly Ser Gly Glu Gly Arg Gly Ser Leu Leu
305                 310                 315                 320

Thr Cys Gly Asp Val Glu Glu Asn Pro Gly Pro Met Ala Cys Pro Gly
                325                 330                 335

Phe Leu Trp Ala Leu Val Ile Ser Thr Cys Leu Glu Phe Ser Met Ala
            340                 345                 350

Gln Thr Val Thr Gln Ser Gln Pro Glu Met Ser Val Gln Glu Ala Glu
        355                 360                 365

Thr Val Thr Leu Ser Cys Thr Tyr Asp Thr Ser Glu Ser Asp Tyr Tyr
    370                 375                 380

Leu Phe Trp Tyr Lys Gln Pro Pro Ser Arg Gln Met Ile Leu Val Ile
385                 390                 395                 400

Arg Gln Glu Ala Tyr Lys Gln Gln Asn Ala Thr Glu Asn Arg Phe Ser
                405                 410                 415

Val Asn Phe Gln Lys Ala Ala Lys Ser Phe Ser Leu Lys Ile Ser Asp
            420                 425                 430

Ser Gln Leu Gly Asp Ala Ala Met Tyr Phe Cys Ala Tyr Trp Ser Asn
        435                 440                 445

Asn Asn Ala Arg Leu Met Phe Gly Asp Gly Thr Gln Leu Val Val Lys
    450                 455                 460

Pro Asn Ile Gln Asn Pro Asp Pro Ala Val Tyr Gln Leu Arg Asp Ser
465                 470                 475                 480

Lys Ser Ser Asp Lys Ser Val Cys Leu Phe Thr Asp Phe Asp Ser Gln
                485                 490                 495

Thr Asn Val Ser Gln Ser Lys Asp Ser Asp Val Tyr Ile Thr Asp Lys
            500                 505                 510

Thr Val Leu Asp Met Arg Ser Met Asp Phe Lys Ser Asn Ser Ala Val
        515                 520                 525

Ala Trp Ser Asn Lys Ser Asp Phe Ala Cys Ala Asn Ala Phe Asn Asn
    530                 535                 540

Ser Ile Ile Pro Glu Asp Thr Phe Phe Pro Ser Pro Glu Ser Ser Cys
545                 550                 555                 560

Asp Val Lys Leu Val Glu Lys Ser Phe Glu Thr Asp Thr Asn Leu Asn
                565                 570                 575

Phe Gln Asn Leu Ser Val Ile Gly Phe Arg Ile Leu Leu Leu Lys Val
            580                 585                 590

Ala Gly Phe Asn Leu Leu Met Thr Leu Arg Leu Trp Ser Ser
        595                 600                 605

<210> 50
<211> 379
<212> PRT
<213> Artificial Sequence

<220>
<223> Amino acid sequence of ba2

<400> 50

Met Leu Ser Leu Leu Leu Leu Leu Leu Gly Leu Gly Ser Val Phe Ser
1               5                   10                  15

Glu Asp Leu Lys Asn Val Phe Pro Pro Glu Val Ala Val Phe Glu Pro
            20                  25                  30

Ser Glu Ala Glu Ile Ser His Thr Gln Lys Ala Thr Leu Val Cys Leu
        35                  40                  45

Ala Thr Gly Phe Tyr Pro Asp His Val Glu Leu Ser Trp Trp Val Asn
    50                  55                  60

Gly Lys Glu Val His Ser Gly Val Ser Thr Asp Pro Gln Pro Leu Lys
65                  70                  75                  80

Glu Gln Pro Ala Leu Asn Asp Ser Arg Tyr Cys Leu Ser Ser Arg Leu
                85                  90                  95

Arg Val Ser Ala Thr Phe Trp Gln Asn Pro Arg Asn His Phe Arg Cys
        100                 105                 110

Gln Val Gln Phe Tyr Gly Leu Ser Glu Asn Asp Glu Trp Thr Gln Asp
    115                 120                 125

Arg Ala Lys Pro Val Thr Gln Ile Val Ser Ala Glu Ala Trp Gly Arg
130                 135                 140

Ala Asp Cys Gly Phe Thr Ser Glu Ser Tyr Gln Gln Gly Val Leu Ser
145                 150                 155                 160

Ala Thr Ile Leu Tyr Glu Ile Leu Leu Gly Lys Ala Thr Leu Tyr Ala
                165                 170                 175

Val Leu Val Ser Ala Leu Val Leu Met Ala Met Val Lys Arg Lys Asp
            180                 185                 190

Ser Arg Gly Ser Gly Ser Gly Glu Gly Arg Gly Ser Leu Leu Thr Cys
        195                 200                 205

Gly Asp Val Glu Glu Asn Pro Gly Pro Met Ala Cys Pro Gly Phe Leu
    210                 215                 220

Trp Ala Leu Val Ile Ser Thr Cys Leu Glu Phe Ser Met Ala Asn Ile
225                 230                 235                 240

Gln Asn Pro Asp Pro Ala Val Tyr Gln Leu Arg Asp Ser Lys Ser Ser
                245                 250                 255

Asp Lys Ser Val Cys Leu Phe Thr Asp Phe Asp Ser Gln Thr Asn Val
            260                 265                 270

Ser Gln Ser Lys Asp Ser Asp Val Tyr Ile Thr Asp Lys Thr Val Leu
        275                 280                 285

Asp Met Arg Ser Met Asp Phe Lys Ser Asn Ser Ala Val Ala Trp Ser
    290                 295                 300

Asn Lys Ser Asp Phe Ala Cys Ala Asn Ala Phe Asn Asn Ser Ile Ile
305                 310                 315                 320

Pro Glu Asp Thr Phe Phe Pro Ser Pro Glu Ser Ser Cys Asp Val Lys
                325                 330                 335

Leu Val Glu Lys Ser Phe Glu Thr Asp Thr Asn Leu Asn Phe Gln Asn
            340                 345                 350

Leu Ser Val Ile Gly Phe Arg Ile Leu Leu Leu Lys Val Ala Gly Phe
        355                 360                 365

Asn Leu Leu Met Thr Leu Arg Leu Trp Ser Ser
    370                 375

<210>  51
<211>  353
<212>  PRT
<213>  Artificial Sequence

<220>
<223>  Amino acid sequence of ba3

<400>  51

Met Leu Ser Leu Leu Leu Leu Leu Leu Gly Leu Gly Ser Val Phe Ser
1               5                   10                  15

Ala Thr Leu Val Cys Leu Ala Thr Gly Phe Tyr Pro Asp His Val Glu
            20                  25                  30

Leu Ser Trp Trp Val Asn Gly Lys Glu Val His Ser Gly Val Ser Thr
        35                  40                  45

Asp Pro Gln Pro Leu Lys Glu Gln Pro Ala Leu Asn Asp Ser Arg Tyr
    50                  55                  60

Cys Leu Ser Ser Arg Leu Arg Val Ser Ala Thr Phe Trp Gln Asn Pro
65                  70                  75                  80

Arg Asn His Phe Arg Cys Gln Val Gln Phe Tyr Gly Leu Ser Glu Asn
                85                  90                  95

Asp Glu Trp Thr Gln Asp Arg Ala Lys Pro Val Thr Gln Ile Val Ser
            100                 105                 110

Ala Glu Ala Trp Gly Arg Ala Asp Cys Gly Phe Thr Ser Glu Ser Tyr
        115                 120                 125

Gln Gln Gly Val Leu Ser Ala Thr Ile Leu Tyr Glu Ile Leu Leu Gly
    130                 135                 140

Lys Ala Thr Leu Tyr Ala Val Leu Val Ser Ala Leu Val Leu Met Ala
145                 150                 155                 160

Met Val Lys Arg Lys Asp Ser Arg Gly Ser Gly Ser Gly Glu Gly Arg
                165                 170                 175

Gly Ser Leu Leu Thr Cys Gly Asp Val Glu Glu Asn Pro Gly Pro Met
            180                 185                 190

Ala Cys Pro Gly Phe Leu Trp Ala Leu Val Ile Ser Thr Cys Leu Glu
        195                 200                 205

Phe Ser Met Ala Asn Ile Gln Asn Pro Asp Pro Ala Val Tyr Gln Leu
    210                 215                 220

Arg Asp Ser Lys Ser Ser Asp Lys Ser Val Cys Leu Phe Thr Asp Phe
225                 230                 235                 240

Asp Ser Gln Thr Asn Val Ser Gln Ser Lys Asp Ser Asp Val Tyr Ile
                245                 250                 255

Thr Asp Lys Thr Val Leu Asp Met Arg Ser Met Asp Phe Lys Ser Asn
            260                 265                 270

Ser Ala Val Ala Trp Ser Asn Lys Ser Asp Phe Ala Cys Ala Asn Ala
        275                 280                 285

Phe Asn Asn Ser Ile Ile Pro Glu Asp Thr Phe Phe Pro Ser Pro Glu
    290                 295                 300

Ser Ser Cys Asp Val Lys Leu Val Glu Lys Ser Phe Glu Thr Asp Thr
305                 310                 315                 320

Asn Leu Asn Phe Gln Asn Leu Ser Val Ile Gly Phe Arg Ile Leu Leu
                325                 330                 335

Leu Lys Val Ala Gly Phe Asn Leu Leu Met Thr Leu Arg Leu Trp Ser
            340                 345                 350

Ser

<210>  52
<211>  324
<212>  PRT
<213>  Artificial Sequence

<220>
<223>  Amino acid sequence of ba4

<400>  52

Met Leu Ser Leu Leu Leu Leu Leu Leu Gly Leu Gly Ser Val Phe Ser
1               5                   10                  15

Val Ser Thr Asp Pro Gln Pro Leu Lys Glu Gln Pro Ala Leu Asn Asp

20                  25                  30

Ser Arg Tyr Cys Leu Ser Ser Arg Leu Arg Val Ser Ala Thr Phe Trp
        35                  40                  45

Gln Asn Pro Arg Asn His Phe Arg Cys Gln Val Gln Phe Tyr Gly Leu
    50                  55                  60

Ser Glu Asn Asp Glu Trp Thr Gln Asp Arg Ala Lys Pro Val Thr Gln
65                  70                  75                  80

Ile Val Ser Ala Glu Ala Trp Gly Arg Ala Asp Cys Gly Phe Thr Ser
                85                  90                  95

Glu Ser Tyr Gln Gln Gly Val Leu Ser Ala Thr Ile Leu Tyr Glu Ile
            100                 105                 110

Leu Leu Gly Lys Ala Thr Leu Tyr Ala Val Leu Val Ser Ala Leu Val
        115                 120                 125

Leu Met Ala Met Val Lys Arg Lys Asp Ser Arg Gly Ser Gly Ser Gly
    130                 135                 140

Glu Gly Arg Gly Ser Leu Leu Thr Cys Gly Asp Val Glu Glu Asn Pro
145                 150                 155                 160

Gly Pro Met Ala Cys Pro Gly Phe Leu Trp Ala Leu Val Ile Ser Thr
                165                 170                 175

Cys Leu Glu Phe Ser Met Ala Asn Ile Gln Asn Pro Asp Pro Ala Val
            180                 185                 190

Tyr Gln Leu Arg Asp Ser Lys Ser Ser Asp Lys Ser Val Cys Leu Phe
        195                 200                 205

Thr Asp Phe Asp Ser Gln Thr Asn Val Ser Gln Ser Lys Asp Ser Asp
    210                 215                 220

Val Tyr Ile Thr Asp Lys Thr Val Leu Asp Met Arg Ser Met Asp Phe
225                 230                 235                 240

Lys Ser Asn Ser Ala Val Ala Trp Ser Asn Lys Ser Asp Phe Ala Cys
                245                 250                 255

Ala Asn Ala Phe Asn Asn Ser Ile Ile Pro Glu Asp Thr Phe Phe Pro
            260                 265                 270

Ser Pro Glu Ser Ser Cys Asp Val Lys Leu Val Glu Lys Ser Phe Glu
        275                 280                 285

Thr Asp Thr Asn Leu Asn Phe Gln Asn Leu Ser Val Ile Gly Phe Arg
    290                 295                 300

Ile Leu Leu Leu Lys Val Ala Gly Phe Asn Leu Leu Met Thr Leu Arg
305                 310                 315                 320

Leu Trp Ser Ser

<210>  53
<211>  286
<212>  PRT
<213>  Artificial Sequence

<220>
<223>  Amino acid sequence of ba5

<400>  53

Met Leu Ser Leu Leu Leu Leu Leu Leu Gly Leu Gly Ser Val Phe Ser
1               5                   10                  15

Phe Arg Cys Gln Val Gln Phe Tyr Gly Leu Ser Glu Asn Asp Glu Trp
            20                  25                  30

Thr Gln Asp Arg Ala Lys Pro Val Thr Gln Ile Val Ser Ala Glu Ala
        35                  40                  45

Trp Gly Arg Ala Asp Cys Gly Phe Thr Ser Glu Ser Tyr Gln Gln Gly
    50                  55                  60

Val Leu Ser Ala Thr Ile Leu Tyr Glu Ile Leu Leu Gly Lys Ala Thr
65                  70                  75                  80

Leu Tyr Ala Val Leu Val Ser Ala Leu Val Leu Met Ala Met Val Lys
                85                  90                  95

Arg Lys Asp Ser Arg Gly Ser Gly Ser Gly Glu Gly Arg Gly Ser Leu
            100                 105                 110

Leu Thr Cys Gly Asp Val Glu Glu Asn Pro Gly Pro Met Ala Cys Pro
        115                 120                 125

Gly Phe Leu Trp Ala Leu Val Ile Ser Thr Cys Leu Glu Phe Ser Met
    130                 135                 140

Ala Asn Ile Gln Asn Pro Asp Pro Ala Val Tyr Gln Leu Arg Asp Ser

145                 150                 155                 160

Lys Ser Ser Asp Lys Ser Val Cys Leu Phe Thr Asp Phe Asp Ser Gln
                165                 170                 175

Thr Asn Val Ser Gln Ser Lys Asp Ser Asp Val Tyr Ile Thr Asp Lys
            180                 185                 190

Thr Val Leu Asp Met Arg Ser Met Asp Phe Lys Ser Asn Ser Ala Val
        195                 200                 205

Ala Trp Ser Asn Lys Ser Asp Phe Ala Cys Ala Asn Ala Phe Asn Asn
    210                 215                 220

Ser Ile Ile Pro Glu Asp Thr Phe Phe Pro Ser Pro Glu Ser Ser Cys
225                 230                 235                 240

Asp Val Lys Leu Val Glu Lys Ser Phe Glu Thr Asp Thr Asn Leu Asn
                245                 250                 255

Phe Gln Asn Leu Ser Val Ile Gly Phe Arg Ile Leu Leu Leu Lys Val
            260                 265                 270

Ala Gly Phe Asn Leu Leu Met Thr Leu Arg Leu Trp Ser Ser
        275                 280                 285

<210>  54
<211>  266
<212>  PRT
<213>  Artificial Sequence

<220>
<223>  Amino acid sequence of ba6

<400>  54

Met Leu Ser Leu Leu Leu Leu Leu Leu Gly Leu Gly Ser Val Phe Ser
1               5                   10                  15

Ala Lys Pro Val Thr Gln Ile Val Ser Ala Glu Ala Trp Gly Arg Ala
            20                  25                  30

Asp Cys Gly Phe Thr Ser Glu Ser Tyr Gln Gln Gly Val Leu Ser Ala
        35                  40                  45

Thr Ile Leu Tyr Glu Ile Leu Leu Gly Lys Ala Thr Leu Tyr Ala Val
    50                  55                  60

Leu Val Ser Ala Leu Val Leu Met Ala Met Val Lys Arg Lys Asp Ser
65                  70                  75                  80

Arg Gly Ser Gly Ser Gly Glu Gly Arg Gly Ser Leu Leu Thr Cys Gly
                85                  90                  95

Asp Val Glu Glu Asn Pro Gly Pro Met Ala Cys Pro Gly Phe Leu Trp
            100                 105                 110

Ala Leu Val Ile Ser Thr Cys Leu Glu Phe Ser Met Ala Asn Ile Gln
        115                 120                 125

Asn Pro Asp Pro Ala Val Tyr Gln Leu Arg Asp Ser Lys Ser Ser Asp
    130                 135                 140

Lys Ser Val Cys Leu Phe Thr Asp Phe Asp Ser Gln Thr Asn Val Ser
145                 150                 155                 160

Gln Ser Lys Asp Ser Asp Val Tyr Ile Thr Asp Lys Thr Val Leu Asp
                165                 170                 175

Met Arg Ser Met Asp Phe Lys Ser Asn Ser Ala Val Ala Trp Ser Asn
            180                 185                 190

Lys Ser Asp Phe Ala Cys Ala Asn Ala Phe Asn Asn Ser Ile Ile Pro
        195                 200                 205

Glu Asp Thr Phe Phe Pro Ser Pro Glu Ser Ser Cys Asp Val Lys Leu
    210                 215                 220

Val Glu Lys Ser Phe Glu Thr Asp Thr Asn Leu Asn Phe Gln Asn Leu
225                 230                 235                 240

Ser Val Ile Gly Phe Arg Ile Leu Leu Leu Lys Val Ala Gly Phe Asn
                245                 250                 255

Leu Leu Met Thr Leu Arg Leu Trp Ser Ser
            260                 265

<210>  55
<211>  359
<212>  PRT
<213>  Artificial Sequence

<220>
<223>  Amino acid sequence of ba7

<400>  55

Met Leu Ser Leu Leu Leu Leu Leu Leu Gly Leu Gly Ser Val Phe Ser
1               5                   10                  15

Glu Asp Leu Lys Asn Val Phe Pro Pro Glu Val Ala Val Phe Glu Pro
            20                  25                  30

Ser Glu Ala Glu Ile Ser His Thr Gln Lys Ala Thr Leu Val Cys Leu
        35                  40                  45

Ala Thr Gly Phe Tyr Pro Asp His Val Glu Leu Ser Trp Trp Val Asn
    50                  55                  60

Gly Lys Glu Val His Ser Gly Val Ser Thr Asp Pro Gln Pro Leu Lys
65                  70                  75                  80

Glu Gln Pro Ala Leu Asn Asp Ser Arg Tyr Cys Leu Ser Ser Arg Leu
                85                  90                  95

Arg Val Ser Ala Thr Phe Trp Gln Asn Pro Arg Asn His Phe Arg Cys
            100                 105                 110

Gln Val Gln Phe Tyr Gly Leu Ser Glu Asn Asp Glu Trp Thr Gln Asp
        115                 120                 125

Arg Ala Lys Pro Val Thr Gln Ile Val Ser Ala Glu Ala Trp Gly Arg
    130                 135                 140

Ala Asp Cys Gly Phe Thr Ser Glu Ser Tyr Gln Gln Gly Val Leu Ser
145                 150                 155                 160

Ala Thr Ile Leu Tyr Glu Ile Leu Leu Gly Lys Ala Thr Leu Tyr Ala
                165                 170                 175

Val Leu Val Ser Ala Leu Val Leu Met Ala Met Val Lys Arg Lys Asp
            180                 185                 190

Ser Arg Gly Ser Gly Ser Gly Glu Gly Arg Gly Ser Leu Leu Thr Cys
        195                 200                 205

Gly Asp Val Glu Glu Asn Pro Gly Pro Met Ala Cys Pro Gly Phe Leu
    210                 215                 220

Trp Ala Leu Val Ile Ser Thr Cys Leu Glu Phe Ser Met Ala Ser Val
225                 230                 235                 240

Cys Leu Phe Thr Asp Phe Asp Ser Gln Thr Asn Val Ser Gln Ser Lys
                245                 250                 255

Asp Ser Asp Val Tyr Ile Thr Asp Lys Thr Val Leu Asp Met Arg Ser
            260                 265                 270

Met Asp Phe Lys Ser Asn Ser Ala Val Ala Trp Ser Asn Lys Ser Asp
        275                 280                 285

Phe Ala Cys Ala Asn Ala Phe Asn Asn Ser Ile Ile Pro Glu Asp Thr
    290                 295                 300

Phe Phe Pro Ser Pro Glu Ser Ser Cys Asp Val Lys Leu Val Glu Lys
305                 310                 315                 320

Ser Phe Glu Thr Asp Thr Asn Leu Asn Phe Gln Asn Leu Ser Val Ile
                325                 330                 335

Gly Phe Arg Ile Leu Leu Leu Lys Val Ala Gly Phe Asn Leu Leu Met
            340                 345                 350

Thr Leu Arg Leu Trp Ser Ser
        355

<210>  56
<211>  339
<212>  PRT
<213>  Artificial Sequence

<220>
<223>  Amino acid sequence of ba8

<400>  56

Met Leu Ser Leu Leu Leu Leu Leu Leu Gly Leu Gly Ser Val Phe Ser
1               5                   10                  15

Glu Asp Leu Lys Asn Val Phe Pro Pro Glu Val Ala Val Phe Glu Pro
            20                  25                  30

Ser Glu Ala Glu Ile Ser His Thr Gln Lys Ala Thr Leu Val Cys Leu
        35                  40                  45

Ala Thr Gly Phe Tyr Pro Asp His Val Glu Leu Ser Trp Trp Val Asn
    50                  55                  60

Gly Lys Glu Val His Ser Gly Val Ser Thr Asp Pro Gln Pro Leu Lys
65                  70                  75                  80

Glu Gln Pro Ala Leu Asn Asp Ser Arg Tyr Cys Leu Ser Ser Arg Leu
                85                  90                  95

Arg Val Ser Ala Thr Phe Trp Gln Asn Pro Arg Asn His Phe Arg Cys
            100                 105                 110

Gln Val Gln Phe Tyr Gly Leu Ser Glu Asn Asp Glu Trp Thr Gln Asp
        115                 120                 125

Arg Ala Lys Pro Val Thr Gln Ile Val Ser Ala Glu Ala Trp Gly Arg
    130                 135                 140

Ala Asp Cys Gly Phe Thr Ser Glu Ser Tyr Gln Gln Gly Val Leu Ser
145                 150                 155                 160

Ala Thr Ile Leu Tyr Glu Ile Leu Leu Gly Lys Ala Thr Leu Tyr Ala
                165                 170                 175

Val Leu Val Ser Ala Leu Val Leu Met Ala Met Val Lys Arg Lys Asp
            180                 185                 190

Ser Arg Gly Ser Gly Ser Gly Glu Gly Arg Gly Ser Leu Leu Thr Cys
        195                 200                 205

Gly Asp Val Glu Glu Asn Pro Gly Pro Met Ala Cys Pro Gly Phe Leu
    210                 215                 220

Trp Ala Leu Val Ile Ser Thr Cys Leu Glu Phe Ser Met Ala Asp Val
225                 230                 235                 240

Tyr Ile Thr Asp Lys Thr Val Leu Asp Met Arg Ser Met Asp Phe Lys
                245                 250                 255

Ser Asn Ser Ala Val Ala Trp Ser Asn Lys Ser Asp Phe Ala Cys Ala
            260                 265                 270

Asn Ala Phe Asn Asn Ser Ile Ile Pro Glu Asp Thr Phe Phe Pro Ser
        275                 280                 285

Pro Glu Ser Ser Cys Asp Val Lys Leu Val Glu Lys Ser Phe Glu Thr
    290                 295                 300

Asp Thr Asn Leu Asn Phe Gln Asn Leu Ser Val Ile Gly Phe Arg Ile
305                 310                 315                 320

Leu Leu Leu Lys Val Ala Gly Phe Asn Leu Leu Met Thr Leu Arg Leu
                325                 330                 335

Trp Ser Ser

<210>  57
<211>  322

<212>  PRT
<213>  Artificial Sequence

<220>
<223>  Amino acid sequence of ba9

<400>  57

Met Leu Ser Leu Leu Leu Leu Leu Leu Gly Leu Gly Ser Val Phe Ser
1               5                   10                  15

Glu Asp Leu Lys Asn Val Phe Pro Pro Glu Val Ala Val Phe Glu Pro
            20                  25                  30

Ser Glu Ala Glu Ile Ser His Thr Gln Lys Ala Thr Leu Val Cys Leu
        35                  40                  45

Ala Thr Gly Phe Tyr Pro Asp His Val Glu Leu Ser Trp Trp Val Asn
    50                  55                  60

Gly Lys Glu Val His Ser Gly Val Ser Thr Asp Pro Gln Pro Leu Lys
65                  70                  75                  80

Glu Gln Pro Ala Leu Asn Asp Ser Arg Tyr Cys Leu Ser Ser Arg Leu
                85                  90                  95

Arg Val Ser Ala Thr Phe Trp Gln Asn Pro Arg Asn His Phe Arg Cys
            100                 105                 110

Gln Val Gln Phe Tyr Gly Leu Ser Glu Asn Asp Glu Trp Thr Gln Asp
        115                 120                 125

Arg Ala Lys Pro Val Thr Gln Ile Val Ser Ala Glu Ala Trp Gly Arg
    130                 135                 140

Ala Asp Cys Gly Phe Thr Ser Glu Ser Tyr Gln Gln Gly Val Leu Ser
145                 150                 155                 160

Ala Thr Ile Leu Tyr Glu Ile Leu Leu Gly Lys Ala Thr Leu Tyr Ala
                165                 170                 175

Val Leu Val Ser Ala Leu Val Leu Met Ala Met Val Lys Arg Lys Asp
            180                 185                 190

Ser Arg Gly Ser Gly Ser Gly Glu Gly Arg Gly Ser Leu Leu Thr Cys
        195                 200                 205

Gly Asp Val Glu Glu Asn Pro Gly Pro Met Ala Cys Pro Gly Phe Leu
    210                 215                 220

Trp Ala Leu Val Ile Ser Thr Cys Leu Glu Phe Ser Met Ala Lys Ser
225                 230                 235                 240

Asn Ser Ala Val Ala Trp Ser Asn Lys Ser Asp Phe Ala Cys Ala Asn
                245                 250                 255

Ala Phe Asn Asn Ser Ile Ile Pro Glu Asp Thr Phe Phe Pro Ser Pro
            260                 265                 270

Glu Ser Ser Cys Asp Val Lys Leu Val Glu Lys Ser Phe Glu Thr Asp
        275                 280                 285

Thr Asn Leu Asn Phe Gln Asn Leu Ser Val Ile Gly Phe Arg Ile Leu
    290                 295                 300

Leu Leu Lys Val Ala Gly Phe Asn Leu Leu Met Thr Leu Arg Leu Trp
305                 310                 315                 320

Ser Ser

<210>  58
<211>  298
<212>  PRT
<213>  Artificial Sequence

<220>
<223>  Amino acid sequence of ba10

<400>  58

Met Leu Ser Leu Leu Leu Leu Leu Leu Gly Leu Gly Ser Val Phe Ser
1               5                   10                  15

Glu Asp Leu Lys Asn Val Phe Pro Pro Glu Val Ala Val Phe Glu Pro
            20                  25                  30

Ser Glu Ala Glu Ile Ser His Thr Gln Lys Ala Thr Leu Val Cys Leu
        35                  40                  45

Ala Thr Gly Phe Tyr Pro Asp His Val Glu Leu Ser Trp Trp Val Asn
    50                  55                  60

Gly Lys Glu Val His Ser Gly Val Ser Thr Asp Pro Gln Pro Leu Lys
65                  70                  75                  80

Glu Gln Pro Ala Leu Asn Asp Ser Arg Tyr Cys Leu Ser Ser Arg Leu
                85                  90                  95

Arg Val Ser Ala Thr Phe Trp Gln Asn Pro Arg Asn His Phe Arg Cys
            100                 105                 110

Gln Val Gln Phe Tyr Gly Leu Ser Glu Asn Asp Glu Trp Thr Gln Asp
        115                 120                 125

Arg Ala Lys Pro Val Thr Gln Ile Val Ser Ala Glu Ala Trp Gly Arg
    130                 135                 140

Ala Asp Cys Gly Phe Thr Ser Glu Ser Tyr Gln Gln Gly Val Leu Ser
145                 150                 155                 160

Ala Thr Ile Leu Tyr Glu Ile Leu Leu Gly Lys Ala Thr Leu Tyr Ala
                165                 170                 175

Val Leu Val Ser Ala Leu Val Leu Met Ala Met Val Lys Arg Lys Asp
            180                 185                 190

Ser Arg Gly Ser Gly Ser Gly Glu Gly Arg Gly Ser Leu Leu Thr Cys
        195                 200                 205

Gly Asp Val Glu Glu Asn Pro Gly Pro Met Ala Cys Pro Gly Phe Leu
    210                 215                 220

Trp Ala Leu Val Ile Ser Thr Cys Leu Glu Phe Ser Met Ala Ile Pro
225                 230                 235                 240

Glu Asp Thr Phe Phe Pro Ser Pro Glu Ser Ser Cys Asp Val Lys Leu
                245                 250                 255

Val Glu Lys Ser Phe Glu Thr Asp Thr Asn Leu Asn Phe Gln Asn Leu
            260                 265                 270

Ser Val Ile Gly Phe Arg Ile Leu Leu Leu Lys Val Ala Gly Phe Asn
        275                 280                 285

Leu Leu Met Thr Leu Arg Leu Trp Ser Ser
    290                 295

<210>  59
<211>  333
<212>  PRT
<213>  Artificial Sequence

<220>
<223>  Amino acid sequence of ba11

<400>  59

Met Leu Ser Leu Leu Leu Leu Leu Leu Gly Leu Gly Ser Val Phe Ser

1               5                   10                  15

Ala Thr Leu Val Cys Leu Ala Thr Gly Phe Tyr Pro Asp His Val Glu
            20                  25                  30

Leu Ser Trp Trp Val Asn Gly Lys Glu Val His Ser Gly Val Ser Thr
        35                  40                  45

Asp Pro Gln Pro Leu Lys Glu Gln Pro Ala Leu Asn Asp Ser Arg Tyr
    50                  55                  60

Cys Leu Ser Ser Arg Leu Arg Val Ser Ala Thr Phe Trp Gln Asn Pro
65                  70                  75                  80

Arg Asn His Phe Arg Cys Gln Val Gln Phe Tyr Gly Leu Ser Glu Asn
                85                  90                  95

Asp Glu Trp Thr Gln Asp Arg Ala Lys Pro Val Thr Gln Ile Val Ser
            100                 105                 110

Ala Glu Ala Trp Gly Arg Ala Asp Cys Gly Phe Thr Ser Glu Ser Tyr
        115                 120                 125

Gln Gln Gly Val Leu Ser Ala Thr Ile Leu Tyr Glu Ile Leu Leu Gly
    130                 135                 140

Lys Ala Thr Leu Tyr Ala Val Leu Val Ser Ala Leu Val Leu Met Ala
145                 150                 155                 160

Met Val Lys Arg Lys Asp Ser Arg Gly Ser Gly Ser Gly Glu Gly Arg
                165                 170                 175

Gly Ser Leu Leu Thr Cys Gly Asp Val Glu Glu Asn Pro Gly Pro Met
            180                 185                 190

Ala Cys Pro Gly Phe Leu Trp Ala Leu Val Ile Ser Thr Cys Leu Glu
        195                 200                 205

Phe Ser Met Ala Ser Val Cys Leu Phe Thr Asp Phe Asp Ser Gln Thr
    210                 215                 220

Asn Val Ser Gln Ser Lys Asp Ser Asp Val Tyr Ile Thr Asp Lys Thr
225                 230                 235                 240

Val Leu Asp Met Arg Ser Met Asp Phe Lys Ser Asn Ser Ala Val Ala
                245                 250                 255

Trp Ser Asn Lys Ser Asp Phe Ala Cys Ala Asn Ala Phe Asn Asn Ser
            260                 265                 270

Ile Ile Pro Glu Asp Thr Phe Phe Pro Ser Pro Glu Ser Ser Cys Asp
        275                 280                 285

Val Lys Leu Val Glu Lys Ser Phe Glu Thr Asp Thr Asn Leu Asn Phe
    290                 295                 300

Gln Asn Leu Ser Val Ile Gly Phe Arg Ile Leu Leu Leu Lys Val Ala
305                 310                 315                 320

Gly Phe Asn Leu Leu Met Thr Leu Arg Leu Trp Ser Ser
                325                 330

<210>  60
<211>  313
<212>  PRT
<213>  Artificial Sequence

<220>
<223>  Amino acid sequence of ba12

<400>  60

Met Leu Ser Leu Leu Leu Leu Leu Leu Gly Leu Gly Ser Val Phe Ser
1               5                   10                  15

Ala Thr Leu Val Cys Leu Ala Thr Gly Phe Tyr Pro Asp His Val Glu
            20                  25                  30

Leu Ser Trp Trp Val Asn Gly Lys Glu Val His Ser Gly Val Ser Thr
        35                  40                  45

Asp Pro Gln Pro Leu Lys Glu Gln Pro Ala Leu Asn Asp Ser Arg Tyr
    50                  55                  60

Cys Leu Ser Ser Arg Leu Arg Val Ser Ala Thr Phe Trp Gln Asn Pro
65                  70                  75                  80

Arg Asn His Phe Arg Cys Gln Val Gln Phe Tyr Gly Leu Ser Glu Asn
                85                  90                  95

Asp Glu Trp Thr Gln Asp Arg Ala Lys Pro Val Thr Gln Ile Val Ser
            100                 105                 110

Ala Glu Ala Trp Gly Arg Ala Asp Cys Gly Phe Thr Ser Glu Ser Tyr
        115                 120                 125

Gln Gln Gly Val Leu Ser Ala Thr Ile Leu Tyr Glu Ile Leu Leu Gly

130                 135                 140

Lys Ala Thr Leu Tyr Ala Val Leu Val Ser Ala Leu Val Leu Met Ala
145                 150                 155                 160

Met Val Lys Arg Lys Asp Ser Arg Gly Ser Gly Ser Gly Glu Gly Arg
                165                 170                 175

Gly Ser Leu Leu Thr Cys Gly Asp Val Glu Glu Asn Pro Gly Pro Met
            180                 185                 190

Ala Cys Pro Gly Phe Leu Trp Ala Leu Val Ile Ser Thr Cys Leu Glu
        195                 200                 205

Phe Ser Met Ala Asp Val Tyr Ile Thr Asp Lys Thr Val Leu Asp Met
    210                 215                 220

Arg Ser Met Asp Phe Lys Ser Asn Ser Ala Val Ala Trp Ser Asn Lys
225                 230                 235                 240

Ser Asp Phe Ala Cys Ala Asn Ala Phe Asn Asn Ser Ile Ile Pro Glu
                245                 250                 255

Asp Thr Phe Phe Pro Ser Pro Glu Ser Ser Cys Asp Val Lys Leu Val
            260                 265                 270

Glu Lys Ser Phe Glu Thr Asp Thr Asn Leu Asn Phe Gln Asn Leu Ser
        275                 280                 285

Val Ile Gly Phe Arg Ile Leu Leu Leu Lys Val Ala Gly Phe Asn Leu
    290                 295                 300

Leu Met Thr Leu Arg Leu Trp Ser Ser
305                 310

<210>  61
<211>  296
<212>  PRT
<213>  Artificial Sequence

<220>
<223>  Amino acid sequence of ba13

<400>  61

Met Leu Ser Leu Leu Leu Leu Leu Leu Gly Leu Gly Ser Val Phe Ser
1               5                   10                  15

Ala Thr Leu Val Cys Leu Ala Thr Gly Phe Tyr Pro Asp His Val Glu
            20                  25                  30

Leu Ser Trp Trp Val Asn Gly Lys Glu Val His Ser Gly Val Ser Thr
        35                  40                  45

Asp Pro Gln Pro Leu Lys Glu Gln Pro Ala Leu Asn Asp Ser Arg Tyr
    50                  55                  60

Cys Leu Ser Ser Arg Leu Arg Val Ser Ala Thr Phe Trp Gln Asn Pro
65                  70                  75                  80

Arg Asn His Phe Arg Cys Gln Val Gln Phe Tyr Gly Leu Ser Glu Asn
                85                  90                  95

Asp Glu Trp Thr Gln Asp Arg Ala Lys Pro Val Thr Gln Ile Val Ser
            100                 105                 110

Ala Glu Ala Trp Gly Arg Ala Asp Cys Gly Phe Thr Ser Glu Ser Tyr
        115                 120                 125

Gln Gln Gly Val Leu Ser Ala Thr Ile Leu Tyr Glu Ile Leu Leu Gly
    130                 135                 140

Lys Ala Thr Leu Tyr Ala Val Leu Val Ser Ala Leu Val Leu Met Ala
145                 150                 155                 160

Met Val Lys Arg Lys Asp Ser Arg Gly Ser Gly Ser Gly Glu Gly Arg
                165                 170                 175

Gly Ser Leu Leu Thr Cys Gly Asp Val Glu Glu Asn Pro Gly Pro Met
            180                 185                 190

Ala Cys Pro Gly Phe Leu Trp Ala Leu Val Ile Ser Thr Cys Leu Glu
        195                 200                 205

Phe Ser Met Ala Lys Ser Asn Ser Ala Val Ala Trp Ser Asn Lys Ser
    210                 215                 220

Asp Phe Ala Cys Ala Asn Ala Phe Asn Asn Ser Ile Ile Pro Glu Asp
225                 230                 235                 240

Thr Phe Phe Pro Ser Pro Glu Ser Ser Cys Asp Val Lys Leu Val Glu
                245                 250                 255

Lys Ser Phe Glu Thr Asp Thr Asn Leu Asn Phe Gln Asn Leu Ser Val
            260                 265                 270

Ile Gly Phe Arg Ile Leu Leu Leu Lys Val Ala Gly Phe Asn Leu Leu

275                 280                 285

Met Thr Leu Arg Leu Trp Ser Ser
    290                 295

<210>  62
<211>  272
<212>  PRT
<213>  Artificial Sequence

<220>
<223>  Amino acid sequence of ba14

<400>  62

Met Leu Ser Leu Leu Leu Leu Leu Leu Gly Leu Gly Ser Val Phe Ser
1               5                   10                  15

Ala Thr Leu Val Cys Leu Ala Thr Gly Phe Tyr Pro Asp His Val Glu
            20                  25                  30

Leu Ser Trp Trp Val Asn Gly Lys Glu Val His Ser Gly Val Ser Thr
        35                  40                  45

Asp Pro Gln Pro Leu Lys Glu Gln Pro Ala Leu Asn Asp Ser Arg Tyr
    50                  55                  60

Cys Leu Ser Ser Arg Leu Arg Val Ser Ala Thr Phe Trp Gln Asn Pro
65                  70                  75                  80

Arg Asn His Phe Arg Cys Gln Val Gln Phe Tyr Gly Leu Ser Glu Asn
                85                  90                  95

Asp Glu Trp Thr Gln Asp Arg Ala Lys Pro Val Thr Gln Ile Val Ser
            100                 105                 110

Ala Glu Ala Trp Gly Arg Ala Asp Cys Gly Phe Thr Ser Glu Ser Tyr
        115                 120                 125

Gln Gln Gly Val Leu Ser Ala Thr Ile Leu Tyr Glu Ile Leu Leu Gly
    130                 135                 140

Lys Ala Thr Leu Tyr Ala Val Leu Val Ser Ala Leu Val Leu Met Ala
145                 150                 155                 160

Met Val Lys Arg Lys Asp Ser Arg Gly Ser Gly Ser Gly Glu Gly Arg
                165                 170                 175

Gly Ser Leu Leu Thr Cys Gly Asp Val Glu Glu Asn Pro Gly Pro Met
            180                 185                 190

Ala Cys Pro Gly Phe Leu Trp Ala Leu Val Ile Ser Thr Cys Leu Glu
        195                 200                 205

Phe Ser Met Ala Ile Pro Glu Asp Thr Phe Phe Pro Ser Pro Glu Ser
    210                 215                 220

Ser Cys Asp Val Lys Leu Val Glu Lys Ser Phe Glu Thr Asp Thr Asn
225                 230                 235                 240

Leu Asn Phe Gln Asn Leu Ser Val Ile Gly Phe Arg Ile Leu Leu Leu
                245                 250                 255

Lys Val Ala Gly Phe Asn Leu Leu Met Thr Leu Arg Leu Trp Ser Ser
            260                 265                 270

<210>  63
<211>  246
<212>  PRT
<213>  Artificial Sequence

<220>
<223>  Amino acid sequence of ba15

<400>  63

Met Leu Ser Leu Leu Leu Leu Leu Leu Gly Leu Gly Ser Val Phe Ser
1               5                   10                  15

Ala Lys Pro Val Thr Gln Ile Val Ser Ala Glu Ala Trp Gly Arg Ala
            20                  25                  30

Asp Cys Gly Phe Thr Ser Glu Ser Tyr Gln Gln Gly Val Leu Ser Ala
        35                  40                  45

Thr Ile Leu Tyr Glu Ile Leu Leu Gly Lys Ala Thr Leu Tyr Ala Val
    50                  55                  60

Leu Val Ser Ala Leu Val Leu Met Ala Met Val Lys Arg Lys Asp Ser
65                  70                  75                  80

Arg Gly Ser Gly Ser Gly Glu Gly Arg Gly Ser Leu Leu Thr Cys Gly
                85                  90                  95

Asp Val Glu Glu Asn Pro Gly Pro Met Ala Cys Pro Gly Phe Leu Trp
            100                 105                 110

Ala Leu Val Ile Ser Thr Cys Leu Glu Phe Ser Met Ala Ser Val Cys
        115                 120                 125

Leu Phe Thr Asp Phe Asp Ser Gln Thr Asn Val Ser Gln Ser Lys Asp
    130                 135                 140

Ser Asp Val Tyr Ile Thr Asp Lys Thr Val Leu Asp Met Arg Ser Met
145                 150                 155                 160

Asp Phe Lys Ser Asn Ser Ala Val Ala Trp Ser Asn Lys Ser Asp Phe
                165                 170                 175

Ala Cys Ala Asn Ala Phe Asn Asn Ser Ile Ile Pro Glu Asp Thr Phe
            180                 185                 190

Phe Pro Ser Pro Glu Ser Ser Cys Asp Val Lys Leu Val Glu Lys Ser
        195                 200                 205

Phe Glu Thr Asp Thr Asn Leu Asn Phe Gln Asn Leu Ser Val Ile Gly
    210                 215                 220

Phe Arg Ile Leu Leu Leu Lys Val Ala Gly Phe Asn Leu Leu Met Thr
225                 230                 235                 240

Leu Arg Leu Trp Ser Ser
                245

<210>  64
<211>  226
<212>  PRT
<213>  Artificial Sequence

<220>
<223>  Amino acid sequence of ba16

<400>  64

Met Leu Ser Leu Leu Leu Leu Leu Leu Gly Leu Gly Ser Val Phe Ser
1               5                   10                  15

Ala Lys Pro Val Thr Gln Ile Val Ser Ala Glu Ala Trp Gly Arg Ala
            20                  25                  30

Asp Cys Gly Phe Thr Ser Glu Ser Tyr Gln Gln Gly Val Leu Ser Ala
        35                  40                  45

Thr Ile Leu Tyr Glu Ile Leu Leu Gly Lys Ala Thr Leu Tyr Ala Val
    50                  55                  60

Leu Val Ser Ala Leu Val Leu Met Ala Met Val Lys Arg Lys Asp Ser
65                  70                  75                  80

Arg Gly Ser Gly Ser Gly Glu Gly Arg Gly Ser Leu Leu Thr Cys Gly
                85                  90                  95

Asp Val Glu Glu Asn Pro Gly Pro Met Ala Cys Pro Gly Phe Leu Trp
            100                 105                 110

Ala Leu Val Ile Ser Thr Cys Leu Glu Phe Ser Met Ala Asp Val Tyr
        115                 120                 125

Ile Thr Asp Lys Thr Val Leu Asp Met Arg Ser Met Asp Phe Lys Ser
    130                 135                 140

Asn Ser Ala Val Ala Trp Ser Asn Lys Ser Asp Phe Ala Cys Ala Asn
145                 150                 155                 160

Ala Phe Asn Asn Ser Ile Ile Pro Glu Asp Thr Phe Phe Pro Ser Pro
                165                 170                 175

Glu Ser Ser Cys Asp Val Lys Leu Val Glu Lys Ser Phe Glu Thr Asp
            180                 185                 190

Thr Asn Leu Asn Phe Gln Asn Leu Ser Val Ile Gly Phe Arg Ile Leu
        195                 200                 205

Leu Leu Lys Val Ala Gly Phe Asn Leu Leu Met Thr Leu Arg Leu Trp
    210                 215                 220

Ser Ser
225

<210>  65
<211>  209
<212>  PRT
<213>  Artificial Sequence

<220>
<223>  Amino acid sequence of ba17

<400>  65

Met Leu Ser Leu Leu Leu Leu Leu Leu Gly Leu Gly Ser Val Phe Ser
1               5                   10                  15

Ala Lys Pro Val Thr Gln Ile Val Ser Ala Glu Ala Trp Gly Arg Ala
            20                  25                  30

Asp Cys Gly Phe Thr Ser Glu Ser Tyr Gln Gln Gly Val Leu Ser Ala
        35                  40                  45

Thr Ile Leu Tyr Glu Ile Leu Leu Gly Lys Ala Thr Leu Tyr Ala Val

50                  55                  60

Leu Val Ser Ala Leu Val Leu Met Ala Met Val Lys Arg Lys Asp Ser
65                  70                  75                  80

Arg Gly Ser Gly Ser Gly Glu Gly Arg Gly Ser Leu Leu Thr Cys Gly
                85                  90                  95

Asp Val Glu Glu Asn Pro Gly Pro Met Ala Cys Pro Gly Phe Leu Trp
            100                 105                 110

Ala Leu Val Ile Ser Thr Cys Leu Glu Phe Ser Met Ala Lys Ser Asn
        115                 120                 125

Ser Ala Val Ala Trp Ser Asn Lys Ser Asp Phe Ala Cys Ala Asn Ala
    130                 135                 140

Phe Asn Asn Ser Ile Ile Pro Glu Asp Thr Phe Phe Pro Ser Pro Glu
145                 150                 155                 160

Ser Ser Cys Asp Val Lys Leu Val Glu Lys Ser Phe Glu Thr Asp Thr
                165                 170                 175

Asn Leu Asn Phe Gln Asn Leu Ser Val Ile Gly Phe Arg Ile Leu Leu
            180                 185                 190

Leu Lys Val Ala Gly Phe Asn Leu Leu Met Thr Leu Arg Leu Trp Ser
        195                 200                 205

Ser

<210>  66
<211>  185
<212>  PRT
<213>  Artificial Sequence

<220>
<223>  Amino acid sequence of ba18

<400>  66

Met Leu Ser Leu Leu Leu Leu Leu Leu Gly Leu Gly Ser Val Phe Ser
1               5                   10                  15

Ala Lys Pro Val Thr Gln Ile Val Ser Ala Glu Ala Trp Gly Arg Ala
            20                  25                  30

Asp Cys Gly Phe Thr Ser Glu Ser Tyr Gln Gln Gly Val Leu Ser Ala
        35                  40                  45

Thr Ile Leu Tyr Glu Ile Leu Leu Gly Lys Ala Thr Leu Tyr Ala Val
    50                  55                  60

Leu Val Ser Ala Leu Val Leu Met Ala Met Val Lys Arg Lys Asp Ser
65                  70                  75                  80

Arg Gly Ser Gly Ser Gly Glu Gly Arg Gly Ser Leu Leu Thr Cys Gly
                85                  90                  95

Asp Val Glu Glu Asn Pro Gly Pro Met Ala Cys Pro Gly Phe Leu Trp
            100                 105                 110

Ala Leu Val Ile Ser Thr Cys Leu Glu Phe Ser Met Ala Ile Pro Glu
        115                 120                 125

Asp Thr Phe Phe Pro Ser Pro Glu Ser Ser Cys Asp Val Lys Leu Val
    130                 135                 140

Glu Lys Ser Phe Glu Thr Asp Thr Asn Leu Asn Phe Gln Asn Leu Ser
145                 150                 155                 160

Val Ile Gly Phe Arg Ile Leu Leu Leu Lys Val Ala Gly Phe Asn Leu
                165                 170                 175

Leu Met Thr Leu Arg Leu Trp Ser Ser
            180                 185

<210>  67
<211>  20
<212>  DNA
<213>  Artificial Sequence

<220>
<223>  Base sequence of TRAC

<400>  67
gagaatcaaa atcggtgaat                                                   20

<210>  68
<211>  20
<212>  DNA
<213>  Artificial Sequence

<220>
<223>  Base sequence of TRBC

<400>  68
caaacacagc gacctcgggt                                                   20

<210>  69

<211>  24
<212>  DNA
<213>  Artificial Sequence

<220>
<223>  Synthesized oligonucleotide for primer

<400>  69
taccagctga gagactctaa atcc                                              24


<210>  70
<211>  25
<212>  DNA
<213>  Artificial Sequence

<220>
<223>  Synthesized oligonucleotide for primer

<400>  70
tctgtctgcc tattcaccga ttttg                                             25


<210>  71
<211>  30
<212>  DNA
<213>  Artificial Sequence

<220>
<223>  Synthesized oligonucleotide for primer

<400>  71
aatgtgtcac aaagtaagga ttctgatgtg                                        30


<210>  72
<211>  31
<212>  DNA
<213>  Artificial Sequence

<220>
<223>  Synthesized oligonucleotide for primer

<400>  72
gatgtgtata tcacagacaa aactgtgcta g                                      31


<210>  73
<211>  27
<212>  DNA
<213>  Artificial Sequence

<220>
<223>  Synthesized oligonucleotide for primer

<400>  73
ctagacatga ggtctatgga cttcaag                                           27


<210>  74
<211>  21
<212>  DNA
<213>  Artificial Sequence

<220>
<223>  Synthesized oligonucleotide for primer

<400>  74
aagagcaaca gtgctgtggc c                                                21


<210>  75
<211>  25
<212>  DNA
<213>  Artificial Sequence

<220>
<223>  Synthesized oligonucleotide for primer

<400>  75
agcaacaaat ctgactttgc atgtg                                            25


<210>  76
<211>  23
<212>  DNA
<213>  Artificial Sequence

<220>
<223>  Synthesized oligonucleotide for primer

<400>  76
tgtgcaaacg ccttcaacaa cag                                              23


<210>  77
<211>  27
<212>  DNA
<213>  Artificial Sequence

<220>
<223>  Synthesized oligonucleotide for primer

<400>  77
ttcaacaaca gcattattcc agaagac                                          27


<210>  78
<211>  24
<212>  DNA
<213>  Artificial Sequence

<220>
<223>  Synthesized oligonucleotide for primer

<400>  78
attccagaag acaccttctt cccc                                             24


<210>  79
<211>  25
<212>  DNA
<213>  Artificial Sequence

<220>
<223>  Synthesized oligonucleotide for primer

<400>  79
tcagaagcag agatctccca caccc                                          25


<210>  80
<211>  21
<212>  DNA
<213>  Artificial Sequence

<220>
<223>  Synthesized oligonucleotide for primer

<400>  80
gccacactgg tgtgcctggc c                                              21


<210>  81
<211>  21
<212>  DNA
<213>  Artificial Sequence

<220>
<223>  Synthesized oligonucleotide for primer

<400>  81
gtggagctga gctggtgggt g                                              21


<210>  82
<211>  25
<212>  DNA
<213>  Artificial Sequence

<220>
<223>  Synthesized oligonucleotide for primer

<400>  82
gtgaatggga aggaggtgca cagtg                                          25


<210>  83
<211>  22
<212>  DNA
<213>  Artificial Sequence

<220>
<223>  Synthesized oligonucleotide for primer

<400>  83
gtcagcacag acccgcagcc cc                                             22


<210>  84
<211>  25
<212>  DNA
<213>  Artificial Sequence

<220>
<223>  Synthesized oligonucleotide for primer

<400>  84
gccctcaatg actccagata ctgcc                                          25

<210>  85
<211>  20
<212>  DNA
<213>  Artificial Sequence

<220>
<223>  Synthesized oligonucleotide for primer

<400>  85
cgcctgaggg tctcggccac                                                    20


<210>  86
<211>  22
<212>  DNA
<213>  Artificial Sequence

<220>
<223>  Synthesized oligonucleotide for primer

<400>  86
cagaaccccc gcaaccactt cc                                                 22


<210>  87
<211>  25
<212>  DNA
<213>  Artificial Sequence

<220>
<223>  Synthesized oligonucleotide for primer

<400>  87
ttccgctgtc aagtccagtt ctacg                                              25


<210>  88
<211>  22
<212>  DNA
<213>  Artificial Sequence

<220>
<223>  Synthesized oligonucleotide for primer

<400>  88
tacgggctct cggagaatga cg                                                 22


<210>  89
<211>  26
<212>  DNA
<213>  Artificial Sequence

<220>
<223>  Synthesized oligonucleotide for primer

<400>  89
gagtggaccc aggatagggc caaacc                                             26


<210>  90

<211>  24
<212>  DNA
<213>  Artificial Sequence

<220>
<223>  Synthesized oligonucleotide for primer

<400>  90
gccaaacctg tcacccagat cgtc                                              24


<210>  91
<211>  25
<212>  DNA
<213>  Artificial Sequence

<220>
<223>  Synthesized oligonucleotide for primer

<400>  91
agccatgcta aattcaagac aggtg                                             25


<210>  92
<211>  24
<212>  DNA
<213>  Artificial Sequence

<220>
<223>  Synthesized oligonucleotide for primer

<400>  92
actgaacaca gagcctagtc ccag                                              24